# EUROPEAN PATENT SPECIFICATION

(11) **EP 1 976 381 B1**
(45) Date of publication and mention of the grant of the patent: **24.07.2013**
(21) Application number: 07710092.3
(22) Date of filing: 12.01.2007
(51) Int. Cl.: C07D 309/10, C07D 309/14, C07D 405/12, A61K 31/351, A61P 25/24

(54) **TRI-SUBSTITUTED 2-BENZHYDRYL-5-BENZLAMINO-TETRAHYDRO-PYRAN-4-OL AND 6-BENZHYDRYL-4-BENZYLAMINO-TETRAHYDRO-PYRAN-3-OL ANALOGUES, AND NOVEL 3,6-DISUBSTITUTED PYRAN DERIVATIVES**
TRISUBSTITUIERTE 2-BENZHYDRYL-5-BENZYLAMINO-TETRAHYDROPYRAN-4-OL- UND 6-BENZHYDRYL-4-BENZYLAMINOTETRAHYDROPYRAN-3-OL-ANALOGE UND NEUE 3,6-DISUBSTITUIERTE PYRANDERIVATE
ANALOGUES DE 2-BENZHYDRYL-5-BENZYLAMINO-TÉTRAHYDRO-PYRAN-4-OL ET DE 6-BENZHYDRYL-4-BENZYLAMINO-TÉTRAHYDRO-PYRAN-3-OL TRISUBSTITUÉS ET NOUVEAUX DÉRIVÉS DE PYRANE DISUBSTITUÉS EN POSITIONS 3 ET 6

(30) Priority: 12.01.2006 US 330972
(43) Date of publication of application: 08.10.2008
(73) Proprietor: WAYNE STATE UNIVERSITY, Detroit, MI 48202 (US)
(72) Inventor: DUTTA, Aloke, K., Novi, MI 48375 (US)
(74) Representative: Hart, Deborah Mary
(86) International application number: PCT/US2007/060455
(87) International publication number: WO 2007/082292

(56) References cited:
- WO-A1-2005/105075
- WO-A1-2005/105075
- US-B1- 6 387 389
- US-B1- 6 387 389
- ZHANG S ET AL: "Design,Synthesis and Activity of Novel cis- and trans-3,6-Disubstituted Pyran Biomimetics of 3,6-Disubstituted Piperidine as Potential Ligands for the Dopamine Transporter" BIOORGANIC & MEDICINAL CHEMISTRY LETTERS, PERGAMON, ELSEVIER SCIENCE, GB LNKD- DOI:10.1016/S0960-894X(03)00169-0, vol. 13, 1 May 2003 (2003-05-01), pages 1591-1595, XP002988640 ISSN: 0960-894X
- SHIJUN ZHANGA, JUAN ZHENB, C, MAARTEN E.A. REITHB, C AND ALOKE K. DUTTA: "Structural requirements for 2,4- and 3,6-disubstituted pyran biomimetics of cis-(6-benzhydryl-piperidin-3-yl)-benzylam ine compounds to interact with monoamine transporters" BIOORGANIC & MEDICINAL CHEMISTRY, vol. 12, no. 23, 1 December 2004 (2004-12-01), pages 6301-6315, XP002583212
- ZHANG ET AL.: 'Discovery of Novel Trisubstituted Asymmetric Derivatives of (2S,4R,5R)-2-benzhydryl-5-benzylaminotetrah ydropyran-4-ol, Exhibiting High Affinity for Serotonin and Norepinephrine Transporters in a Stereospecific Manner' J. MED. CHEM. vol. 48, 2005, pages 4962 - 4971, XP008128170
- ZHANG S ET AL: "Design,Synthesis and Activity of Novel cis- and trans-3,6-Disubstituted Pyran Biomimetics of 3,6-Disubstituted Piperidine as Potential Ligands for the Dopamine Transporter", BIOORGANIC & MEDICINAL CHEMISTRY LETTERS, PERGAMON, ELSEVIER SCIENCE, GB, vol. 13, 1 May 2003 (2003-05-01), pages 1591-1595, XP002988640, ISSN: 0960-894X, DOI: 10.1016/S0960-894X(03)00169-0
- SHIJUN ZHANGA ET AL: "Structural requirements for 2,4- and 3,6-disubstituted pyran biomimetics of cis-(6-benzhydryl-piperidin-3-yl)-benzylam ine compounds to interact with monoamine transporters", BIOORGANIC & MEDICINAL CHEMISTRY, PERGAMON, GB, vol. 12, no. 23, 1 December 2004 (2004-12-01), pages 6301-6315, XP002583212, ISSN: 0968-0896, DOI: 10.1016/J.BMC.2004.07.069 [retrieved on 2004-09-28]

## Description

### CROSS-REFERENCE TO RELATED APPLICATION

This application is a continuation-in-part of International Patent Application No. PCT/US2005/012748, filed April 15, 2005 and published in English, which claims the benefit of U.S. Provisional Application Serial No. 60/563,189, filed April 16, 2004. This application is also a continuation-in-part of U.S. Application Serial No. 10/311,796, filed March 28, 2003.

### BACKGROUND OF THE INVENTION

### 1. Field of the Invention

The invention pertains to pharmacologically active 3,6-disubstituted pyran compounds and similar compounds having additional substitution on the pyran ring. The compounds show high activity at monoamine transporters, and thus can be used to alter reuptake of monoamines in treatment of numerous diseases in mammalian species for which alteration of the monoamine transport system is indicated.

### 2. Background Art

The monoamine transporters terminate the action of released biogenic amines such as dopamine (DA), norepinephrine (NE) and serotonin (5-HT) in the central nervous system (CNS) and are known as dopamine transporter (DAT), norepinephrine transporter(NET) and serotonin transporter (SERT), respectively. These transporters play a vital role in maintaining the extracellular concentration of biogenic amine neurotransmitters. Drugs binding to the DAT are typically regarded as stimulants. Cocaine- and amphetamine-related compounds are known to produce their action by binding to both DAT and SERT with cocaine acting as a blocker and amphetamine as a substrate. On the other hand, drugs binding to the SERT and NET are known to produce, among other effects, potent antidepressant activity.⁸⁻¹⁰

Major depression disorder is a significant health problem, and behind cardiovascular disease, depression is considered as the second most debilitating disease in the world. Unipolar depression is ranked number 1 before all other somatic and psychiatric illness. It is believed that more than 20% of individuals suffer from a depressive episode at least once in their lifetime. Depression is potentially fatal since many people suffering from depression contemplate suicide and other life threatening acts.

Selective monoamine uptake inhibitors have been implicated in the treatment of depression. In these classes specifically, serotonin and norepinephrine transporter blockers have been used in therapy for depression. Antidepressants are thought to elicit their therapeutic effects by increasing synaptic concentrations of serotonin and norepinephrine in the synapse. Earlier developed tricyclic antidepressants acted by enhancing both serotonin and norepinephrine transmissions. However, due to their non-specific interactions with the other CNS receptors, they exhibited toxic side effects which have limited their clinical use. Development of selective serotonin reuptake inhibitors (SSRI) alleviated many side effects exhibited by traditional trycyclic antidepressants and thus have proven to be more effective. However, the delayed onset action of SSRI sometime proved to have fatal consequences for patients afflicted with manic depression and in need of immediate help. SSRIs also have been implicated in number of other side effects which include insomnia, sexual dysfunction and nausea, etc. More recently, SSRIs have been implicated in suicide risk in adolescent population who were medicated with these drugs, raising some serious questions on the safety of SSRI. Lately, serotonin and norepinephrine dual uptake inhibitors have proven to be more efficacious in that regard. Fast onset of action associated with serotonin norepinephrine reuptake inhibitors (SNRI) was found to be more desirable as there is a pressing need for more faster acting antidepressant agents with reduced undesirable side effects.

Zhang et al (2003) "Design, Synthesis and Activity of Novel cis- and trans 3,6-disubstituted pyran biomimetics of 3,6 disubstituted piperidine as potential ligands for the dopamine transport" Bioorganic & Medicinal Chemistry Letters 13:1591-1595 describe pyran derivatives of a 3,6 disubstituted piperidine template which are ligands for dopamine transporters.

Zhang et al (2004) "Structural requirements for 2,4- and 3,6-disubstituted pyran biomimetics of cis-benzhydryl-piperidin-3-yl)- benzylamine compounds to interact with monoamine transporters" Bioorganic & Medicinal Chemistry 12 (2004) 6301-6315 describe *cis* and *trans* 2,4- and 3,6-disubstituted derivatives of *cis*-(6-benzy lhydryl-piperidin-3-yl) benzylamine which act on monoamine transporters, such as dopamine transporters.

Asymetric derivatives of (2S,4R,5R)-2-benzylhydryl-5-benzylaminotetrahydropropyran-4-ol which act on serotonin and norepinephrine transporters were disclosed by Zhang et al (Journal of Medicinal Chemistry 48 (2005) 4962-4971).

### SUMMARY OF THE INVENTION

It has been surprisingly discovered that 3,6-disubstituted pyrans as hereinafter defined, and in particular 3,6-disubstituted pyrans also containing a further substituent on the pyran ring, exhibit potent activity on monoamine transport systems, and are thus useful in probing the effects of binding to monoamine transport systems and the corresponding relationships to various afflictions affecting the CNS, or as a treatment for various CNS-related disorders in which the monoamine transport system is implicated. It has been surprisingly and unexpectedly discovered that the novel 3,6-disubstituted and 2,5,-4-trisubstituted pyran molecules of the present invention operate as powerful blockers for monoamine transporters.

### BRIEF DESCRIPTION OF THE DRAWINGS

Figure 1 provides a scheme for the preparation of compounds an embodiment of the present invention;
Figure 2 provides a scheme for the preparation of compounds an embodiment of the present invention;
Figure 3 provides a scheme for the preparation of compounds an embodiment of the present invention;
Figure 4 provides a scheme for the preparation of compounds an embodiment of the present invention;
Figure 5 provides a scheme for the preparation of compounds an embodiment of the present invention;

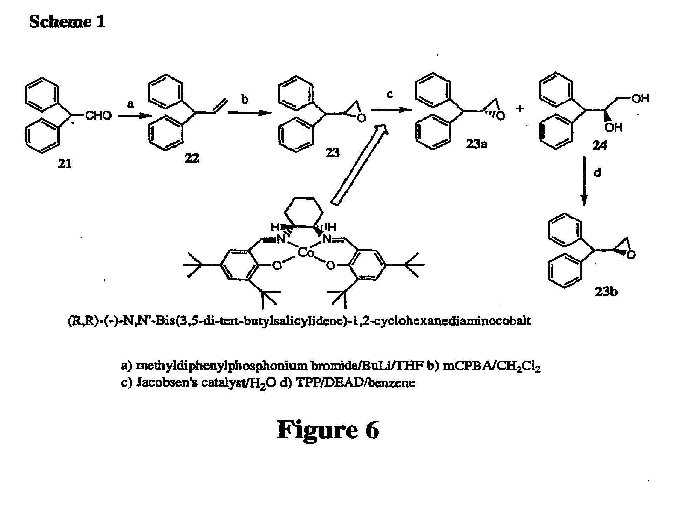

Figure 6 provides a scheme for the preparation of compounds an embodiment of the present invention;

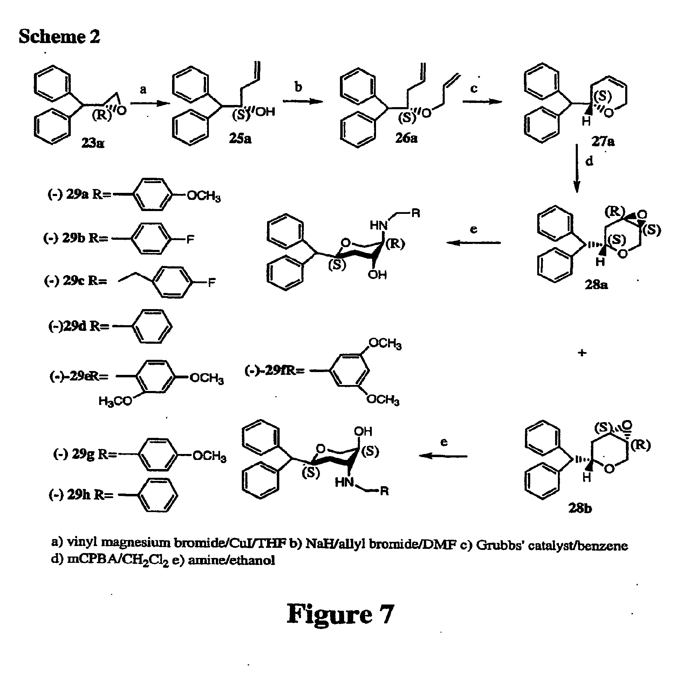

Figure 7 provides a scheme for the preparation of compounds an embodiment of the present invention;

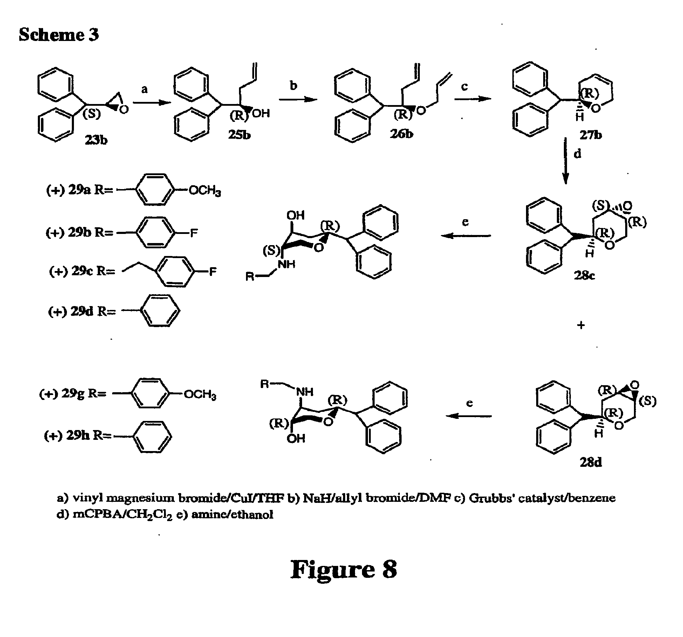

Figure 8 provides a scheme for the preparation of compounds an embodiment of the present invention;

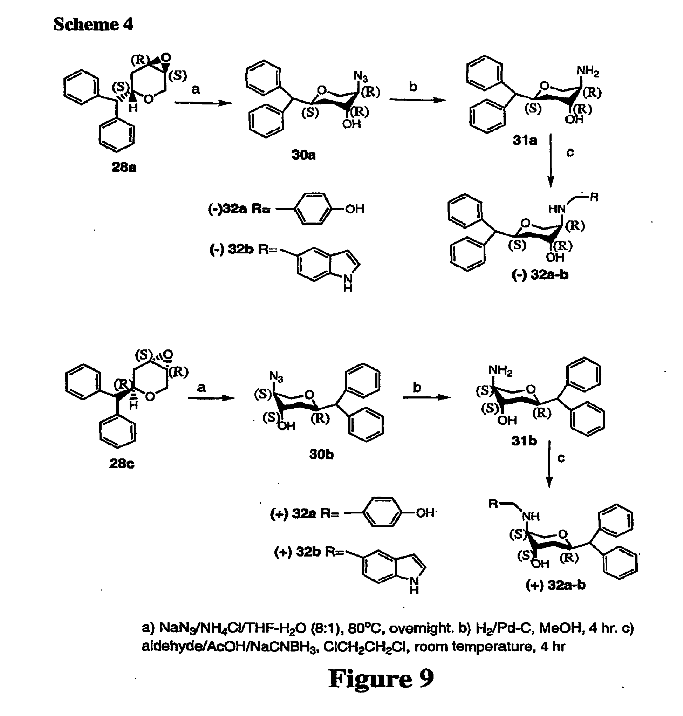

Figure 9 provides a scheme for the preparation of compounds an embodiment of the present invention;

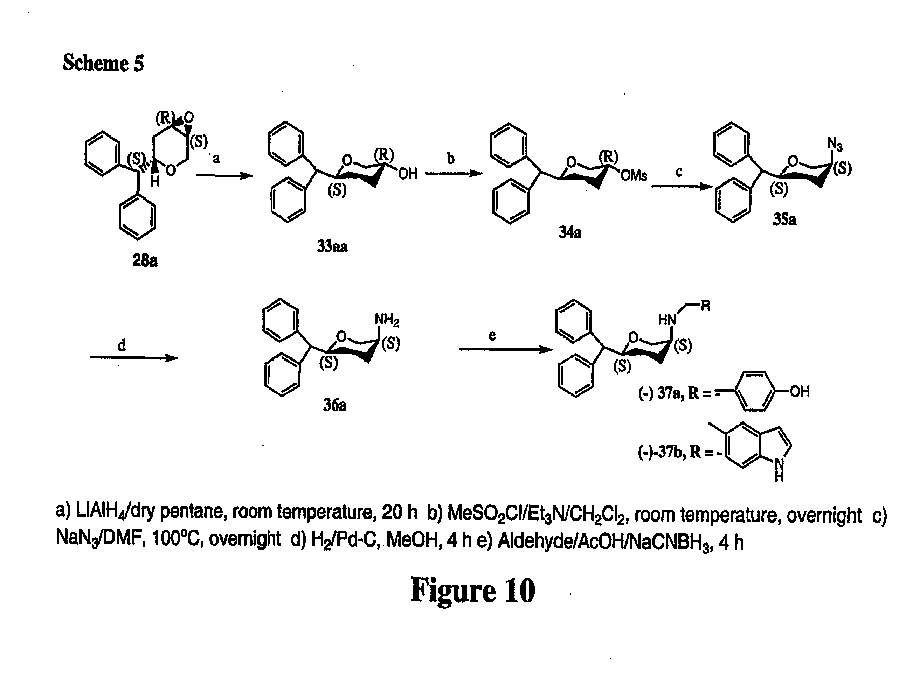

Figure 10 provides a scheme for the preparation of compounds an embodiment of the present invention;

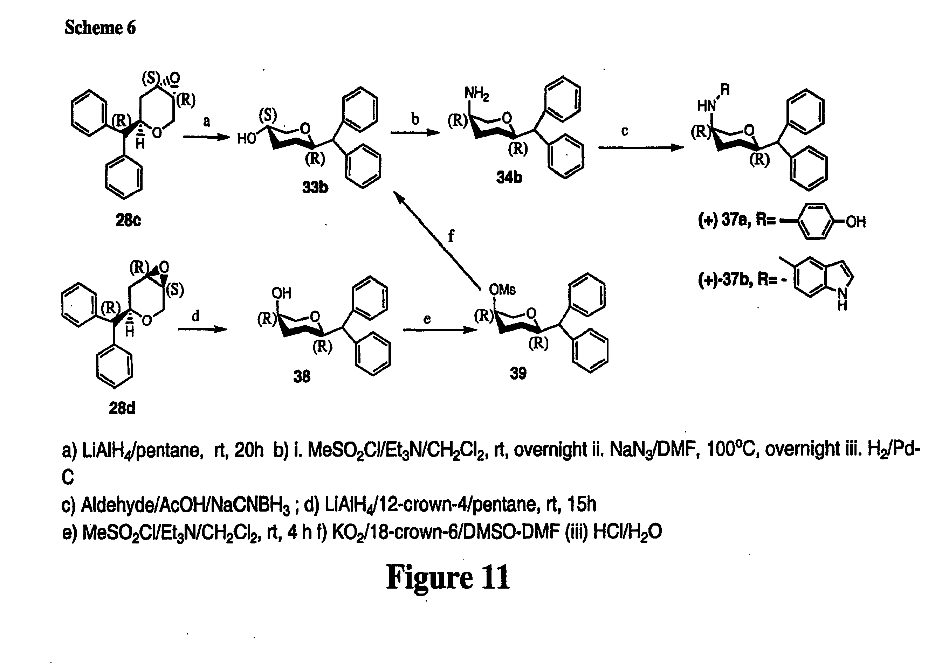

Figure 11 provides a scheme for the preparation of compounds an embodiment of the present invention;

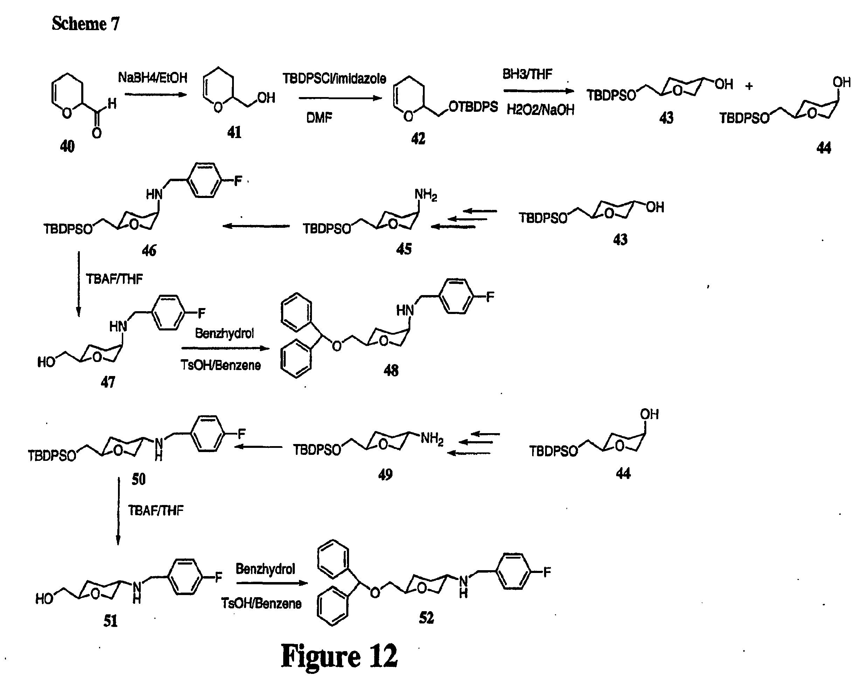

Figure 12 provides a scheme for the preparation of compounds an embodiment of the present invention;

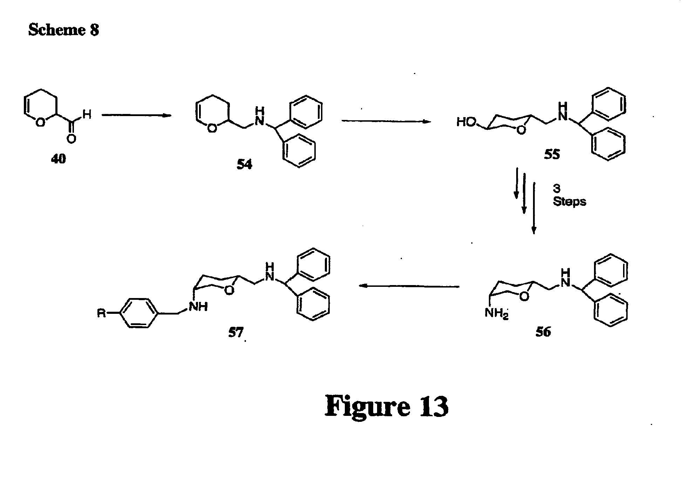

Figure 13 provides a scheme for the preparation of compounds an embodiment of the present invention;

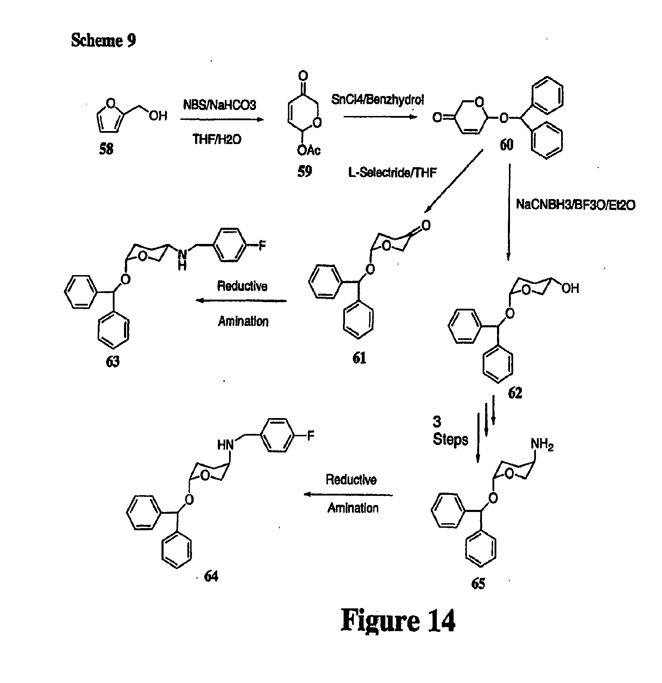

Figure 14 provides a scheme for the preparation of compounds an embodiment of the present invention;
Figure 15 provides a scheme for the preparation of compounds having formula VIb;
Figures 16A and 16B provide schema for the preparation of compounds having formula VII;
Figure 17 provides a scheme for the preparation of compounds having formulae VIIIa and VIIIb; and
Figure 18 provides a scheme for the preparation of compounds having formulae IXa and IXb.

### DETAILED DESCRIPTION OF THE PREFERRED EMBODIMENTS

3,6-disubstituted pyran derivatives have been discovered to be powerful agents targeting monoamine transporter systems. The pyran analogs are the bioisosteric versions of earlier structurally constrained cis-3,6-disubstituted piperidine derivatives which exhibited potent and selective affinities toward DAT in a stereo-selective manner, for Example compound 1b as shown below. The pyran series of compounds yielded results which indicate that the mode of interactions of these pyran molecules with monoamine transporters is different from their piperidine counterparts even though similar stereoselectivity, cis-configuration of most active piperidine 1a and pyran 1b, was maintained for optimal DAT activity in both cases.

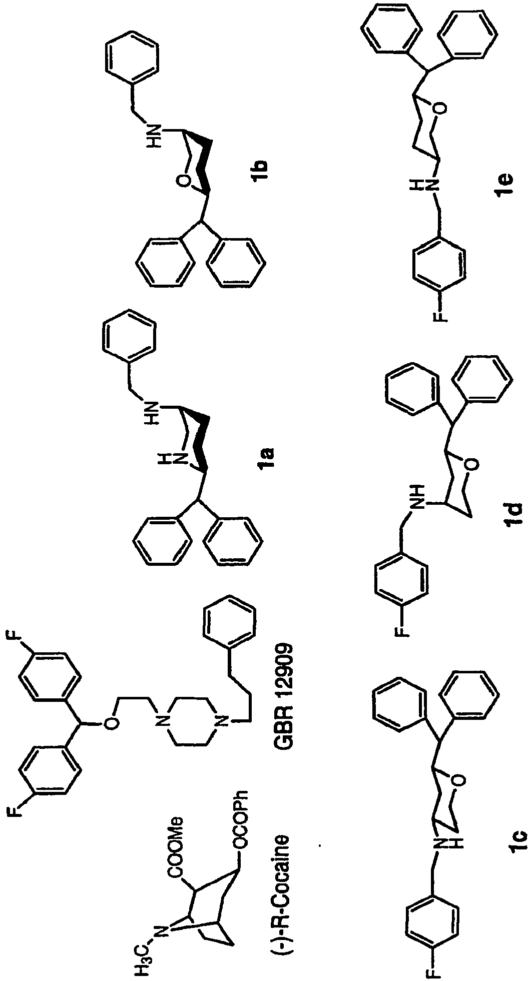

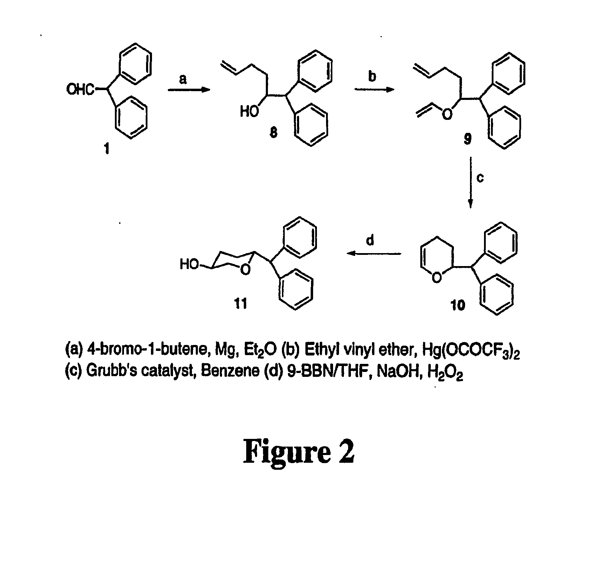

In general, there is a slight reduction of affinity in these pyran derivatives for the DAT compared to their piperidine counterparts (see Table 1). This loss of affinity could be due to the replacement of the basic N-atom in the piperidine derivative by a less basic O-atom resulting in an altered mode of interactions. In this regard, the cis-3,6-disubstituted pyran derivatives, as shown in structure 1b above, actually represent pharmacophoric structures for DAT interaction, as either cis- or trans-2,4-disubstituted and trans-3,6-disubstituted compounds, shown as 1c, 1d and 1e and Figure 2, were much weaker at DAT (see Table 1). Interestingly, one of the notable features observed in pyran derivatives bearing a potential H-bonding hydroxyl or amino functionality in the aromatic ring, was their significant increase of activity towards NET which was not observed for the corresponding piperidine counterparts. This affinity for NET is attributed to a formation of H-bonding between the functional groups in the benzyl moiety of the pyran molecules and the NET. Support for this came from the design of a molecule in which the original potential H-bonding bearing functional hydroxyl group connected to a phenyl moiety was modified into a bio-isosteric equivalent indole substituent where an indole amino moiety effectively replaced the hydroxyl group. The resulting indole derivative was also potent at NET, thus, confirming the potential involvement of an H-bond interaction.

**Table 1**

| Affinity of Drugs at Dopamine, Serotonin, and Norephinephrine Transporters in Rat Striatum | | | | |
|---|---|---|---|---|
| Compound | Inhibition of [³H]Win 35, 428 binding to DAT IC₅₀, nM,^{a} | Inhibition of [³H]citalopra m binding to SERT, IC₅₀, nM^{a}, | Inhibition of [³H]nisoxetin e binding to NET, IC₅₀, nM^{a} | Inhibition of [³H]DA1^{a} uptake by DAT, IC₅₀, nM, |
| GBR 12909 | 10.6 ± 1.9 | 132 ± 0 | 496 ± 22 | |
| 1c | 1,302 ± 68 | 3,313 ± 170 | 5,101 ± 1,037 | |
| 1d | 1,581 ± 283 | 4,778 ± 1,808 | 17,543 ± 2,153 | |
| 1e^{b} | 313 ± 71 | 8,410 ± 163 | 12,700 ± 3,180 | |
| 1b | 303 ± 14 | 1577 ± 97 | 274 ± 29 | 242 ± 39 |
| 1a | 114 ± 10.6 | 2130 ± 110 | 612 ± 130 | |

The present inventor contemplated that introduction of a hydroxyl group as a third substitutent in the inventive 3,6-disubstituted pyran templates could allow additional interaction with the monoamine transporter, potentially resulting in compounds with interesting activity and selectivity. While introducing such a hydroxy group in the pyran ring, it was also desired to explore the additional influence of stereospecificity and regioselectivity in the interaction of the pyran compounds with monoamine transporters. For this purpose, a novel asymmetric synthesis method via isomeric epoxide ring opening was used to introduce all three subsitutuents in a stereo- and regio-specific manner, followed by their biological evaluation at all three monoamine transporters.

The results of the work described above was the generation of a novel trisubstituted pyran template based on 3,6-disubstituted pyran derivatives. These trisubstituted derivatives represent a unique molecular template with a pyranyl backbone structure as blockers for monoamine transporters. Successful design and asymmetric synthesis of these analogs has been accomplished. The results indicate a clear separation of activity between enantiomers and demonstrate the presence of (2S, 4R, 5R) absolute configuration in the most active enantiomer for interaction with NET and SERT. It has been further surprisingly discovered that there are interesting differences in the activity profiles of these compounds, depending on the nature of the substitution on the phenyl ring of an N-benzyl moiety.

The compounds defined herein may be synthesized by methods known to chemists, in general. However, certain of the synthesis steps leading to stereoisomers of the trisubstituted pyrans are novel, and their use is also claimed herein. Several general reaction schemes are worthy of some discussion. Details of the synthesis and a more complete description of reaction schemes follows.

The compounds described herein are all potent inhibitors of monoamine transport, and exhibit reversible but strong binding affinities for the various monoamine transporters. However, some of the compounds exhibited preferable binding to the NET and/or SERT. This binding behavior places these compounds in a different category than analogues not containing a 3,6-substituted pyran ring system such as piperidine compounds with otherwise similar structure.

For example, *in vitro* data, which has been shown by many studies to correlate with *in vivo* activity, indicates that (-) isomers of the present invention are potent blockers for serotonin (SERT) and norepinephrine (NET) transporters. Compounds (-)29a, (-)-29e-, (-)29f, (-)32b and (-)37a are dual transport blockers as they bind to both the SERT and NET. Compounds of this class are known to those skilled in the art as SNRI (serotonin and norepinephrine reuptake inhibitors) and are considered potent anti-depressants. Compounds (-)29b, (-)29d, (-)32a, (+)32a, and (+)37a, are more selective for the NET and are known as NRI, also considered potent anti-depressants. Reboxetine, an NRI, was recently approved for use as an anti-depressant. SNRI are now considered to have favorable pharmokinetics as compared to SSRI (serotonin blocker only). Other disorders for which use of such compounds have been documented include panic disorder, post traumatic stress disorder, social phobia, and obsessive-compulsive disorder.

In one embodiment, the CNS-active compounds of the present invention are described by formulae VIa-d: 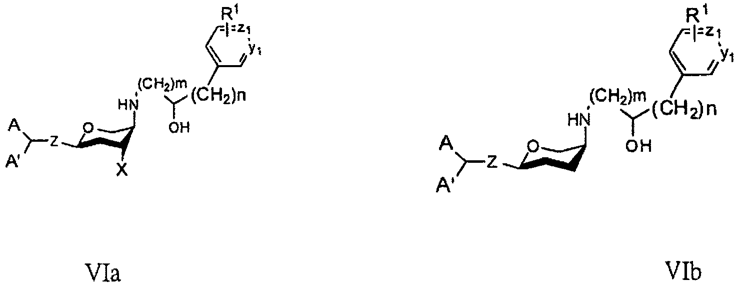 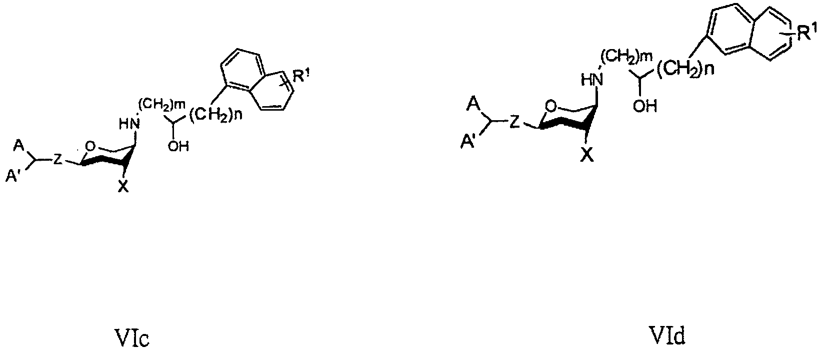 or a pharmaceutically acceptable derivative or salt thereof, wherein:
m and n are 0-4;
X is H, OH, NH₂, or NHR;
Z¹ and Y¹ are C- or N-atom;
Z is selected from the group consisting of a chemical bond and -Y-(CH₂)ₒ -;
Y is NH or O;
o is 0, 1, 2, 3, or 4; R is H, C₁₋₈ alkyl, C₂₋₈ alkenyl or C₂₋₈ alkynyl; at least one of A and A' are individually selected from the group of optionally substituted C₄-C₁₄ aryl and heteroaryl wherein heteroatoms of heteroaryl A and/or A' are selected from the group consisting of O, N and S,
   wherein at least one of A and A' are selected from the group consisting of: 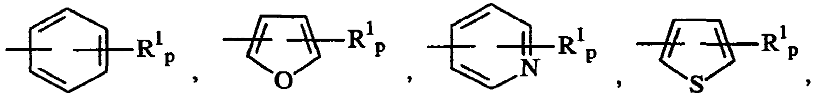 and 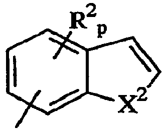
p is 0-6;
R¹ is C₁₋₄ alkyl, C₂₋₆ alkenyl, C₂₋₆ optionally halogenated alkynyl, C₂₋₆ hydroxyalkynyl, halo, -CN, -COOR⁴, -OH, -NO₂, -NH₂, -NHR⁴, -SO₂NH₂, - NHSO₂R⁸, -OCF₃, or -OR⁸;
R² has the meaning of R¹ and also a 5 or 6 membered heterocycle containing 1 or more heteroatoms selected from the group consisting of N, O, and S;
X² is N, O, or S;
R⁴ is H, C₁₋₁₈ alkyl, C₅₋₁₀ cycloalkyl, or C₂₋₁₈ alkylene; and
R⁸ is C₁₋₈ alkyl, C₅₋₆ cycloalkyl, C₂₋₈ alkenyl, or a 5 or 6-member aromatic ring including heterocyclic rings.

In another variation of the compounds having formula I, the CNS-active compounds of the present invention are described by formulae VII: 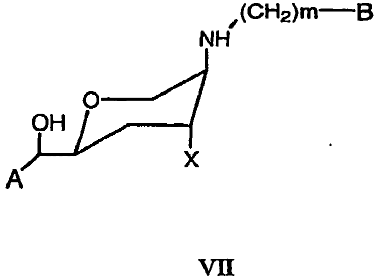 or a pharmaceutically acceptable derivative or salt thereof, wherein:
m is 0-4;
X is - H, -OH,- NH₂, or -NHR;
at least one of A and A' are selected from the group consisting of: 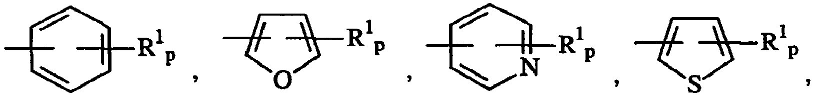 and 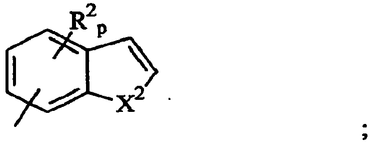
p is 0-6;
R¹ is C₁₋₄ alkyl, C₂₋₆ alkenyl, C₂₋₆ optionally halogenated alkynyl, C₂₋₆ hydroxyalkynyl, halo, -CN, -COOR⁴, -OH, -NO₂, -NH₂, -NHR⁴, -SO₂NH₂, - NHSO₂R⁸, -OCF₃, or -OR⁸;
R² has the meaning of R¹ and also a 5 or 6 membered heterocycle containing 1 or more heteroatoms selected from the group consisting of N, O, and S;
X² is N, O, or S;
R⁴ is H, C₁₋₁₈ alkyl, C₅₋₁₀ cycloalkyl, or C₂₋₁₈ alkylene; and
R⁸ is C₁₋₆ alkyl, C₅₋₅ cycloalkyl, C₂₋₈ alkenyl, or a 5 or 6-member aromatic ring including heterocyclic rings; and
B is selected from the group consisting of

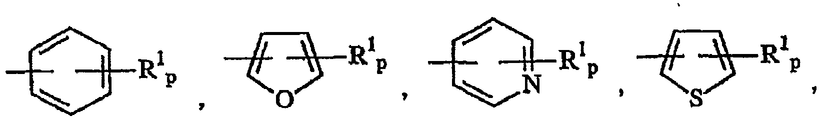

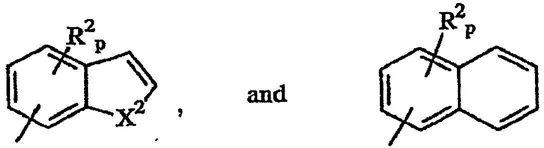

The subject invention compounds may be used as such or in the form of their pharmaceutically acceptable derivatives and/or salts. By the term "derivative" is meant a chemically modified form of the "base compound" which will liberate an active form of the base compound or metabolite thereof following administration, and does not include salts of the base compound. However, derivatives may also, when appropriate, also be used in the form of salts. The particular type of derivative is dependent, in most cases, on the nature of functional group(s) present on the base compound or its salt, and selection of a suitable derivative is within the skill of the art. For example, when hydroxyl groups are present, ethers or esters are common derivatives, especially the latter, as are also carbamates.

In general, the derivative is hydrolyzable to the base compound *in vivo* or is enzymatically converted, in one or more steps, to the base compound (or a salt thereof). In the case of primary or secondary amino groups, common derivatives include amides, imides, ureas, and the like. Preparation of all these derivatives may take place by standard methods of organic chemistry. Simple esters may be produced from hydroxyl groups by esterification with a carboxylic acid, sulfonic acid, etc., a carboxylic acid anhydride, a carboxylic acid chloride, etc. Carbamates may be prepared by reaction with an organic isocyanate.

Further derivatives include inclusion compounds and clathrates, for example inclusion complexes formed from the contact of host molecules such as α, β, and γ-cyclodextrins, or chemically modified cyclodextrins well known to the art. Urea inclusion compounds are also derivatives. In these derivatives, the gurst molecules (base compounds) are not chemically bound, but are present due to molecular attraction, hydrogen bonding, surface energy effects, etc. In general, such complexes are stoichiometric, but non-stoichiometric complexes may also be used. Such complexes are easily prepared by one skilled in the art. For example, cyclodextrin complexes may be prepared by kneading together cyclodextrin and base compound in water followed by removal of free water.

Salts are most useful forms of the subject invention compounds, and are formed by the neutralization of basic nitrogen atoms in the base compound by an organic or inorganic acid. Useful organic acids are in particular carboxylic acids and sulfonic acids. Examples of mono-, di-, and polycarboxylic acids which are useful include formic acid, acetic acid, propionic acid, butyric acid, maleic acid, fumaric acid, malic acid, tartaric acid, citric acid, succinic acid, sulfosuccinic acid, tannic acid, and the like. An example of a sulfonic acid is toluene sulfonic acid. Examples of inorganic acids include hydrochloric acid, sulfuric acid, nitric acid, phosphoric acid, polyphosphoric acid, molybdic acid, nitrous acid, sulfurous acid, and the like. The salts are prepared by simply neutralizing the base compound all or in part, generally in aqueous solution. In such cases, water of hydration may be a part of the salt thus produced.

The compounds may be administered by any suitable technique, including intravenous administration, but are preferably administered in solid form, for example as a tablet or capsule, optionally in conjunction with conventional pharmaceutical additives such as tableting aids, lubricants, fillers, pH-adjusting substances, pH-regulating substances (buffers), emulsifiers, dispersing aids, antioxidants, UV-stabilizers, etc. Such ingredients are well known. The compositions may also be administered in other forms, such as syrups, dispersions, etc.

The dosage to be administered to a mammalian species is dependent on numerous factors such as the particular species, its weight, the type of disorder, the desired degree of treatment, and the individual itself. Dosages can be readily determined by one skilled in the art by routine tests, for example time/serum level measurements, dose/response curves, etc. The dosages are in particular easy to range, as numerous monoamine transport-affecting drugs are commercially available, have extensive *in vitro* and *in vivo* results presented in the literature, or are in clinical trials. This is true for both human and non-human subjects, anti-anxiety medication being common for use in domestic dogs and cats, for example.

Dosage ranges which are useful also vary with respect to the activity of the individual compounds, for example the measured *in vitro* or *in vivo* activities reported in Tables 1 to 5 herein, as well as whether the compound is administered in a fast or slow release formulation, its solubility, its rate of transfer into the plasma or into the extracellular space, etc. Preferable serum concentrations range from 200 ng/mL to 80 ng/mL, more preferably 180 ng/mL to 85 ng/mL, with the foregoing constraints in mind. In non-slow-release formulations, dosages for the average mammal may range from 0.05 mg/Kg of body weight to about 10 mg/Kg of body weight, more preferably 0.1 mg/Kg to 5 mg/Kg. Slow release formulations will involve greater amounts of active ingredient.

### Chemistry

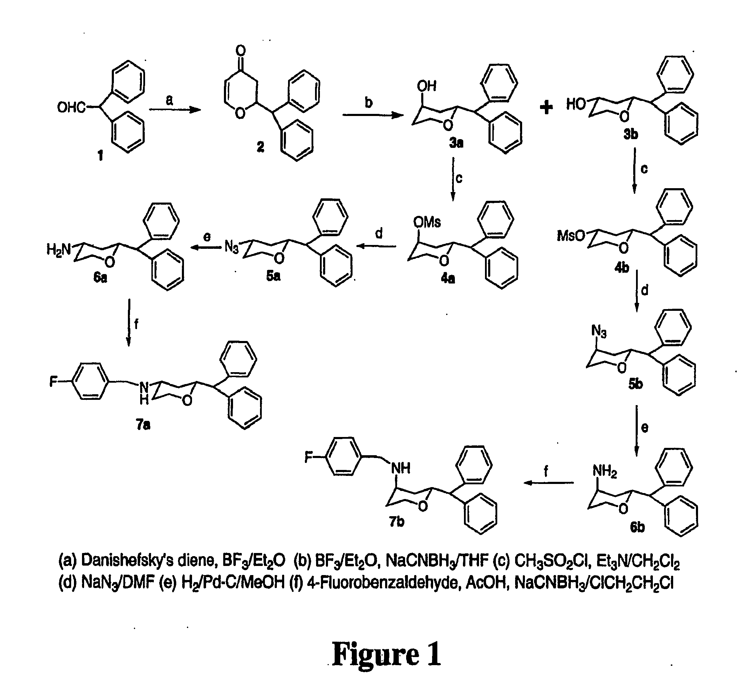

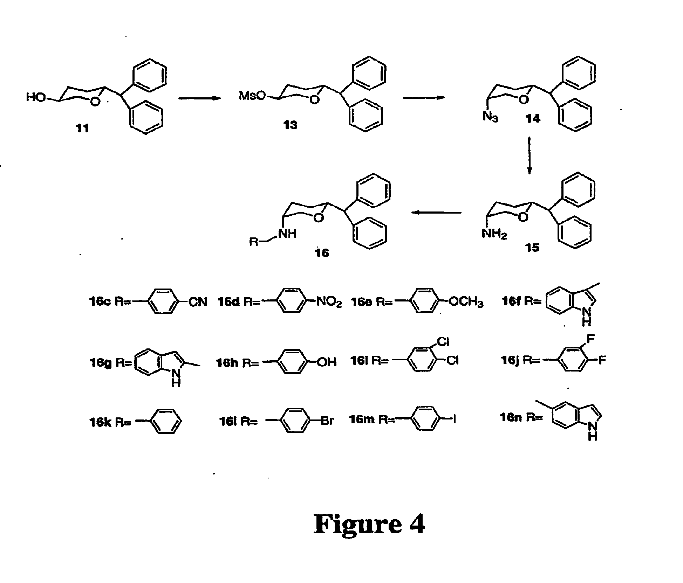

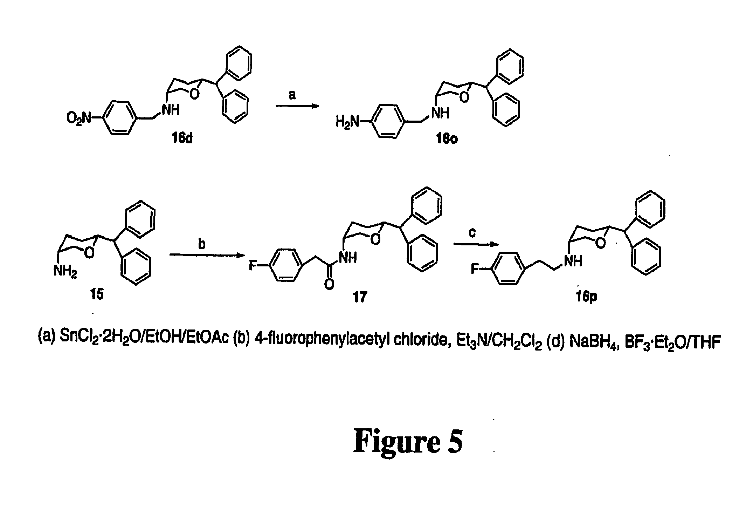

Target compounds **7a,b** and **16a-p** were synthesized by following synthetic procedures shown in **Scheme 1** to **Scheme 5** depicted in Figures 1 - 5.

Synthesis of the target compounds **7a** and **7b,** shown in **Scheme 1,** was accomplished in high yields by following efficient synthetic routes. The basic pyranose ring structure in compound **2** was achieved by [4+2] Hetero-Diels-Alder cycloaddition **(a)** of Danishefsky's diene and aldehyde **1** in the presence of BF₃·Et₂O which produced **2** in 80% yield. Reduction of **2** with NaCNBH₃ in presence of BF₃-Et₂O in THF **(b)** produced racemic cis- and trans-mixture **of 3a** and **3b** (2.5: 1) in 96% yield. The two isomers were separated by careful flash chromatography, and their structures were assigned by NMR and NOE. Compounds **6a** and **6b** were synthesized from **3a** and **3b** respectively in high yields by three steps **(c,d,e)** which involve first mesylation with methanesulfonyl chloride in dry dichloromethane to produce **4a** and **4b,** followed by treatment with sodium azide in DMF with inversion of configuration to produce azides **5a** and **5b.** This azido displacement reaction resulted in production of the cis-isomer **5a** from *trans*-4a and the trans-isomer **5b** from *cis-***4b.** Finally, catalytic hydrogenation of the azides **5a** and **5b** with Pd/C produced the amine precursors **6a** and **6b** in good yield. Reductive amination **(f)** of 6a and **6b** furnished **7a** and **7b,** respectively, in 72.6% and 54% yield.

**Scheme 2** delineates the preparation of the key pyran 3,6-disubstituted intermediate **11** with trans-stereochemistry. Briefly, aldehyde 1 was converted (a) into **8** by reacting with an *in situ* prepared Grignard reagent prepared from 4-bromo-1-butene and magnesium in dry ether, in 91% yield. O-vinyllation of **8** with ethyl vinyl ether (b) in the presence of Hg(OCOCF₃)₂ at room temperature produced **9** in 66 % yield. Ring closing metathesis (c) of **9** in presence of a Grubb's catalyst in refluxing benzene afforded olefin **10** in 92.6% yield. Hydroboration of **10** with 9-BBN in THF, followed by oxidation (d) gave exclusively *trans*-isomer **11** in 93.5% yield. Compound **11** was used next as a starting precursor for the synthesis of various derivatives with different substitutions at the exocyclic N-atom as shown in the scheme **3** and scheme **4.**

First, as shown in scheme **3,** compound **11** was subjected to a Swern oxidation reaction (a) which produced ketone **12** in 91 % yield. Reductive amination of **12** with 4-fluorobenzylamine (b) produced **16a** as a major product in 45% yield. As described in the synthesis of compound **6a-b** in **Scheme 1,** compound **11** was next converted as shown in **Scheme 4,** into a cis-amine intermediate **15** via three steps consisting first, of mesylation with methanesulfonyl chloride in dry dichloromethane (a), followed by substitution with sodium azide in DMF (b), and finally, catalytic hydrogenation with Pd-C in methanol (c). Reductive amination of **15** with various aldehydes (d) furnished target compounds **16b-n** in good yield ***(*Scheme 4**).

The synthesis of compounds **160** and **16p** is described in **Scheme 5. 160** was synthesized by the reduction of **16d** with tin(II) chloride dihydrate in ethanol and ethyl acetate in 60% yield (a). Amide Intermediate **17** was obtained from the reaction of amino-compound **15** with 4-fluoro-phenylacetyl chloride (b). Reduction of **17** with freshly generated borohydrate (c) gave the target compound **16p.**

Following synthesis of 2,4-disubstituted cis and trans compounds **7a** and **7b,** they were characterized in binding assays for the three monoamine transporters (Table 2). Note that Table 2 contains data from numerous compounds and is more extensive in this regard than Table 1. In Table 1, compounds 1c, 1d, 1e, and 1b, are compounds 7a, 7b, 16a, and 16k of Table 2, respectively. Results indicated that the positional change from 3,6-disubstitution to 2,4-disubstitution adversely affected the binding activity of these two molecules. It is interesting to note that even though the activity of the 2,4-species was low, the preferential affinity for the DAT was still exhibited in the cis version. These results unexpectedly confirmed that the cis-3,6-disubstituted pyran template is a basic pharmacophoric requirement for interaction with DAT.

In the 3,6-disubstituted, replacement of a fluoro-substituent in the "B" aryl moiety by electron withdrawing substituents resulted in more potent compounds for the DAT as illustrated in the cyano-substituted molecule 16c and nitro-substituted molecule **16d.** Nitro-substitution produced the most active compound among these synthesized analogs for the DAT (IC50 = 38.3 nM). Surprisingly, however, the electron donating methoxy substitutent in **16e** produced comparable potency at the DAT (IC₅₀ = 84 nM). Introduction of 3,4-difluoro substituents in **16j** reduced potency at all three transporters compared to the 4-fluoro **16b.** With the dichlorosubstituted compound **16i,** no improvement in activity was observed compared to unsubstituted **16k,** indicating no correlation with, and a different mode of binding interaction of, as compared to tropane- and methylphenidate-type of compounds. As far as other halogen derivatives are concerned, the bromo compound **16l** exhibited somewhat higher activity at DAT compared to unsubstituted **16k** whereas the iodo compound 16m displayed comparable potency.

Compared to the methoxy substituted compound 16e, the hydroxy substituted compound **16h** retained the activity at DAT (IC50 = 78.4 nM for 16h and IC50 = 84nM for **16e**), but its selectivity was shifted in favor of NET shown by the much higher activity at NET (IC50 = 22.6 nM for the NET, NET/DAT = 0.29) (Table 3). The amino-substituted compound **16o** also exhibited high potency at NET. These two substitutents can act as both hydrogen-bond donor or acceptor site, although in different capacity. The big shift towards activity and selectivity at NET caused by these two polar substitutents might indicate a critical involvement of hydrogen bond in interaction with NET. Similar results were not observed in structurally constrained piperidine analogs, reflecting the existence of different interaction modes between these two templates, and again confirming the unpredictability as between these respective classes of compounds. Since a high degree of homogeneity has been demonstrated between the DAT and NET structural sequence, it is highly surprising to observe that a subtle change in pyran structure can induce differential interactions in favor of the NET.

The nature of hydrophobic interaction of the aromatic moiety, was investigated by replacing the phenyl aromatic moiety in the benzyl group by bioisosteric indole moieties. Thus, replacement with a 2- and 3-indole moiety as illustrated in compounds **16g** and **16f,** led to moderate to diminished potency at DAT. Interestingly, the 2-indole substituted derivative **16g** was 3.5 fold more active at DAT compared to the 3-substituted **16f** (227 vs. 794 nM) and was also more active than the unsubstituted **16k.** A similar increase in affinity for the NET was also observed for the 2-substituted indole compared to the 3-substituted compound (401 vs. 1860 nM). To assay the importance of the position of the indole N-atom along with hydrophobic interaction, the 5-substituted indole derivative **16n** was designed and synthesized. In this regard, 5-substitution was chosen as it will assume the bioisosteric configuration of the p-hydroxyphenyl moiety of **16h.** The binding results for **16n** indicated high affinity, similar to **16h,** for the NET, indicating the involvement of H-bonding with the indole amino moiety. This result further demonstrates the existence of an H-bond donor or acceptor site in the NET which, when oriented correctly with respect to ligand's H-bond forming functionality, can provide potent interaction.

In compound **16p,** the fluorobenzyl moiety was replaced by a 4-fluorophenylethyl moiety which did not result, surprisingly, in decreased activity at DAT compared to **16b,** in contrast to the results observed in constrained piperidine counterparts where a drop in DAT activity resulted from such modification. This result likely indicates that a different pharmacophoric orientation is required, probably via a distance geometry approach, to produce optimum activity in the pyran template. As we expected, exocyclic-N-substituion with an aromatic moiety is necessary in pyran derivatives for their activity at the monoamine transporter systems, as compound 15 exhibited little or no activity at the DAT.

Selected compounds with relatively higher activity at the DAT were tested in the DA uptake assay. For the most part no differential uptake and binding activity was observed with the exception of compound **16d** which showed a three fold higher potency in inhibiting binding than uptake.

In order to demonstrate a difference in spatial distribution in the lowest energy conformers between 3,6-disubstituted and 2,4-disubstituted pyran derivatives, a preliminary molecular modeling study was performed. 2,4-Disubstituted compound **7a** and the 3,6-disubstituted compound **16b** were chosen for this study. Compounds were minimized first with the SYBYL molecular modeling program (version 6.9, 2002, Tripos Associates, Inc., St. Louis, MO), On a Silicon Graphics Octane IRIX 6.5 workstation. Minimized molecules obtained from this operation were next subjected to a grid search protocol to search for the lowest energy conformer.

First, each structure was fully minimized using standard Tripos force field with a distance dependent dielectric function, a 0.05 Kcal/mol Å energy gradient convergence criterion was used and the six-membered pyran ring was treated as an aggregate. The Powell method was used during minimization, and charges were computed using the Gasteiger-Huckel method within Sybyl 6.9. The number of iterations was 1000. After minimization the energy for 2,4-disubstituted molecule **7a** was 5.85 Kcal/mol and the energy for 3,6-disubstituted molecule **16b** was 5.63 Kcal/mol.

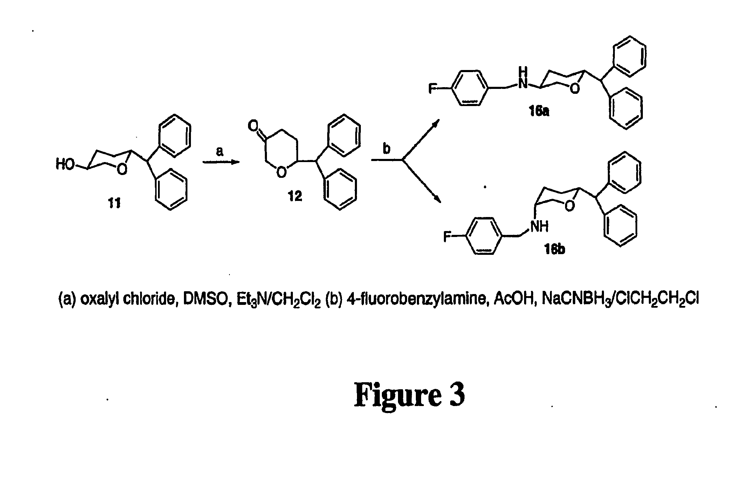

In the next step, using grid search protocol, the conformational search on each minimized molecule was performed by rotating the torsion angle of compounds **7a** and **16b** formed by atoms α-β-γ-δ (see Figure 3) from 0° to 360° by 10° increments. This method was used to perform a simple systematic search such that each specified torsion angle is varied over a grid of equally spaced values. While searching for the lowest energy conformer, a cutoff value of 8 Kcal/mol was specified relative to the lowest conformer, and charges were computed using the Gasteiger-Hückel method. Also, the six-membered pyran ring was treated as an aggregate. For compound **7a,** a conformer with torsional angle 77.8°C was found to have lowest energy, 3.16 Kcal/mol, whereas compound **16b** produced lowest energy 5.61 Kcal/mol with a torsion angle 300°. These two lowest energy conformers were used next for overlapping.

In the final step, the two minimized structures were overlapped. During overlapping, the alignment program within Syby16.9 was employed, and the method used was common structure method. The compound **16b** was used as template molecule and the six-membered pyran ring was used as common substructure for overlapping.

The pharmcophoric activity of the cis-3,6-disubstituted tetrahydropyran template at monoamine transporter systems was thus confirmed by SAR exploration with this template with various substituents on the exocyclic N-atom, producing potent activities at both DAT and NET. Compound **16d** with the electron withdrawing nitro-substituent turned out to be the most active for the DAT. Interestingly, the compounds **16h** and the **16o** with para-hydroxy and para-amino substituents exhibited high potency for the NET, indicating formation of H-bonding. This was further confirmed by the bioisosteric version **16n** which exhibited strong selective potency at NET. The SAR results for the current pyran molecules do not correspond with those for otherwise analogous piperidine derivatives, indicating differential interaction modes with monoamine transporters.

In still another embodiment of the present invention, the compounds of the invention can be used in a method of reducing monoamine reuptake in a mammalian species. The method comprises administering a binding amount of a monoamine receptor binder comprising at least one compound of the invention set forth above. In a variation of this embodiment, a compound of the invention can be used in a method for treating a patient exhibiting signs of depression, in an amount effective to inhibit reuptake of serotonin at the SERT and norepinephrine at the NET. In still another variation of this embodiment, a compound of the invention can be used in a method for treating signs of depression, in an amount effective to inhibit norepinephrine reuptake at the NET.

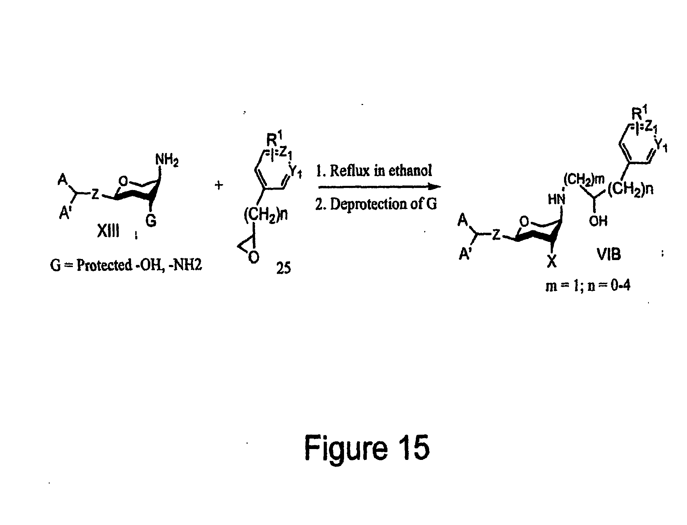

Figure 15 provides a scheme for the preparation of compounds having formula VIb. Preparation of compounds having formulae VIa, VIc, and VId is accomplished by analogous methodology.

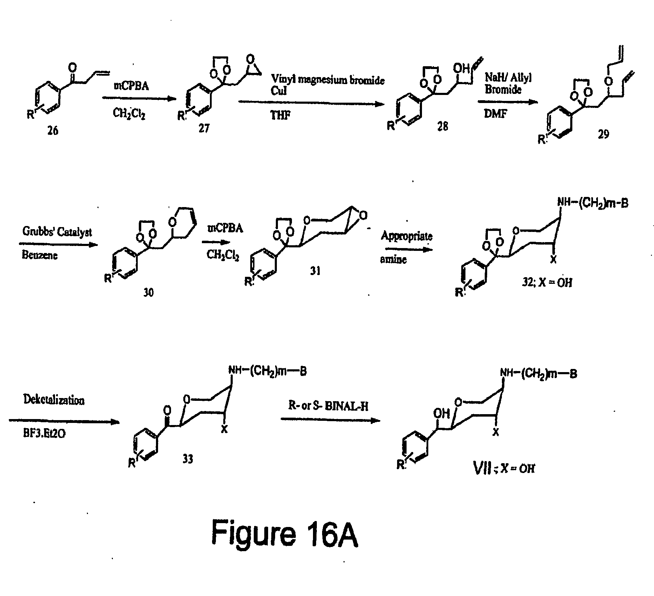

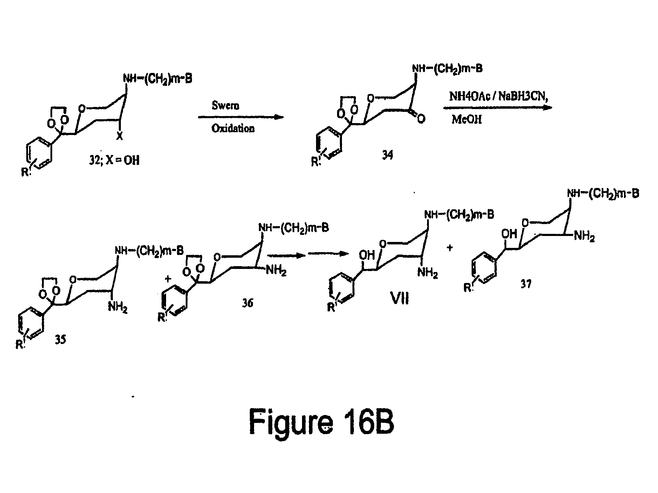

Figures 16A and 16B provide a scheme for the preparation of compounds having formula VII. Appropriate starting material 26 is converted into intermediate 31. Epoxide ring opening followed by deketalization produces intermediate 33. Chiral reduction is next provide asymmetric target compounds.

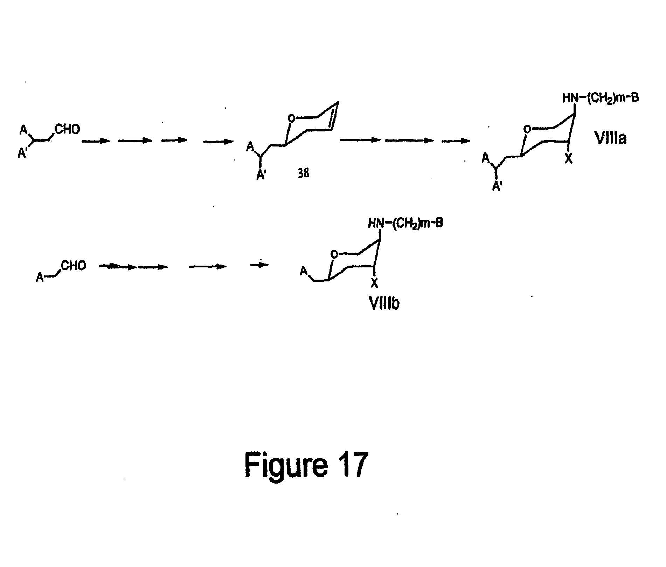

Figure 17 provides a scheme for the preparation of compounds having formulae VIIIa and VIIIb.

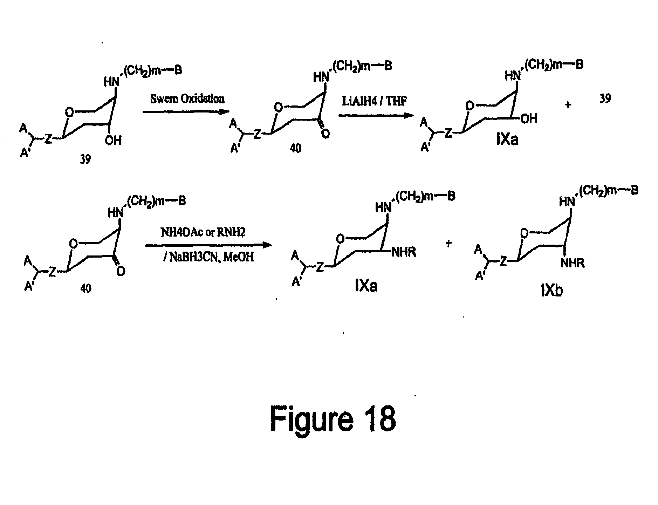

Figure 18 provides a scheme for the preparation of compounds having formulae IXa and IXb. The synthesis will start from a suitable starting material represented by structure 39.

### Experimental Details

Reagents and solvents were obtained from commercial suppliers and used as received unless otherwise indicated. Dry solvent was prepared according to the standard procedure as described by Vogel. All reactions were performed under inert atmosphere (N₂) unless otherwise noted. Analytical silica gel-coated TLC plates (Si 250F) were purchased from Baker, Inc. and were visualized with UV light or by treatment with phosphomolybdic acid (PMA). Flash chromatography was carried out on Baker Silica Gel 40 mM. ¹H NMR spectra were routinely obtained with GE300 MHz and 400 MHz FT NMR. The NMR solvent used was CDCl₃ as indicated. TMS was used as an internal standard. Elemental analyses were performed by Atlantic Microlab, Inc and were within ± 0.4% of the theoretical value, but are not reported herein for reasons of brevity.

[³H]WIN 35,428 (86.0 Ci/mmol), [³H]nisoxetine (80.0 Ci/mmol) and [³H]dopamine (48.2 Ci/mmol) were obtained from Dupont-New England Nuclear (Boston, MA, U.S.A). [³H]citalopram (85.0 Ci/mmol) was from Amersham Pharmacia Biotech Inc. (Piscataway, NJ, U.S.A.). Cocaine hydrochloride was purchased from Mallinckrodt Chemical Corp. (St. Louis, MO, U.S.A.). WIN 35,428 napthalene sulfonate was purchased from Research Biochemicals, Inc. (Natick, MA, U.S.A.). (-)-Cocaine HCl was obtained from the National Institute on Drug Abuse. GBR 12909 Dihydrochloride (1-[2-[bis(4-Fluorophenyl)-methoxy]ethyl]-4-[3-phenylpropyl]piperazine) was purchased from SIGMA-ALDRICH (#D-052; St. Louis, MO).

### Synthesis of 2-benzhydryl-2,3-dihydro-4H-pyran-4-one (2)

A solution of boron trifluoride diethyl etherate (7.8 g, 55 mmol) in dry ether (50 ml) was added to a stirred mixture of E-1-methoxy-3-trimethylsilyloxybuta-1,3-diene(8.3 g, 48 mmol), diphenylacetaldehyde 1 (11.4 g, 58 mmol) and dry ether (300 ml) cooled to -78°C. After one hour, the mixture was allowed to reach 0°C for three hours. The deep red reaction mixture was quenched with saturated aqueous NaHCO₃, and the mixture was allowed to come to room temperature. The organic phase was separated and the aqueous phase was extracted with ether (3x70 ml). The combined organic phases were washed with brine, and dried over anhydrous Na₂SO₄. Evaporation of solvent under reduced pressure and purification of the crude product by chromatography (hexane/ethyl acetate 8:2) gave 2-diphenylmethyl-2,3-dihydro-4H-pyran-4-one 2 (10.2 g, 80.2%, yield) as a yellow solid.

¹H NMR(400Mhz, CDCl₃) 2.38(dd, J=3.2Hz, 16.8Hz, 1H, H-3) 2.51(m, 1H, H-3) 4.23(d, J=9.2Hz, 1H, (Ph)₂CH) 5.15(dt, J=3.2Hz, 8.8Hz, 1H, H-2) 5.44(d, J=6.4Hz, 1H, H-5), 7.16-7.38(m, 11H, H-6, aromatic-CH).

### Synthesis of Cis and Trans-2-benzhydryl-tetrahydropyran-4-ol 3a and 3b

NaCNBH₃ (0.75 g, 12 mmol) was added portionwise to a mixture of 2-diphenylmethyl-2,3-dihydro-4H-pyran-4-one **2** (1.05 g, 4 mmol) and boron trifluoride etherate(1.99 g, 14 mmol) in dry THF(50 ml) cooled to -78°C. The reaction mixture was allowed to reach room temperature and the reaction was quenched with saturated aqueous NaHCO₃ (30 ml). The organic phase was separated, and the aqueous phase was extracted with ethyl ether (3 x 20 ml). The organic phases were combined and dried over anhydrous Na₂SO₄. Removal of the solvent under reduced pressure, and purification by flash chromatography (hexane/ethyl acetate 7:3) first afforded ***trans*-2-benzhydryl-tetrahydropyran-4-ol 3a** (0.73 g, 68% yield).

¹H NMR(400MHz, CDCl₃) 1.22(q, J=12Hz, 1H, H-3ax) 1.46(dq, J=4.8Hz, 12 Hz, 1H, H-5ax) 1.74-1.86(m, 2H, H-3eq, H-5eq) 3.40(dt, J=2Hz, 12Hz, 1H, H-6ax) 3.707(m, 1H, H-4) 3.941-4.039(m, 2H, H-6eq, (Ph)₂CH) 7.15-7.4(m, 10H, aromatic-CH).

Eluted second was *cis-***2-benzhydryl-tetrahydropyran-4-ol, 3b** (0.3 g, 28.1 % yield).

¹H NMR(400MHz, CDCl₃) 1.5-1.58(m, 4H, H-3, H-5eq, OH) 1.84(m, 1H, H-5ax) 3.79(m, 1H, H-6eq) 3.876(d, J=8.8Hz, (Ph)₂CH) 3.908(dt, J=3.2Hz, 111.2Hz, 1H, H-6ax) 4.184(m, 1H, H-4eq) 4.524(dt, J=4Hz, 8.8Hz, 1H, H-2) 7.16-7.38(m, 10H, aromatic-CH).

### Procedure A. Synthesis of methanesulfonic acid

### Trans-2-benzhydryl-tetrahydropyran-4-yl ester 4a

Methanesulfonyl chloride (0.62 g, 5.41 mmol) in dry methylene chloride (10 ml) was added dropwise to a mixture of *trans*-2-diphenylmethyl-4-hydroxypyran 3a (0.73 g, 2.70 mmol), triethylamine (0.41 g, 4.06 mmol) in methylene chloride (10 ml) and was cooled to 0°C. After one hour, the reaction was gradually allowed to reach room temperature over a period of four hours. Additional methylene chloride (20 ml) was added to the reaction mixture, and the mixture was washed in turn with saturated aqueous sodium bicarbonate, brine and water, then dried over anhydrous sodium sulfate. The solvent was removed under reduced pressure, and purification by flash chromatography gave compound 4a (0.93 g, 99.9% yield) as an oil.

¹H NMR (300mHz, CDCl₃): 1.54 (m, 1H, H-3ax) 1.82 (m, 1H, H-5ax) 1.95(m, 1H, H-3eq) 2.1(m,1H, H-5eq) 2.95(s, 3H, CH₃SO₂) 3.46(dt, 1H, H-6ax) 3.96(d, 1H, (Ph)₂CH) 4.1(m, 2H, H-2, H-6eq) 4.83(m, 1H, H-4) 7.15-7.38(m, 10H, aromatic-CH).

### Synthesis of methanesulfonic acid cis-2-benzhydryl-tetrahydropyran-4-ylester 4b

*Cis*-2-diphenylmethyl-4-hydroxy-pyran **3b** (0.3 g, 1.12 mmol) was reacted with methanesulfonyl chloride (0.26 g, 2.24 mmol) (Procedure A) to give compound **4b** (0.38 g, 98%) as an oil.

¹H NMR (300MHz, CDCl₃): 1.609(m, 1H, H-3ax) 1.8-1.96(m, 4H, -OH, H-3eq, H-5) 2.96(s, 3H, CH₃SO₂) 3.8-3.94(m, 3H, H-6, (Ph)₂CH) 4.46(dt, J=2Hz, 10Hz, 1H, H-2) 5.1(m, 1H, H-4) 7.16-7.38(m, 10H, aromatic-CH).

### Procedure B. Synthesis of cis-4-azido-2-benzhydryl-tetrahydropyran (5a)

Into a solution of *trans*-2-diphenylmethylpyran-4-yl methanesulfonate **4a** (0.33 g, 0.95 mmol) in dry DMF (40 ml) was added sodium azide (0.18 g, 2.85 mmol). The mixture was heated to 100°C and stirred for 4 hr. The mixture was diluted with ethyl ether, washed with 2M aqueous NaHCO₃ and brine, and then dried over anhydrous Na₂SO₄. Removal of the solvent and purification by flash chromatography (Hexane/Ethyl Acetate 9: 1) afforded compound Sa (0.23 g, 82.7% yield) as a liquid.

¹H NMR (400MHz, CDCl₃) 1.5-1.68 (m, 3H, H-3, H-5eq) 1.855(m, 1H, H-5ax) 3.74-3.86(m, 2H, H-6) 3.87(d, J=9.2Hz, 1H, (Ph)₂CH) 4.02(m, 1H, H-4) 4.393(dt, J=3.2Hz, 13Hz, 1H, H-2) 7.16-7.38(m, 10H, aromatic-CH).

### Synthesis of trans-4-azido-2-benzhydryl-tetrahydropyran 5b

Cis-2-diphenylmethylpyran-4-yl methanesulfonate **4b** (0.38 g, 1.10 mmol) was reacted with sodium azide (0.29 g, 4.4 mmol) in dry DMF (Procedure B) to yield compound **5b** (0.26 g, 80%) as a liquid.

¹H NMR(500MHz, CDCl₃) 1.32(q, J= 11Hz, 1H, H-3ax) 1.61(dq, J=5.5Hz_{,} 13Hz, 1H, H-5ax) 1.82(m, 1H, H-3eq) 1.90(m, 1H, H-5eq) 3.44-3.50(m, 2H, H-4, H-6ax) 3.96(d, J=8.5Hz, 1H, (Ph)₂CH) 4.03(dt, J=2Hz, 9Hz, 1H, H-2) 4.08(ddd, J=2Hz, 5.5Hz, 12.5Hz, 1H, H-6eq) 7.16-7.38(m,10H, aromatic-CH).

### Procedure C. Synthesis of cis-(2-benzhydryl-tetrahydropyran-4-yl)-amine (6a)

*Cis*-4-azido-2-diphenylmethyltetrahydropyran **5a** (0.23 g, 0.78 mmol) was hydrogenated (60 psi) in the presence of 10% Pd-C (0.02 g, 10%wt) for 4hr. The reaction mixture was filtered through a short bed of celite, and removal of the solvent afforded 0.21 g (quantitative yield) of product. This product was pure enough to continue to the next reaction step.

¹H NMR(300MHz, CDCl₃) 1.21-1.4(m, 4H, H-3, NH₂) 1.59(m, 1H, H-5ax) 1.87(m, 1H, H-5eq) 3.37(m, 1H, H-4) 3.77(m, 1H, H-6eq) 3.91(dt, J=2.4Hz, 11.7Hz, 1H, H-6ax) 3.94(d, J=9.3Hz, 1H, (Ph)₂CH) 4.56(dt, J=2.4Hz, 10.2Hz, 1H, H-2) 7.16-7.38(m, 10H, aromatic-CH).

### Synthesis of Trans-(2-benzhydryl-tetrahydropyran-4-yl)-amine (6b)

*Trans*-4-azido-2-diphenylmethyltetrahydropyran **5b** (0.26 g, 0.89 mmol) was hydrogenated (Procedure C) to yield compound **6b** (0.24 g, quantitative).

¹H NMR(400MHz, CDCl₃) 1.15-1.25(m, 1H, H-3) 1.4-1.52(m, 1H, H-3) 1.7-1.88(m, 2H, H-5) 2.99(m, 1H, H-4) 3.41(dt, J=2Hz, 12.4Hz, 1H, H-6ax) 3.9-4.06(m, 3H, H-2, H-6ax, (Ph)₂CH) 4.7(bs, 2H, NH₂) 7.16-7.38(m, 10H, aromatic-CH).

### Procedure D. Syntheis of cis-(2-benzhydryl-tetrahydropyran-4-yl)-(4-fluorobenzyl-amine (7a)

To a solution of cis-4-amino-2-diphenylmethyl pyran 6a (0.2 g, 0.75 mmol), 4-flurobenzaldehyde (0.83 g, 0.67 mmol) and glacial acetic acid (0.45 g, 0.75 mmol) in 1,2-dichloroethane (20 ml), was added portion wise NaCNBH₃ (0.57 g, 0.9 mmol) dissolved in methanol (5 ml). After 4hr, water was added to quench the reaction and the mixture was stirred for 30 minutes at 0°C. Then the mixture was made basic with saturated aqueous NaHCO₃ and extracted thrice with methylene chloride (3 x 30 ml). The combined organic phases were washed with brine, water and dried over anhydrous Na₂SO₄. Solvent was removed *in vacuo* to collect the crude residue, which was purified by flash chromatography (Hexane/Ethyl Acetate/Triethylamine 3:2:0.2) to give *cis*-2-diphenylmethyl-4-(4-flurobenzylamino)-tetrahydropyran 7a (0.20 g, 72.6%) as a liquid.

¹H NMR (400MHz, CDCl₃) 1.24(bis, 1H, -NH) 1.28(m, 1H, H-3) 1.45-1.58(m, 2H, H-3, H-5eq) 1.83(tt, J=4Hz, 13Hz, 1H, H-5ax) 3.07(m, 1H, H-4) 3.65(s, 2H, (F)Ph-CH₂) 3.75(m, 1H, H-6eq) 3.91(d, J=9.6Hz, 1H, (Ph)₂CH) 3.94(dt, J=2.4Hz, 12Hz, 1H, H-6ax) 4.59(dt, J=3.2Hz, 9.6Hz, 1H, H-2) 6.9-7.4(m, 14H, aromatic-CH).

The free base **7a** was converted into its oxalate salt: mp 177-181°C. C,H,N Anal: [C₂₅H₂₆NOF·(COOH)₂].

### Synthesis of trans-(2-benzhydryl-tetrahydropyran-4-yl)-(4-fluro-benzyl)-amine 7b

*trans*-4-Amino-2-diphenylmethyl pyran **6b** (0.24 g, 0.90 mmol) was reacted with 4-fluorobenzaldehyde (0.11 g, 0.90 mmol) in presence of acetic acid (0.05 g, 0.9 mmol), and then reduced with NaCNBH₃ (0.07 g, 1.08 mmol) to yield compound **7b** (0.18 g, 54%) (Procedure D).

¹H NMR(500MHz, CDCl₃) 1.13(q, J=10.5Hz, 1H, H-3ax) 1.32(broad, NH) 1.38(dq, J=5Hz, 12.5Hz, 1H, H-5ax) 1.74(m, 1H, H-3eq) 1.87(m, 1H, H-5eq) 2.722(tt, J=4Hz, 11.5Hz, 1H, H-4) 3.444(dt, J=2Hz, 12Hz, 1H, H-6ax) 3.683(d, J=13.5Hz, 1H, (F)Ph-CH) 3.754(d, J=13Hz, 1H, (F)Ph-CH) 3.936(d, J=9Hz, 1H, (Ph)₂CH) 4.0-4.08(m, 2H, H-2, H-6eq) 6.9-7.38(m, 14H, aromatic-CH).

Free base was converted into its oxalate salt: mp 185-187°C. C,H,N Anal: [C₂₅H₂₆NOF·(COOH)₂].

### Synthesis of 1,1-diphenyl-hex-5-en-2-ol (8)

A dry three-neck, round-bottom flask fitted with a reflux condensor, air-balance drop funnel and nitrogen inlet was charged with Mg (0.11 g, 4.44 mmol) and a crystal of I₂, The flask was warmed (heat gun) to volatilize the I₂ under vacuum, and then was allowed to cool. Dry ethyl ether (10 ml) was added next followed by introduction of catalytic neat 4-bromo-1-butene (0.02 g).

The reaction was initiated by brief warming and then the rest of total amount of bromide (0.4 g, 2.96 mmol) in dry ethyl ether (5 ml) was added dropwise over 5 minutes. The mixture was refluxed for 30 minutes and then was allowed to reach 0°C. Into the stirred Grignard reagent solution was added dropwise a solution of diphenylacetaldehyde 1 (0.64 g, 3.26 mmol) in dry ethyl ether (5 ml), and the reaction mixture was stirred for an additional 3.5 hr at room temperature. Saturated aqueous NaHCO₃ was added to the reaction mixture at 0°C, the organic phase was separated and the aqueous phase was extracted with ethyl ether (3 x 20 ml). The combined organic phases were washed with brine and water, then dried over anhydrous Na₂SO₄. The solvent was removed under reduced pressure, and flash chromatography of the crude residue (SiO₂, hexane/Ethyl Acetate 9:1) gave 1,1-diphenyl-hex-5-en-2-ol **8** (0.68 g, 91%) as a liquid.

¹H NMR(400MHz, CDCl₃) 1.45-1.70(m, 2H, H-3) 1.69(bd, -OH) 2.1-2.4(m, 2H, H-4) 3.91(d, J=8.4 Hz, 1H, H-1) 4.39(m, 1H, H-2) 4.95-5.1(m, 2H, H-6) 5.81(m, 1H, H-5) 7.16-7.38(m, 10H, aromatic-CH).

### Synthesis of 1,1-diphenyl-2-(1-ethenoxy)-hex-5-ene (9)

Into a mixture of 1,1-diphenyl-hex-5-en-2-ol 2 (7 g, 27.78 mmol) in ethyl vinyl ether (250 ml) was added Hg(OCOCF₃)₂(2.37 g, 5.56 mmol) and was stirred overnight at room temperature. The reaction mixture was neutralized by addition of sat. aqueous NaHCO₃. The organic phase was separated and the aqueous layer was extracted with ethyl ether, and dried over anhydrous Na₂SO₄. Removal of the solvent and purification by flash chromatography (Hexane/Ethyl Acetate 20:1) gave 1,1-diphenyl-2-(1-ethenoxy)-hex-5-ene **9** (5.1 g, 66%) as a liquid.

¹H NMR(400MHz, CDCl₃) 1.58-1.78(m, 2H, H-3) 2.08-2.30(m, 2H, H-4) 3.86(dd, J=1.6Hz, 8.4Hz, 1H, H-2') 4.15(d, J=8Hz, 1H, Ph₂CH) 4.25(dd, J=1.6Hz, 14Hz, 1H, H-2') 4.50(m, 1H, H-2) 5.00(m, 2H, H-6) 5.77(m, 1H, H-5) 6.15(dd, J=6.8Hz, 14.8Hz, 1H, H-1') 7.16-7.38(m, 10H, aromatic-CH).

### Synthesis of 2-benzhydryl-3,4-dihydro-2H-pyran (10)

A solution of 1,1-diphenyl-2-(1-ethenoxy)-hex-5-ene **9** (5.1 g, 18.3 mmol) and Grubb's catalyst (1.5 g, 1.83 mmol) in benzene (200 ml) was heated under reflux for 20 hr. The solvent was removed under vacuo and the residue was chromatographed over silica gel (Hexane/Ethyl Acetate 20:1) to give 2-diphenyl-3,4-dihydro-2H-pyran **10** (4.25 g, 92.6%) as a liquid.

¹H NMR(400MHz, CDCl₃) 1.52-1.66(m, 1H, H-3) 1.76-1.84(m, 1H, H-3) 1.92-2.14(m, 2H, H-4) 4.08(d, J=9.2Hz, 1H, Ph₂CH) 4.59(dt, J=2.4Hz, 8.8Hz, 1H, H-2) 4.72(m, 1H, H-5) 6.38(d, J=6.4Hz, 1H, H-6) 7.16-7.50(m, 10H, aromatic-CH).

### Synthesis of Trans-6-benzhydryl-tetrahydropyran-3-ol (11)

Into a solution of 0.5M 9-BBN-THF complex (24 ml, 12 mmol) in dry THF (20 ml) was added in a drop wise manner 2-diphenyl-3,4-dihydro-2H-pyran **10** (1 g, 4 mmol) dissolved in dry THF(10 ml). The mixture was kept under stirring at room temperature. After the completion of initial addition reaction, the intermediate reaction mixture was oxidized with 5.3 ml 3N sodium hydroxide and 3 ml of 30 % hydrogen peroxide. The reaction was continued at 55°C for 1 hr to insure the completion of oxidation. After the mixture was diluted with sat. aqueous NaHCO₃, the organic layer was separated, and the aqueous layer was extracted with ethyl acetate (3 x 40 ml). The combined extract was dried over anhydrous Na₂SO₄. The solvent was removed in vacuo and the crude product was purified by flash chromatography (Hexane/Ethyl Acetate 7:3) to furnish *trans*-6-diphenyltetrahydropyran-3-ol **11** (1 g, 93.5%) as a liquid.

¹H NMR(300MHz, CDCl₃) 1.32-1.44(m, 2H, H-5) 1.54-1.64(m, 1H, H-4) 1.75(bs, 1H, OH) 2.02-2.14(m, 1H, H-4) 3.14(t, J=10.2Hz, 1H, H-2ax) 3.67(m, 1H, H-3) 3.90(d, J=9.3Hz, 1H, Ph₂CH) 3.95-4.04(m, 2H, H-2eq, H-6) 7.16-7.38(m, 10H, aromatic-CH).

### Synthesis of 6-benzhydryl-dihydropyran-3-one (12)

Into a solution of DMSO (0.13 g, 1.64 mmol) in methylene chloride (5 ml) at -78°C was added a solution of oxalyl chloride (0.11 g, 0.82 mmol) in methylene chloride (1 ml) in a dropwise manner. A solution of *trans*-2-diphenylmethyl-tetrahydropyran-5-ol 11 (0.2 g, 0.75 mmol) in methylene chloride (2 ml) was added next. The reaction was continued for 15 minutes, triethylamine (0.38 g, 3.73 mmol) was next added portion wise and the reaction mixture was allowed to come to room temperature for over a period of 30 minutes. Additional methylene chloride (10 ml) was added, and washed with sat. aqueous NaHCO₃, brine, and then dried over anhydrous Na₂SO₄. Removal of the solvent and purification by flash chromatography (SiO₂, Hexane/Ethyl Acetate 8.5:1.5) gave 2-diphenylmethyl-dihydro-pyran-5-one 12 (0.18 g, 91 %) as a liquid.

¹H NMR(300MHz, CDCl₃) 1.9-1.98(m, 2H, H-5) 2.38-2.62(m, 2H, H-4) 4.0(d, J=17.1Hz, 1H, H-2) 4.05(d, J=9Hz, 1H, Ph₂CH) 4.17(dd, J=1.8Hz, 16.2Hz, 1H, H-2) 4.44(dt, J=5.2Hz, 8.4Hz, 1H, H-6) 7.16-7.38 (m, 10H, aromatic-CH).

¹³C NMR(75MHz, CDCl₃) (ppm) 21.50, 32.00, 55.72, 65.62, 76.05, 126.89, 127.09, 128.60, 128.68, 128.90, 128.97, 141.36, 141.62, 146.77.

### Synthesis of Trans-(6-benzhydryl-tetrahydropyran-3-yl)-(34-fluorobenzy)-amine (16a) (comparative)

2-diphenylmethyl-dihydropyran-5-one 12 (0.18 g, 0.68 mmol) was reacted with 4-fluorobenzylamine (0.08 g, 0.68 mmol) in the presence of glacial acetic acid (0.041 g, 0.68 mmol) in 1,2-dichloroethane (10 ml) at room temperature, and then reduced by NaCNBH₃(0.051 g, 0.81 mmol) (Procedure D) to yield a mixture of 16a and 16b. *cis*-2-Diphenylmethyl-5-(4-flurobenzylamino)-tetrahydropyran 16b eluted first (0.04 g, 15%).

¹H NMR(300MHz, CDCl₃) 1.33(m, 1H, H-5) 1.46-1.72(m, 2H, H-5, H-4) 1.935(m, 1H, H-4) 2.031(bm, 1H, NH) 2.641(m, 1H, H-3) 3.571(dd, J=1.8Hz, 11.4Hz, 1H, H-2ax) 3.75(m, 2H, (F)Ph-CH₂) 3.95-4.14(m, 3H, H-6, H-2eq, Ph₂CH) 6.9-7.38(m, 14H, aromatic-CH).

Free base **16b** was converted into oxalate: mp 229-230°C. C,H,N Anal: [C₂₅H₂₆NOF·(COOH)₂].

Eluted second was *trans*-2-diphenylmethyl-5-(4-flurobenzylamino)-tetrahydropyran **16a** (0.11 g, 45%).

¹H NMR(300MHz, CDCl₃) 1.24-1.44(m, 2H, H-5) 1.55(m, 1H, H-4) 1.748(bm, NH) 2.02(m, 1H, H-4) 2.68(m, 1H, H-3) 3.11(t, J=10.8Hz, 1H, H-2ax) 3.76(s, 2H, (F)-Ph-CH₂) 3.89(d, J=9Hz, 1H, Ph₂CH) 3.99(dt, J=3Hz, 8.7Hz, 1H, H-6) 4.08(m, 1H, H-2eq) 6.9-7.38(m, 14H, aromatic-CH).

Free base **16a** was converted into the oxalate: mp 141-143°C. C,H,N Anal: [C₂₅H₂₆NOF·(COOH)₂0.65H₂O].

### Synthesis of methanesulfonic acid

### trans-6-benzhydryl-tetra-hydropyran-3-yl ester (13)

Methanesulfonyl chloride (0.33 g, 2.87 mmol) was reacted with trans-2-diphenylmethyl-tetrahydropyran-5-ol **11** (0.38 g, 1.43 mmol) in the presence of triethylamine (0.22 g, 2.15 mmol) in methylene chloride (10 ml) to give trans-2-diphenylmethyl-tetrahydropyran-5-yl methanesulfonate 13 (0.39 g, 77.8 %) as an oil (Procedure A).

¹H NMR(400MHz, CDCl₃) 1.47(m, 1H, H-5) 1.62-1.78(m, 2H, H-5, H-4) 2.25(m, 1H, H-4) 2.96(s, 3H, CH₃SO₂) 3.36(t, J=10.4Hz, 1H, H-2ax) 3.89(d, J=8.8Hz, 1H, Ph₂CH) 4.00(dt, J=2Hz, 9.6Hz, 1H, H-6) 4.14(m, 1H, H-2eq) 4.61(m, 1H, H-3) 7.16-7.38(m, 10H, aromatic-CH).

### Synthesis of Cis-3-azido-6-benzhydryl-tetrahydropyran (14)

*Trans*-2-diphenylmethyl-tetrahydropyran-5-yl methanesulfonate 13 (0.39 g, 1.12 mmol) in dry DMF (50 ml) was reacted with sodium azide (0.22 g, 3.35 mmol) to yield cis-5-azido-2-diphenylmethyl-tetrahydropyran **14** (0.3 g, 92%) as an oil (Procedure B).

¹H NMR (300MHz, CDCl₃) 1.36 (m, 1H, H-5) 1.54-1.85 (m, 2H, H-5, H-4) 1.98 (m, 1H, H-4), 3.55 (m, 1H, H-3), 3.64 (dd, J=1.8Hz, 12.6Hz, 1H, H-2) 3.95-4.15(m, 3H, H-2, H-6, Ph₂CH) 7.16-7.38(m, 10H, aromatic-CH).

### Synthesis of Cis-(6-benzhydryl-tetrahydropyran-3-yl)-amine (15)

Cis-5-azido-2-diphenylmethyl-tetrahydropyran 14 (0.3 g, 1.02 mmol) in methanol (25 ml) was hydrogenated under the catalyst of 10% Pd-C (0.03 g, 10% wt) for 4 hr (Procedure C) to give *cis*-5-amino-2-diphenylmethyl-tetrahydropyran **15** (0.21 g, 78%) as an oil.

¹H NMR(400MHz, CD₃OD) 1.31(m, 1H, H-5eq) 1.54(m, 1H, H-5ax) 1.70-1.86(m, 2H, H-4) 2.90(bs, bs, 1H, H-3) 3.68(m, 2H, H-2) 3.96(d, J=9.2Hz, 1H, Ph₂CH) 4.13(dt, J=2Hz, 9.6Hz, 1H, H-6) 7.10-7.40(m, 10H, aromatic-CH). Free base **15** was converted to the HCl salt: mp 260-261°C. C,H,N Anal: [C₁₈H₂₁NO·HCl 0.2H₂O].

### Synthesis of Cis-(6-benzhydryl-tetrahydropyran-3-yl)-(4-fluoro-benzyl)-amine (16b)

### (Comparative example)

*Trans-*5-amino-2-diphenylmethyl-tetrahydropyran **15** (0.21 g, 0.79 mmol) was reacted with 4-flurobenzaldehyde (0.098 g, 0.79 mmol) in the presence of glacial acetic acid (0.047 g, 0.79 mmol) in 1,2-dichloroethane (20 ml), and then reduced by NaCNBH₃ (0.059 g, 0.95 mmol) in methanol (5 ml) (Procedure D) to give compound **16b** (0.24 g, 82%).

¹H NMR(300MHz, CDCl₃) 1.33(m, 1H, H-5) 1.46-1.72(m, 2H, H-5, H-4) 1.935(m,1H, H-4) 2.031(bm, 1H, NH) 2.641(m, 1H, H-3) 3.571(dd, J=1.8Hz, 11.4Hz, 1H, H-2ax) 3.75(m, 2H, (F)Ph-CH₂) 3.95-4.14(m, 3H, H-6, H-2eq, Ph₂CH) 6.9-7.38(m, 14H, aromatic-CH).

Free base 16b was converted into the oxalate: mp 229-230°C. C,H,N Anal: [C₂₅H₂₆NOF·(COOH)₂].

### Synthesis of Cis-(6-benzhydryl-tetrahydropyran-3-yl)-(4-cyano-benzyl)-amine (16c)

### (Comparative)

*Trans*-5-amino-2-diphenylmethyl-tetrahydropyran 15 (0.15 g, 0.56 mmol) was reacted with 4-cyanobenzaldehyde (0.07 g, 0.56 mmol) in the presence of glacial acetic acid (0.033 g, 0.56 mmol) in 1,2-dichloroethane (20 ml), and NaCNBH₃(0.042 g, 0.67 mmol) in methanol (5 ml) (Procedure D) to give compound **16c** (0.17 g, 80%) as an oil.

¹H NMR (300MHz, CDCl₃) 1.36(m, 1H, H-5) 1.46-1.58(m, 1H, H-5) 1.58-1.74(m, 1H, H-4) 1.931(m, 1H, H-4) 2.615(bm, 1H, H-3) 3.59(dd, J=1.8Hz, 11.7Hz, H-2ax) 3.83(m, 2H, (CN)Ph-CH₂) 3.95-4.16(m, 3H, H-6, H-2eq, Ph₂CH) 7.16-7.62(m, 14H, aromatic-CH). Free base 16c was converted into the oxalate: mp 241-242C. C,H,N Anal: [C₂₆H₂₆N₂O · (COOH)₂].

### Synthesis of Cis-(6-benzhydryl-tetrahydropyran-3-yl)-(4-nitro-benzyl)-amine (16d)

### (Comparative)

*Trans*-5-amino-2-diphenylmethyl-tetrahydropyran 15 (0.1g, 0.38 mmol) was reacted with 4-nitrobenzaldehyde (0.057 g, 0.38 mmol) in the presence of glacial acetic acid (0.023 g, 0.38 mmol) in 1,2-dichloroethane (20 ml), and then reduced by NaCNBH₃(0.03 g, 0.45 mmol) in methanol (5 ml) (Procedure D) to give compound **16d** (0.12 g, 80%) as an oil.

¹H NMR (300MHz, CDCl₃) 1.35(m, 1H, H-5) 1.53(m, 1H, H-5) 1.67(tt, J=3.6Hz, 13.5Hz, 1H, H-4) 1.91(m, 2H, H-4, NH) 2.62(m, 1H, H-3) 3.58(dd, J=1.8Hz, 9.6Hz, 1H, H-2ax) 3.87(m, 2H, (NO₂)Ph-CH₂) 3.92-4.14(m, 3H, H-6, H-2eq, Ph₂CH) 7.14-7.54, 8.12-8.2(m, 14H, aromatic-CH). Free base **16d** was converted into the oxalate: mp 236-238°C. C,H,N Anal: [C₂₅H₂₆N₂O₃·(COOH)₂].

### Synthesis of Cis-(6-benzhydryl-tetrahydropyran-3-yl)-(4-methoxy-benzyl)-amine (16e)

### (Comparative)

*Trans*-5-amino-2-diphenylmethyl-tetrahydropyran **15** (0.15 g, 0.56 mmol) was reacted with 4-methoxybenzaldehyde (0.078 g, 0.56 mmol) in the presence of glacial acetic acid (0.033 g, 0.56 mmol) in 1,2-dichloroethane (20 ml), and NaCNBH₃ (0.042 g, 0.67 mmol) in methanol (5 ml) (Procedure D) to give compound **16e** (0.17 g, 78%) as an oil.

¹H NMR (300MHz, CDCl₃) 1.35(m, 1H, H-5) 1.48-1.76(m, 2H, H-5, H-4) 1.88-2.02(m, 1H, H-4) 2.68(bs, 1H, H-3) 3.59(dd, J=12.3Hz, 2.4Hz, 1H, H-2ax) 3.76(d, J=7.2Hz, 2H, (CH₃O)Ph-CH₂) 3.825(s, 3H, CH₃O-3.98-4.16(m, 3H, H-6, H-2eq, Ph₂CH) 6.88-6.94, 7.18-7.44(m, 14H, aromatic-CH). Free base **16e** was converted into the oxalate: mp 215-217°C. C,H,N Anal: [C₂₆H₂₉NO₂·(COOH)₂].

### Synthesis of Cis-(6-benzhydryl-betrahydropyran-3-yl)-(3-indole-methyl)-amine (16f)

### (Comparative)

*Trans*-5-amino-2-diphenylmethyl-tetrahydropyran **15** (0.12g, 0.45 mmol) was reacted with 3-indole-carboxaldehyde (0.065 g, 0.45 mmol) in the presence of glacial acetic acid (0.027 g, 0.45 mmol) in 1,2-dichloroethane (20 ml), and NaCNBH₃(0.034 g, 0.54 mmol) in methanol (5 ml) (Procedure D) to give compound **16f** (0.15 g, 82%) as an oil.

¹H NMR (400MHz, CDCl₃) 1.34(m, 1H, H-5) 1.53(m, 1H, H-5) 1.67(tt, J=14Hz, 4Hz, 1H, H-4) 1.93(m, 1H, H-4) 2.37(bm, 1H, NH) 2.65(bs, 1H, H-3) 3.57(dd, J=10.7Hz, 1.6Hz, 1H, H-2ax) 3.96(s, 2H, 2-Indole-CH₂) 3.92-4.14(m, 3H, H-6, H-2eq, Ph₂CH) 6.35(s, 1H, Indole-3-H) 7.05-7.6(m, 14H, aromatic-CH) 9.1(s, 1H, Indole-NH). Free base **16f** was converted into the oxalate: mp 177-179°C. C, H, N Anal: [C₂₇H₂₈N₂O·(COOH)₂·0.5H₂O].

### Synthesis of Cis-(6-benzhydryl-tetrahydropyran-3-yl)-(2-indole-methyl)-amine (16g)

### (Comparative)

*Trans*-5-amino-2-diphenylmethyl-tetrahydropyran 15 (0.067 g, 0.25 mmol) was reacted with 2-indole-carboxaldehyde (0.036 g, 0.25 mmol) in the presence of glacial acetic acid (0.015 g, 0.25 mmol) in 1,2-dichloroethane (20 ml), and then reduced by NaCNBH₃(0.019 g, 0.3 mmol) in methanol (5 ml) (Procedure D) to give compound 16g (0.081 g, 82%) as an oil.

¹H NMR (300MHz, CDCl₃) 1.33(m, 1H, H-5) 1.48-1.76(m, 2H, H-5, H-4) 1.99(m, 1H, H-4) 2.27(bs, 1H, NH) 2.79(m, 1H, H-3) 3.6(dd, J=1.8Hz, 12.3Hz, 1H, H-2ax) 3.998(s, 2H, Indole-3-CH₂) 4.02-4.2(m, 3H, H-6, H-2eq, Ph₂CH) 7.0-7.8(m, 14H, aromatic-CH) 8.42(s, 1H, Indole-NH). Free base **16g** was converted into the oxalate: mp 215-216°C. C, H, N Anal: [C₂₇H₂₈N₂O·(COOH)₂.0.5H₂O].

### Synthesis of Cis-(6-benzhydryl-tetrahydropyran-3-yl)(4-hydroxy-benzyl)amine (16h)

### (Comparative)

*Trans*-5-amino-2-diphenylmethyl-tetrahydropyran **15** (0.15 g, 0.56 mmol) was reacted with 4-hydroxybenzaldehyde (0.067 g, 0.56 mmol) in the presence of glacial acetic acid (0.034 g, 0.56 mmol) in 1,2-dichloroethane (20 ml), and NaCNBH₃(0.042 g, 0.67 mmol) in methanol (5 ml) (Procedure D) to give compound **16h** (0.17 g, 80%) as an oil.

¹H NMR (400MHz, CDCl₃) 1.34(m, 1H, H-5) 1.50(m, 1H, H-5) 1.67(tt, J=4Hz, 13.6Hz, 1H, H-4) 2.02(m, 1H, H-4) 2.71(m, 1H, H-3) 3.56(dd, J=1.6Hz, 11.6Hz, 1H, H-2ax) 3.64(m, 2H, (HO)Ph-CH₂) 3.95(d, J=8.0Hz, 1H, Ph₂CH) 4.02-4.14(m, 2H, H-6, H-2eq) 6.52(m, 2H, aromatic-CH) 6.9-7.38(m, 12H, aromatic-CH). Free base **16h** was converted into oxalate: mp 136-138°C. C, H, N Anal: [C₂₅H₂₇NO₂·(COOH)₂].

### Synthesis of Cis-(6-benzhydryl-tetrahydropyran-3-yl)-(3,4-dichloro-benzyl)-amine (16i)

### (Comparative)

*Trans*-5-amino-2-diphenylmethyl-tetrahydropyran **15** (0.1g, 0.38 mmol) was reacted with 3,4-dichlorobenzaldehyde (0.066 g, 0.38 mmol) in the presence of glacial acetic acid(0.023 g, 0.38 mmol) in 1,2-dichloroethane (20 ml), and NaCNBH₃ (0.03 g, 0.45 mmol) in methanol (5 ml) (Procedure D) to give compound **16i** (0.12 g, 75%) as an oil.

¹H NMR (500MHz, CDCl₃) 1.34(m, 1H, H-5) 1.52(m, 1H, H-5) 1.66(m, 1H, H-4) 1.79(bs, 1H, NH) 1.91(m, 1H, H-4) 2.61(m, 1H, H-3) 3.57(dd, J=1.5Hz, 11.5Hz, 1H, H-2ax) 3.72(m, 2H, (Cl,Cl)Ph-CH₂) 3.94-4.05(m, 2H, H-2eq, Ph₂CH) 4.08(dt, J=2Hz, 8.5Hz, 1H, H-6) 7.1-7.5(m, 14H, aromatic-CH). Free base **16i** was converted into the oxalate: mp 251-252°C. C, H, N Anal: [C₂₅H₂₅NOCl₂·(COOH)₂].

### Synthesis of Cis-(6-benzhydryl-tetrahydropyran-3-yl)-(3,4-difluoro-benzyl)-amine (16j)

### (Comparative)

*Trans*-5-amino-2-diphenylmethyl-tetrahydropyran **15** (0.1 g, 0.38 mmol) was reacted with 3,4-difluorobenzaldehyde (0.055 g, 0.38 mmol) in the presence of glacial acetic acid (0.023 g, 0.38 mmol) in 1,2-dichloroethane (20 ml), and NaCNBH₃(0.03 g, 0.45 mmol) in methanol (5 ml) (Procedure D) to give compound **16j** (0.12 g, 80%).

¹H NMR (300MHz, CDCl₃) 1.34(m, 1H, H-5) 1.52(m, 1H, H-5) 1.66(tt, J=3.6Hz, 13.5Hz, 1H, H-4) 1.76(bs, 1H, NH) 1.92(m, 1H, H-4) 2.61(m, 1H, H-3) 3.57(dd, J=1.8Hz, 11.4Hz, 1H, H-2ax) 3.72(m, 2H, (F,F)Ph-CH₂) 3.94-4.14(m, 3H, H-6, H-2eq, Ph₂CH) 6.9-7.38(m, 14H, aromatic-CH). Free base **16j** was converted into the oxalate: mp 234-235°C. C, H, N Anal: [C₂₅H₂₅NOF₂·(COOH)₂].

### Synthesis of Cis-(6-benzhydryl-tetrahydropyran-3-yl)-benzyl-amine (16k) (Comparative)

*Trans*-5-amino-2-diphenylmethyl-tetrahydropyran **15** (0.03 g, 0.11 mmol) was reacted with benzaldehyde (0.012 g, 0.11 mmol) in the presence of glacial acetic acid (0.007 g, 0.11 mmol) in 1,2-dichloroethane (20 ml), and NaCNBH₃(0.009 g, 0.14 mmol) in methanol (5 ml) (Procedure D) to give compound **16k** (0.034 g, 85%).

¹H NMR (300MHz, CDCl₃) 1.30(m, 1H, H-5) 1.44-1.70(m, 2H, H-5, H-4) 1.80(bs, 1H, NH) 1.92(m, 1H, H-4) 2.64(m, 1H, H-3) 3.55(dd, J=1.8Hz, 11.7Hz, 1H, H-2ax) 3.77(m, 2H, Ph-CH₂) 3.92-4.1(m, 3H, Ph₂CH, H-6, H-2eq) 7.0-7.38(m, 15H, aromatic-CH). Free base **16k** was converted into the oxalate: mp 208-210°C. C, H, N Anal: [C₂₅H₂₇NO·(COOH)₂].

### Synthesis of Cis-(6-benzhydryl-tetrahydropyran-3-yl)-(4-bromo-benzyl)-amine (161)

### (Comparative)

*Trans-*5-amino-2-diphenylmethyl-tetrahydropyran **15** (0.04 g, 0.15 mmol) was reacted with 4-bromobenzaldehyde (0.028 g, 0.15 mmol) in the presence of glacial acetic acid (0.009 g, 0.15 mmol) in 1,2-dichloroethane (20 ml), and NaCNBH₃(0.012 g, 0.18 mmol) in methanol (5 ml) (Procedure D) to give compound **161** (0.052 g, 80%) as an oil.

¹H NMR (400MHz, CDCl₃) 1.31(m, 1H, H-5) 1.50(m, 1H, H-5) 1.64(m, 1H, H-4) 1.80(bs, 1H, NH) 1.90(m, 1H, H-4) 2.61(m, 1H, H-3) 3.56(dd, J=1.6Hz, 11.6Hz, 1H, H-2ax) 3.72(m, 2H, (Br)-Ph-CH₂) 3.94-4.30(m, 2H, Ph₂CH, H-2eq) 4.07(dt, J=1.6Hz, J=9.6Hz, 1H, H-6) 7.0-7.42(m, 14H, aromatic-CH). Free base **161** was converted into the oxalate: mp 250-252°C. C, H, N Anal: [C₂₅H₂₆BrNO·(COOH)₂].

### Synthesis of Cis-(6-benzhydryl-tetrahydropyran-3-yl)-(4-iodo-benzyl)-amine (16m)

### (Comparative)

*Trans*-5-amino-2-diphenylmethyl-tetrahydropyran **15** (0.04 g, 0.15 mmol) was reacted with 4-iodobenzaldehyde (0.045 g, 0.15 mmol) in the presence of glacial acetic acid (0.009 g, 0.15 mmol) in 1,2-dichloroethane (20 ml), and NaCNBH₃(0.012 g, 0.18 mmol) in methanol (5 ml) (Procedure D) to give compound **16m** (0.059 g, 81 %) as an oil.

¹H NMR (400MHz, CDCl₃) 1.28(m, 1H, H-5) 1.50(m, 1H, H-5) 1.64(m, 1H, H-4) 1.72(bs, 1H, NH) 1.90(m, 1H, H-4) 2.60(m, 1H, H-3) 3.56(dd, J=1.6Hz, 12.4Hz, 1H, H-2ax) 3.71(m, 2H, (I)-Ph-CH₂) 3.92-4.02(m, 2H, Ph₂CH, H-2eq) 4.06(dt, J=1.6Hz, J=9.2Hz, 1H, H-6) 7.0-7.70(m, 14H, aromatic-CH). Free base **16m** was converted into the oxalate: mp 243-244°C. C, H, N Anal: [C₂₅H₂₆INO·(COOH)₂].

### Synthesis of Cis-(6-benzhydryl-tetrahydropyran-3-yl)-(1H-iodo-5-ylmethyl)-amine (16n)

### (Comparative)

*Trans*-5-amino-2-diphenylmethyl-tetrahydropyran **15** (0.05 g, 0.19 mmol) was reacted with 5-indole-carboxaldehyde (0.027 g, 0.19 mmol) in the presence of glacial acetic acid (0.011 g, 0.19 mmol) in 1,2-dichloroethane (20 ml), and NaCNBH₃(0.024 g, 0.37 mmol) in methanol (5 ml) (Procedure D) to give compound **16n** (0.061 g, 82%) as an oil.

¹H NMR (400MHz, CDCl₃) 1.32(m, 1H, H-5) 1.50-1.70(m, 2H, H-5, H-4) 1.95(m, 2H, H-4, NH) 2.71(bs, 1H, H-3) 3.57(dd, J=2Hz, 12Hz, 1H, H-2ax) 3.88(m, 2H, Indole-CH₂) 3.96-4.12(m, 3H, Ph₂CH, H-2eq, H-6) 6.51, 7.1-7.4, 7.57 (m, 15H, aromatic-CH) 8.36(bs, 1H, NH). Free base **16n** was converted into the oxalate: mp 128-130°C. C, H, N Anal: [C₂₇H₂₈N₂O·(COOH)₂·0.5H₂O].

### Synthesis of Cis-(6-benzhydryl-tetrahydropyran-3-yl)-(4-amino-benzyl)-amine (16o)

### (Comparative)

A mixture of *cis*-(6-benzhydryl-tetrahydropyran-3-yl)-(4-nitro-benzyl)-amine **(16f)** (0.16 g, 0.39 mmol) and SnCl₂/2H₂O (0.35 g, 1.55 mmol) in EtOH/EtOAc (20 ml, 7:3) was heated to reflux for 1.5h (monitored by TLC, Hex/EtOAc/Et₃N 5:5:0.4). After removal of the solvent, the residue was diluted with 10% NaHCO₃ and EtOAc and stirred vigorously for 30 min. After filtration the organic phase was separated and the aqueous phase was extracted with EtOAc (20 ml x 2). The combined organic phase was dried over Na₂SO₄. After removal of the solvent, flash chromatography gave **16o,** *cis*-(6-benzhydryl-tetrahydropyran-3-yl)-(4-amino-benzyl)-amine (0.087 g, 60%).

¹H NMR (400MHz, CDCl₃) 1.3(m, 1H, H-5) 1.47(m, 1H, H-5) 1.64(tt, J=4Hz, 12.8Hz, 1H, H-4) 1.90(m, 1H, H-4) 2.53-2.70(m, 3H, H-3, (NH₂)-PhCH₂) 3.54(dd, J=1.6 Hz, 11.2Hz, 1H, H-2ax) 3.92-4.0(m, 2H, Ph₂CH, H-2eq) 4.06(dt, J=2.4Hz, 9.6Hz, 1H, H-6) 7.06-7.38(m, 14H, aromatic-CH). Free base **16o** was converted into the oxalate: mp 151-153°C. C, H, N Anal: [C₂₅H₂₈N₂O -2(COOH)₂·0.3H₂O].

### Synthesis of Cis-N-(6-benzhydryl-tetrahydro-pyran-3-yl)-2-(4-fluoro-phenyl)-acetamide (17)

Into a solution of 4-fluorophenylacetic acid (0.23 g, 1.46 mmol) in dichloromethane (25 ml) was added oxalyl chloride (0.22 g, 1.76 mmol) dissolved in dichloromethane (5 ml) at 0°C which was followed by addition of one drop of DMF. The reaction mixture was allowed to reach at room temperature over a period of 2 hours. The solvent was removed in vacuo, and the residue was dissolved in dichloromethane (5 ml) and was added into a solution of *cis*-N-(6-benzhydryl-tetrahydropyran-3-yl)-amine (0.26 g, 0.96 mmol) and triethylamine (0.31 g, 1.46 mmol) in dichloromethane (25 ml) at 0°C. After 20 minutes the reaction mixture was allowed to come to room temperature. After 3 hours, more dichloromethane was added and the mixture was washed in turn with 1N NaHCO₃, H₂O and brine, then dried over anhydrous Na₂SO₄. The solvent was removed *in vacuo,* and the residue was purified by flash chromatography (hexane/ethyl acetate 7:3) to give *cis*-N-(6-benzhydryl-tetrahydropyran-3-yl)-2-(4-fluorophenyl)-acetamide 17 (0.31 g, yield 80%) as an oil.

¹H NMR (300MHz, CDCl₃) 1.1-1.4(m, 2H, H-5) 1.6-1.93(m, 2H, H-4) 3.49(s, 2H, Ph-CH₂CO) 3.63(dd, J=1.8Hz, 11.7Hz, 1H, H-2ax) 3.7-3.85(m, 2H, Ph₂CH, H-3) 3.9-4.08(m, 2H, H-6, H-2eq) 6.9-7.4(m, 14H, aromatic-CH).

### Synthesis of Cis-(6-benzhydryl-tetrahydropyran-3yl)-

### [2-(4-fluoro-phenyl)-ethyl]-amine (16p) (Comparative)

Into a suspension of NaBH₄ (0.21 g, 3.33 mmol) in dry THF (20 ml) was added BF₃·Et₂O drop wise at 0°C. The mixture was stirred for 1.5 Hours at room temperature and cooled to 0°C. A solution of *cis*-N-(6-benzhydryl-tetrahydropyran-3-yl)-2-(4-fluorophenyl)-acetamide **17** (0.17 g, 0.42 mmol) in dry THF (10 ml) was added dropwise into the solution. The mixture was refluxed overnight and cooled to room temperature. Methanol was added to quench the reaction followed by removal of solvent *in vacuo.* To the residue was added 20 ml 10% HCl/MeOH and the mixture refluxed for 1 hour. The reaction mixture was cooled down to room temperature and solid NaHCO₃ was added at 0°C to pH 9. The aqueous phase was extracted with dichloromethane (3 x 20 ml). The combined organic phases were dried over anhydrous Na₂SO₄, and the solvent was removed *in vacuo.* Flash chromatography gave **16p** *Cis*-(6-benzhydryl-tetrahydropyran-3yl)-[2-(4-fluorophenyl)-ethyl]-amine (0.13 g, yield 81%).

¹H NMR (300MHz, CDCl₃) 1.2-1.42(m, 2H, H-5, NH) 1.61(m, 1H, H-5) 1.88(m, 2H, H-4) 2.64(m, 1H, H-3) 2.72-2.82(m, 4H, Ph-CH₂CH₂) 3.55(dd, J=1.8Hz, 11.7Hz, 1H, H-2ax) 3.86-3.98(m, 2H, Ph₂CH, H-2eq) 4.03(dt, J=3Hz, 10Hz, 1H, H-6) 6.9-7.4(m, 14H, aromatic-CH). Free base **16p** was converted into the oxalate: mp 240-242°C. C, H, N Anal: [C₂₆H₂₈NOF·(COOH)₂].

**Biology.** The affinity of test compounds in binding to rat DAT, SERT, and NET was assessed by measuring inhibition of binding of 5.0 nM [³H]WIN 35,428, 3.5 nM [³H]citalopram, and 1.1 nM [³H]nisoxetine, respectively, exactly as described by us previously. Briefly, rat striatum was the source for DAT, and cerebral cortex for SERT and NET. Final [Na⁺] was 30 mM for DAT and SERT assays, and 152 nM for NET assays. All binding assays were conducted at 0-4?, for a period of 2h for [³H]WIN 35,428 and [³H]citalopram binding, and 3h for [³H]nisoxetine binding. Nonspecific binding of [³H]WIN 35,428 and [³H]citalopram binding was defined with 100uM cocaine, and that of [³H]nisoxetine binding with 1 uM desipramine. Radioligand Kd values were 2.1, 3.2 and 2.2 nM, respectively. Test compounds were dissolved in dimethyl sulfoxide (DMSO) and diluted out in 10% (v/v) DMSO. Additions from the latter stocks resulted in a final concentration of DMSO of 0.5%, which by itself did not interfere with radioligand binding. At lease five triplicate concentrations of each test compound were studied, spaced evenly around the IC50 value. For DAT uptake assays, uptake of 50 nM [³H]DA into rat striatal synaptosomes was measured exactly as described by us previously. Briefly, rat striatal P₂ membrane fractions were incubated with test compounds for 8min followed by the additional presence of [³H]DA for 4min at 25?. Nonspecific uptake was defined with 100uM cocaine. Construction of inhibition curves and dissolvement of test compounds were as described above.

**(Comparative) TABLE 2**

| Affinity of Drugs at Dopamine, Serotonin, and Norepinephrine Transporters in Rat Striatum | | | | |
|---|---|---|---|---|
| Compound | DAT binding, IC₅₀, nM, [³H]Win 35, 428^{a} | SERT binding, IC₅₀, nM [³H]citalopram^{a} | NET binding, IC₅₀, nM [³H]nisoxetine^{a} | DAT uptake, IC₅₀, nM [³H]DA^{a} |
| Cocaine | 266±37 | 737±160 | 3,130±550 | |
| GBR 12909 | 10.6±1.9 | 132±0 | 496±22 | |
| 1 | 32.5±12.6 | 2,220±590 | 1,020±72 | 45.7±5.1 |
| 7a | 1,302±68 | 3,313±170 | 5,101±1,037 | |

| **Compound** | **DAT binding, IC₅₀, nM, [³H]Win 35, 428^{a}** | **SERT binding, IC₅₀, nM [₃H]citalopram^{a}** | **NET binding, IC₅₀, nM [³H]nisoxetine^{a}** | **DAT uptake, IC₅₀, nM [³H]DA^{a}** |
|---|---|---|---|---|
| **7b** | 1,581±283 | 4,778±1,808 | 17,543±2,153 | |
| **16a** | 313±71^{b} | 8,410±163 | 12,700±3,180 | |
| **16b** | 163±29^{b} | 1,860±22 | 232±46 | 146±36 |
| **16c** | 52.6±5.9^{b} | 863±52 | 1,580±89 | 58.6±13.2 |
| **16d** | 38.3±3.9" | 738±164 | 968±98 | 102±7 |
| **16e** | 84±6.5 | 1,180±269 | 1,550±682 | 59.5±11.6 |
| **16f** | 794±111 | 2,590±1,410 | 1,860±847 | |
| **16g** | 227±67 | 1,640±448 | 401±96 | 135.2±47.5 |
| **16h** | 78.4±9 | 398±22 | 22.6±1.4 | |
| **16i** | 400±31 | 780±84 | 144±25 | 880±136 |
| **16j** | 368±85 | 3,520±831 | 695±142 | |
| **16k** | 303±14 | 1577±97 | 274±29 | 242±39 |
| **161** | 202±13 | 2363±92 | 592±12 | 251±14 |
| **16m** | 319±21 | 2477±145 | 234±17 | 500±34 |
| **16n** | 587±66 | 325±20 | 56±6 | |
| **16o** | 151±13 | 1690±169 | 123±10 | 155±14 |
| **16p** | 129±58 | 3,950±660 | 5,210±678 | |
| **15** | 777±41 | | | 251±31 |

| | | | | |
|---|---|---|---|---|
| a. For binding, the DAT was labeled with [³H]WIN 35, 428, the SERT with [³H]citalopram and the NET with [³H]nisoxetine. For uptake by DAT, [3H]DA accumulation was measured. Results are average ± SEM of three to eight independent experiments assayed in triplicate. b. See reference # 22. | | | | |

**Comparative TABLE 3**

| Selectivity of Various Drugs for their Activity at Monoamine Transporters | | | |
|---|---|---|---|
| Compound | SERT binding/DAT binding | NET binding/DAT binding | [3H]DA uptake/DAT binding |
| Cocaine | 2.8 | 11.8 | |
| GBR 12909 | 12.5 | 46.8 | |
| 1 | 68.3 | 31.4 | 1.4 |
| 7a | 2.5 | 3.9 | |
| 7b | 3 | 11.1 | |
| 16a | 26.9 | 40.6 | |
| 16b | 11.4 | 1.4 | 0.96 |
| 16c | 16.4 | 30 | 1.1 |
| 16d | 19.3 | 25.3 | 2.7 |
| 16e | 14 | 18.5 | 0.71 |
| 16f | 3.3 | 2.3 | |
| 16g | 7.2 | 1.8 | 0.60 |
| 16h | 5.1 | 0.29 | |
| 16i | 1.9 | 0.36 | |
| 16j | 9.6 | 1.9 | |
| 16k | 5.20 | 0.90 | 0.79 |
| 161 | 11.69 | 2.93 | 1.24 |
| 16m | 7.76 | 0.73 | 1.56 |
| 16n | 0.55 | 0.09 | |
| 16o | 11.19 | 0.81 | 1.02 |
| 16p | 30.6 | 40.4 | |
| 15 | | | 0.32 |

### Synthesis of 3,3-Diphenylpropene (22)

Methyltriphenylphosphonium bromide (4 g, 11.12 mmol) was added over a 15-min period to a mixture of butyllithium (7.3 ml of 1.6 M solution in THF, 11.76 mmol) and dry THF (50 ml) with stirring and under nitrogen atmosphere at **<ALOKE, Temp.>** °C. The reaction mixture was stirred for 2h at room temperature and the mixture was then recooled to -78°C. A solution of diphenylacetaldehyde (2.2 g, 11.12 mmol) in dry THF(10 ml) was added to the above mixture over a 15-min period. The reaction mixture was stirred for 24 h at room temperature, followed by addition of ethyl ether (200 ml), and the reaction mixture was then filtered. The ether extracts were washed with water (3 x 50ml), brine (100 ml) and dried over anhydrous sodium sulfate. The crude material was purified by flash chromatography on silical gel (Hexane:Ethyl ether=9:1) to give pure 3,3-diphenylpropene 460 mg (46%).

¹HNMR (CDCl₃, 400MHz) 4.82(d, J=6.4Hz, 1H, H-3) 5.08(d, J=17.2Hz, 1H, H-1) 5.31(d, J=12Hz, 1H, H-1) 6.39(m, 1H, H-2) 7.2-7.4 (m, 10H, aromatic-H) ¹³CNMR (CDCl₃, 100MHz) 55.30, 116.69, 126.67, 128.73, 128.92, 140.94, 143.59.

### Synthesis of 2-Benzhydryl-oxirane (23)

A flask was charged with 3,3-diphenylpropene (5.1 g, 26.3 mmol) in 100 ml CH₂Cl₂. It was followed by by portionwise addition of mCPBA (9.1 g, 70% purity, 52.6 mmol) at 0° C. The mixture was stirred at room temperature for 24h and the reaction was then quenched with 30 ml 1M Na₂SO₃. The aqueous layer was extracted with CH₂Cl₂ (2 x 100 ml). The combined organic phases were washed in turn with saturated NaHCO₃, brine, and then dried over anhydrous Na₂SO₄. Purification by flash chromatography (Hexane/ether=9:1) gave pure 2-benzhydryl-oxirane, 4.7g (85%).

¹HNMR (CDCl₃. 400MHz) 2.54(m, 1H, H-1) 2.87(m, 1H, H-1) 3.54(m, 1H, H-2) 3.86(d, J=7.6Hz, 1H, Ph₂CH), 7.2-7.4(m, 10H, aromatic-H) ¹³CNMR (CDCl₃, 100MHz) 46.80, 53.58, 55.17, 127.06, 127.14, 128.70, 128.81, 141.28.

### Resolution of Racemic 2-benzhydryl-oxirane (23) by HKR Reaction

A mixture of (R,R)-(-)-N,N'-Bis(3,5-di-tert-butylsalicylidene)-1,2-cyclohexane diaminocobalt (II) (0.22 g, 0.37 mmol, 0.8%), toluene (5 ml), and acetic acid (0.044 g, 0.74 mmol) was stirred for 1h at room temperature. The solvent was removed in vacuo and the residue was dried. 2-Benzhydryl-oxirane (9.6 g, 45.7 mmol) was added in one portion and stirred, the mixture was then cooled by means of an ice-bath. H₂O (0.58 g, 32 mmol) was slowly added over a 30-min period. After water addition, the ice bath was removed and the reaction mixture was stirred at room temperature for **72h.** Compounds were separated via flash chromatography on slica gel column to give (2R)-2-benzhydryl-oxirane **(23a)** 4.5 g ([α]_{D}=(+)9.58, c=1; MeOH) and (2S)-3,3-diphenyl-propane-1,2-diol **24** 3.53 g ([α]_{D}=(+)48, c=1, MeOH, ee=97%). The proton and carbon NMR data of (2R)-2-benzhydryl-oxirane was identical to the racemate 2-benzhydryl-oxirane.

¹HNMR (CDCl₃, 400MHz) 2.54(m, 1H, H-1) 2.87(m, 1H, H-1) 3.54(m, 1H, H-2) 3.86(d, J=7.6Hz, 1H, Ph₂CH), 7.2-7.4(m, 10H, aromatic-H). ¹³CNMR (CDCl₃, 100MHz) 46.80, 53.58, 55.17, 127.06, 127.14, 128.70, 128.81, 141.28.

### For (2S)-3,3-diphenyl-propane-1,2-diol:

¹HNMR (CDCl₃, 400MHz) 2.39(bs, 2H, OH) 3.44(m, 1H, H-1) 3.60(m, 1H, H-1), 4.02(D, J=10Hz, 1H, Ph₂CH), 4.44(m, 1H, H-2), 7.16-7.22(m, 10H, aromatic-H). ¹³CNMR (CDCl₃, 100MHz) 55.08, 64.94, 74.26, 127.08, 127.23, 128.35, 128.84, 129.03, 129.17, 141.23, 141.62

### Synthesis of (2S)-2-benzhydryl-oxirane (23b)

A solution of (2S)-3,3-diphenyl-propane-1,2-diol (3.5 g, 15.35 mmol), Ph₃P (8.05 g, 30.7 mmol), and DEAD (5.4 g, 30.7 mmol) in benzene (50 ml) was refluxed for 24h. Solvent was removed and the residue was diluted with ethyl ether (200 ml) to precipitate Ph₂PO. The filtrate was concentrated and the residue was chromatographed on silica gel (hexane/ether=9:1) to give (2S)-2-benzhydryl-oxirane **23b** 2.5g (788%, ([α]_{D}=(-)9.6, c=1, MeOH). The ¹HNMR and ¹³CNMR were identical with (R)-isomer.

¹HNMR (CDCl₃, 400MHz) 2.54(m, 1H, H-1) 2.87(m, 1H, H-1) 3.54(m, 1H, H-2) 3.86(d, J=7.6Hz, 1H, Ph₂CH), 7.2-7.4(m, 10H, aromatic-H). ¹³CNMR (CDCl₃, 100MHz) 46.80, 53.58, 55.17, 127.06, 127.14, 128.70, 128.81, 141.28.

### Procedure A. Synthesis of (2S)-1,1-Diphenyl-pent-4-ene-2-ol (25a)

(2R)-2-benzhydryl-oxirane (0.5 g, 2.38 mmol) **23a** was dissolved in dry THF (5 ml) and was added into a dry THF solution at -78°C containing CuI (0.045 g, 0.24 mmol) and vinylmagnesium bromide (5.95 ml of 1.0M solution in THF, 5.95 mmol). The reaction mixture was stirred and allowed to reach room temperature over a period of 2h, and then quenched with saturated NH₄Cl solution. The aqueous phase was extracted with ethyl acetate (3 × 30 ml). The combined organic phase was washed with brine and dried over anhydrous Na₂SO₄. The solvent was removed and the residue was purified by flash chromatography on silica gel (Hexane/Ethyl Ether=4:1) to give 0.4 g (2S)-1,1-diphenyl-pent-4-ene-2-ol (70%, [α]_{D} = (-)25, c=1, MeOH).

¹HNMR (CDCl₃, 400MHz) 2.14(m, 1H, H-3), 2.33(m, 1H, H-3), 3.93(d, J=8.8Hz, 1H, H-1) 4.44(m, 1H, H-2) 5.1(m, 2H, H-5), 5.9(m, 1H, H-4), 7.16-7.24 (m, 10H, aromatic-H). ¹³CNMR (CDCl₃, 100MHz) 39.75, 58.21, 73.06, 118.23, 126.86, 127.08, 128.51, 128.64, 128.92, 129.00, 135.01.

### Synthesis of (2R)-1,1-diphenyl-pent-4-ene-2-ol (25b)

(2S)-2-benzhydryl-oxirane (0.61 g, 2.91 mmol) was reacted with vinylmagnesium bromide (7.26 ml of 1.0M solution in THF, 7.26 mmol) in the presence of CuI (0.055 g, 0.29 mmol) (Procedure A) to yield (2R)-1,1-diphenyl-pent-4-ene-2-ol 0.48 g (70%, [α]_{D}=(+)26, c=1, MeOH). The ¹HNMR and ¹³CNMR were identical with (2S)-1,1-diphenyl-pent-4-ene-2-ol.

¹HNMR (CDCl₃, 400MHz) 2.14(m, 1H, H-3), 2.33(m, 1H, H-3), 3.93(d, J=8.8Hz, 1H, H-1) 4.44(m, 1H, H-2) 5.1(m, 2H, H-5), 5.9(m, 1H, H-4), 7.16-7.24 (m, 10H, aromatic-H).

¹³CNMR (CDCl₃, 100MHz) 39.75, 58.21, 73.06, 118.23, 126.86, 127.08, 128.51, 128.64, 128.92, 129.00, 135.01.

### Procedure B. Synthesis of (2S)-1,1-Diphenyl-2-Pent-4-en (26a)

(2S)-1,1-diphenyl-pent-4-en-2-ol **25a** (0.37 g, 1.57 mmol) was dissolved in dry DMF (2 ml) and was added to a suspension of NaH (60% in mineral oil, 0.13 g, 3.14 mmol) in dry DMF (20 ml) at 0°C. The reaction mixture was allowed to reach room temperature over a period of 1h. The reaction mixture was cooled again to 0°C employing an ice-bath, and neat allyl bromide (0.57 g, 4.71 mmol) was added via syringe. The reaction mixture was removed from the ice-bath and stirred overnight at room temperature. The reaction was cooled again to 0°C and quenched by slowly adding H₂O (20 ml). The resulting mixture was extracted with Et₂O (3 × 50 ml), and the combined organic phases were washed in turn with H₂O, brine, and then dried over anhydrous Na₂SO₄. Filtration followed by concentration gave crude product as light orange oil. Purification by chromatography (hexane/ethyl ether = 10:1) gave 0.37g (2S)-1,1-Diphenyl-2-Allyloxy-Pent4-en (85%, [α]_{D}=(+)19.7, c=1, MeOH).

¹HNMR (CDCl₃, 500MHz) 2.26(m, 1H, H-3), 2.38(m, 1H, H-3), 3.74(m, 1H, H-3'), 3.96(m, 1H, H-3'), 4.1(m, 2H, H-1, H-2), 5.0-5.16(m, 4H, H-5, H-1'), 5.71(m, 1H, H-2'), 5.93(m, 1H, H-4), 7.2-7.46(m, 10H, aromatic-H). ¹³CNMR (CDCl₃, 125MHz) 37.27 56.24 71.74 81.80 116.71 117.63 126.49 126.62 128.38 128.70 128.83 129.36 135.21 142.26 142.87.

### Synthesis of (2R)-1,1-Diphennyl-2-Allyloxy-Pent-4-en (26b)

(2R)-1,1-diphenyl-pent-4-en-2-ol **25b** (0.42 g, 1.75 mmol) was reacted with allyl bromide (0.63 g, 5.25 mmol) (Procedure B) to yield (2R)-1, 1-Diphenyl-2-Allyloxy-Pent-4-en **26b,** 0.43 g (87%, [α]_{D}=(-)20, c=1, MeOH). The ¹HNMR and ¹³CNMR were identical with (2R)-1,1-diphenyl-2-alluloxy-pent-4-en shown above.

¹HNMR (CDCl₃, 500MHz) 2.26(m, 1H, H-3), 2.38(m, 1H, H-3), 3.74(m, 1H, H-3'), 3.96(m, 1H, H-3'), 4.1(m, 2H, H-1, H-2), 5.0-5.16(m, 4H, H-5, H-1'), 5.71(m, 1H, H-2'), 5.93(m,1H, H-4), 7.2-7.46(m, 10H, aromatic-H). ¹³CNMR (CDCl₃, 125MHz) 37.27 56.24 71.74 81.80 116.71 117.63 126.49 126.62 128.38 128.70 128.83 129.36 135.21 142.26 142.87.

### Procedure C. Synthesis of (2S)-2-benzhydryl-3.6-dibydro-2H-pyran (27a)

Into a solution of of (2S)-1,1-Diphenyl-2-Allyloxy-Pent-4-ene **26a** (0.19 g, 0.68 mmol) in dry benzene was added Grubb catalyst (0.028 g, 0.034 mmol, 5%) and the solution was refluxed under N₂ for 20h. The solvent was removed, and the residue was purified by flash chromatography (hexane/ether=9:1) to give 0.15 g (2S)-2-benzhydryl-3,6-dihydro-2H-pyran, **27a** (88%, [α]_{D}=(-)79.3, c=1, MeOH).

¹HNMR (CDCl₃, 400MHz) 1.82(m, 1H, H-3) 2.09(m, 1H, H-3) 4.0(d, J=8.8Hz, 1H, Ph₂CH) 4.23(m, 2H, H-6) 4.32(dt, J=2.4Hz, 9.6Hz, H-2) 5.77(m, 2H, H-4, H-5) 7.16-7.26(m, 10H, aromatic-H). ¹³CNMR (CDCl₃, 100MHz) 31.10 51.82 55.52 56.66 67.86 68.03 74.20 126.63 126.86 127.38 128.35 128.81 128.57 128.65 128.74 128.96 142.18 142.37.

### Synthesis of (2R)-2-Benzhydryl-3,6-dihydro-2H-pyran (27b)

(2R)-1,1-Diphenyl-2-Allyloxy-Pent-4-en **26b** (0.25 g, 0.90 mmol) was cyclized in the presence of Grubb's catalyst (0.037 g, 0.045 mmol) (Procedure C) to produce (2R)-2-Benzhydryl-3,6-dihydro-2H-pyran **27b** 0.2 g (89%, [α]_{D}=(+)80.8, c=1, MeOH). The ¹HNMR and ¹³CNMR were identical with (2S)-2-benzhydryl-3,6-dihydro-2H-pyran **27a**.

¹HNMR (CDCl₃, 400MHz) 1.82(m, 1H, H-3) 2.09(m, 1H, H-3) 4.0(d, J=8.8Hz, 1H, Ph₂CH) 4.23(m, 2H, H-6) 4.32(dt, J=2.4Hz, 9.6Hz, H-2) 5.77(m, 2H, H-4, H-5) 7.16-7.26(m, 10H, aromatic-H). ¹³CNMR (CDCl₃, 100MHz) 31.10 51.82 55.52 56.66 67.86 68.03 74.20 126.63 126.86 127.38 128.35 128.81 128.57 128.65 128.74 128.96 142.18 142.37.

### Procedure D. Synthesis of (1S, 4S, 6R)-4-benzhydryl-3,7-dioxa-bicyclo[4.1.0]-heptane (28a) and (1R, 4S, 6S)-4-benzhydryl-3,7-dioxa-bicyclo[4.1.0]-heptane (28b)

To a solution of (2S)-2-benzhydryl-3,6-dihydro-2H-pyran **27a** (0.15 g, 0.6 mmol) in CH₂Cl₂ (20 ml) was added mCPBA (0.3 g, 70%, 1.2 mmol) in a portionwise manner at 0°C. The mixture was brought to room temperature and the reaction mixture was stirred for 20 h under N₂. Na₂SO₃ (20 ml 1.0 M solution) was added to the reaction mixture at 0°C to quench the reaction. The aqueous phase was extracted with CH₂Cl₂ (20 ml x 2). The combined organic phase was washed in turn with saturated NaHCO₃ and brine, then dried over anhydrous Na₂SO₄. Evaporation of the solvent gave light brown solid residue. The crude products were purified by flash chromatography on silica gel (hexane/ethyl ether=9:1) to give 0.08 g (1S, 4S, 6R)-4-benzhydryl-3,7-dioxa-bicyclo[4.1.0]-heptane **28a** (50.3%, [α]_{D}=(-)60, c=1, MeOH) and 0.065 g **28b** (1R, 4S, 6S)-4-benzhydryl-3,7-dioxa-bicyclo[4.1.0]-heptane (41 %, [α]_{D}=(-)76, c=1, MeOH).

(1S, 4S, 6R)-4-benzhydryl-3,7-dioxa-bicyclo[4.1.0]-heptane **28a:** ¹HNMR (CDCl₃, 400MHz) 1.71(m, 1H, H-5) 1.89(m, 1H, H-5) 3.27(m, 1H, H-1) 3.34(m, 1H, H-7) 3.82(d, J=9.6Hz, 1H, Ph₂CH) 3.95(d, J=14Hz, 1H, H-2) 4.14(dt, J=2.4Hz, 10.2Hz, H-4) 4.22(dd, J=4Hz, 12.8Hz, 1H, H-2) 7.16-7.36(m, 10H, aromatic-CH). ¹³CNMR (CDCl₃, 100MHz) 31.1 51.82 55.52 56.67 67.86 68.03 74.20 126.63 126.86 127.38 128.35 128.51 128.57 128.65 128.74 128.96 142.18 142.37.

(1R, 4S, 6S)-4-benzhydryl-3,7-dioxa-bicyclo[4.1.0]-heptane **28b:** ¹HNMR (CDCl₃, 400MHz) 1.66-1.86(m, 2H, H-5) 3.06(m, 1H, H-1) 3.28(m, 1H, H-7) 3.78-3.98(m, 3H, Ph2CH, H-2, H-4) 4.19(d, J=13.6Hz, 1H, H-2) 7.16-7.36(m, 10H, aromatic-CH). ¹³CNMR (CDCl₃, 100MHz) 31.1 51.82 55.52 56.67 67.86 68.03 74.20 126.63 126.86 127.38 128.35 128.51 128.57 128.65 128.74 128.96 142.18 142.37.

### Synthesis of (1R, 4R, 6S)-4-benzhydryl-3,7-dioxa-bicyclo[4.1.0]-heptane (28c) and (1S, 4R, 6R)-4-benzhydryl-3,7-dioxa-bicyclo[4.1.0]-heptane (28d)

(2R)-2-benzhydryl-3,6-dihydro-2H-pyran **27b** (0.2 g, 0.79 mmol) was reacted with mCPBA (0.27 g, 70%, 1.58 mmol) (Procedure D) to yield the corresponding (1R, 4R, 6S)-4-benzhydryl-3,7-dioxa-bicyclo[4.1.0]-heptane **28c** 0.11 g (52%, [α]_{D}=(+)60.4, c=1, MeOH)) and (1S, 4R, 6R)-4-benzhydryl-3,7-dioxa-bicyclo[4.1.0]-heptane **28d** 0.086 g (41%, [α]_{D}=(+)78, c=1, MeOH). The ¹HNMR and ¹³CNMR were identical for both (1S, 4S, 6R)-4-benzhydryl-3,7-dioxa-bicyclo[4.1.0]-heptane and (1R, 4S, 6S)-4-benzhydryl-3,7-dioxabicyclo[4.1.0]-heptane.

(1R, 4R, 6S)-4-benzhydryl-3,7-dioxa-bicyclo[4.1.0]-heptane **28c:** ¹HNMR (CDCl₃, 400MHz) 1.71(m, 1H, H-5) 1.89(m, 1H, H-5) 3.27(m, 1H, H-1) 3.34(m, 1H, H-7) 3.82(d, J=9.6Hz, 1H, Ph₂CH) 3.95(d, J=14Hz, 1H, H-2) 4.14(dt, J=2.4Hz, 10.2Hz, H-4) 4.22(dd, J=4Hz, 12.8Hz, 1H, H-2) 7.16-7.36(m, 10H, aromatic-CH). ¹³CNMR (CDCl₃, 100MHz) 31.1 51.82 55.52 56.67 67.86 68.03 74.20 126.63 126.86 127.38 128.35 128.51 128.57 128.65 128.74 128.96 142.18 142.37.

### Procedure E. Synthesis of (2S, 4R, 5R)-2-benzhydryl-5-(4-methoxy-benzylamino)-tetrahydropyran-4-ol (-)29a (Comparative)

A mixture of (1S, 4S, 6R)-4-benzhydryl-3,7-dioxa-bicyclo[4.1.0]-heptane 28a (0.027 g, 0.10 mmol) and p-methoxybenzylamine (0.28 g, 2.03 mmol) in ethanol (1 ml) was refluxed under N₂ overnight. The solvent was removed and the residue was purified by flash chromatography on silica gel (hexane/ethyl acetate/Et₃N=6:4:0.2) to give (2S, 4R, 5R)-2-benzhydryl-5-(4-methoxy-benzylamino)-tetrahydropyran-4-ol, **(-)-29a,** 0.03 g (73.2%, [α]_{D}=(-)71.9, c=1, MeOH).

¹HNMR (CDCl₃, 400MHz) 1.42(m,1H, H-3) 1.72(m, 3H, H-3, NH, OH) 2.44(m, 1H, H-5) 3.66(d, J=12.8Hz, H-6) 3.74-3.84(m, 5H, -OCH3, Ph-CH2) 3.87-3.98(m, 3H, H-4, H-6, Ph₂CH) 4.50(dt, J=2.4Hz, 9.6Hz, 1H, H-2) 6.80-7.40(m, 14H, aromatic-CH). ¹³CNMR (CDCl₃, 100MHz) 33.69 51.04 55.51 56.71 56.79 65.08 67.82 73.81 114.03 126.55 126.75 128.61 128.87 129.47 142.18 142.37.

Free base was converted into oxalate: mp 230-232°C. C, H, N Anal: [C₂₆H₁₉NO₃· (COOH)₂].

### Synthesis of (2R, 4S, 5S)-2-benzhydryl-5-(4-methoxy-benzylamino)-tetahydro-4-ol(+)29a (Comparative)

(1R, 4R, 6S)-4-benzhydryl-3,7-dioxa-bicyclo[4.1.0]-heptane **28c** (0.02 g, 0.075 mmol) was reacted with p-methoxybenzylamine (0.21 g, 1.50 mmol) in ethanol (Procedure E) to yield (2R, 4S, 5S)-2-benzhydryl-5-(4-methoxy-benzylamino)-tetrahydropyran-4-ol **(+)-29a** 0.024 g (80%, [α]_{D}=(+)72.8, c=1, MeOH). The ¹HNMR and ¹³CNMR were identical with (2S, 4R, 5R)-2-benzhydryl-5-(4-methoxy-benzylamino)-tetrahydropyran-4-ol.

¹HNMR (CDCl₃, 400MHz) 1.42(m,1H, H-3) 1.72(m, 3H, H-3, NH, OH) 2.44(m, 1H, H-5) 3.66(d, J=12.8Hz, H-6) 3.74-3.84(m, 5H, -OCH3, Ph-CH2) 3.87-3.98(m, 3H, H-4, H-6, Ph₂CH) 4.50(dt, J=2.4Hz, 9.6Hz, 1H, H-2) 6.80-7.40(m, 14H, aromatic-CH). ¹³CNMR (CDCl₃, 100MHz) 33.69 51.04 55.51 56.71 56.79 65.08 67.82 73.81 114.03 126.55 126.75 128.61 128.87 129.47 142.18 142.37.

Free base **(+)-29a** was converted into the oxalate: mp 230-232°C. C, H, N Anal: [C₂₆H₂₉NO₃· (COOH)₂.0.5H₂O].

### Synthesis of (2R, 4S, 5S)-2-benzhydryl-5-benzylamino-tetrahydro-pyran-4-ol (+)29d

### (Comparative)

(1R, 4R, 6S)-4-benzhydryl-3,7-dioxa-bicyclo[4.1.0]-heptane **28c** (0.022 g, 0.082 mmol) was reacted with benzylamine (0.18 g, 1.64 mmol) in ethanol (Procedure E) to yield (2R, 4S, 5S)-2-benzhydryl-5-benzylamino-tetrahydro-pyran-4-ol, **(+)-29d** 0.025 g (81 %, [α]_{D}=(+)53.7, c=1, MeOH).

¹HNMR (CDCl₃, 400MHz) 1.43(m,1H, H-3) 1.62-1.80(m, 3H, H-3, NH, OH) 2.54(m, 1H, H-5) 3.73(d, J=13.6Hz, 1H, Ph-CH2) 3.79(m, 1H, H-6) 3.86-4.02(m, 4H, H-4, H-6, Ph₂CH, Ph-CH2) 4.50(dt, J=2.4Hz, 9.6Hz, 1H, H-2) 7.00-7.40(m, 15H, aromatic-CH). ¹³CNMR (CDCl₃, 100MHz) 33.67 51.64 56.78 56.83 65.10 67.83 73.80 126.57 126.77 127.24 128.30 128.63 128.89 142.25 142.34.

Free base **(+)-29d** was converted into the oxalate: mp 249-251°C. C, H, N Anal: [C₂₅H₂₇NO₂. (COOH)₂ · 0.3H₂O].

### Synthesis of (2S, 4R, 5R)-2-benzhydryl-5-benzylamino-tetrahydro-pyran-4-ol (-)29d

### (Comparative)

(1S, 4S, 6R)-4-Benzhydryl-3,7-dioxa-bicyclo[4.1.0]-heptane **28a** (0.03 g, 0.09 mmol) reacted with benzylamine (0.20 g, 1.88 mmol) in ethanol (Procedure E) to yield **(-)-29d,** 0.03 g (Yield; 86%), [C]_{D}=(-)54.0, c=1, MeOH).
¹HNMR (CDCl₃, 400 MHz): 1.43 (m, 1H, H-3eq), 1.69 (s, 2H, NH, OH), 1.74 (dt, J = 2.8 Hz, 10.8 Hz, 1H, H-3ax), 2.45 (m, 1H, H-5), 3.73 (d, J = 13.2 Hz, 1H, Ph-CH₂), 3.79 (dd, J = 2.0 Hz, 12.0 Hz, 1H, H-6), 3.86-4.02 (m, 4H, H-4, H-6, Ph₂CH, Ph-CH₂), 4.50 (dt, J = 2.4 Hz, 10.0 Hz, 1H, H-2), 7.00-7.40 (m, 15H, aromatic-CH).

Free base was converted into oxalate: mp 250-252 CC Anal. [C₂₅H₂₇NO₂C (COOH)₂0.5H₂O] C, H, N.

### Synthesis of (3R, 4R, 6S)-6-benzhydryl-4-(4-methoxy-benzylamino)-tetrahydro-pyran-3-ol(-)29g (Comparative)

(1R, 4S, 6S)-4-benzhydryl-3,7-dioxa-bicyclo[4.1.0]heptane **28b** (0.021 g, 0.079 mmol) was reacted with p-methoxybenzylamine (0.22 g, 1.58 mmol) (Procedure E) to yield (3R, 4R, 6S)-6-benzhydryl-4-(4-methoxy-benzylamino)-tetrahydropyran-3-ol, **(-)-29g** 0.02 g (63%, [α]_{D}=(-)63.75, c=1, MeOH).

¹HNMR (CDCl₃, 400MHz) 1.37 (m, 1H, H-5) 1.81 (m, 1H, H-5) 2.95 (m, 1H, H-4) 3.46 (m, 1H, H-3) 3.56-3.72 (m, 3H, H-2, PhCH2) 3.81 (s, 3H, -OCH3) 3.96 (d, J=9.6Hz, 1H, Ph₂CH) 4.04 (dd, J=1.6Hz, 12Hz, 1H, H-2) 4.53 (dt, J=2.4Hz, 9.6Hz, 1H, H-6) 6.8-7.4 (m, 14H, aromatic-CH). ¹³CNMR (CDCl₃, 100MHz) 31.14 51.23 55.45 55.53 56.64 67.84 68.05 74.20 126.63 126.86 127.38 128.35 128.51 128.57 128.65 128.74 128.96 142.18 142.37.

Free base (-)-**29g** was converted into the oxalate: mp 234-235°C. C, H, N Anal: [C₂₆H₂₉NO₃ · (COOH)₂ · 0.2H₂O].

### Synthesis of (3S, 4S, 6R)-6-benzhydryl-4-(4-methoxy-benzylamino)-tetrahydro-pyran-3-ol (+)29g (Comparative)

(1S, 4R, 6R)-4-benzhydryl-3,7-dioxa-bicyclo[4.1.0]heptane **28d** (0.02 g, 0.075 mmol) was reacted with p-methoxy-benzylamine (0.21 g, 1.50 mmol) (Procedure E) to yield (3S, 4S, 6R)-6-benzhydryl-4-(4-methoxy-benzylamino)-tetrahydropyran-3-ol, **(+)-29g**, 0.029 (94%, [α]_{D}=(+)65, c=1, MeOH). The ¹HNMR and ¹³CNMR were identical with (3R, 4R, 6S)-6-benzhydryl-4-(4-methoxy-benzylamino)-tetrahydropyran-3-ol.

¹HNMR (CDCl₃, 400MHz) 1.37 (m, 1H, H-5) 1.81 (m, 1H, H-5) 2.95 (m, 1H, H-4) 3.46 (m, 1H, H-3) 3.56-3.72 (m, 3H, H-2, PhCH2) 3.81 (s, 3H, -OCH3) 3.96 (d, J=9.6Hz, 1H, Ph₂CH) 4.04 (dd, J=1.6Hz, 12Hz, 1H, H-2) 4.53 (dt, J=2.4Hz, 9.6Hz, 1H, H-6) 6.8-7.4 (m, 14H, aromatic-CH). ¹³CNMR (CDCl₃, 100MHz) 31.14 51.23 55.45 55.53 56.64 67.84 68.05 74.20126.63 126.86 127.38 128.35 128.51 128.57 128.65 128.74 128.96 142.18 142.37.

Free base **(+)-29g** was converted into the oxalate: mp 235-237°C. C, H, N Anal: [C₂₆H₂₉NO₃ · (COOH)₂ · 0.2H₂O].

### Synthesis of (3S, 4S, 6R)-6-benzhydryl-4-benzylamino-tetrahydropyran-3-ol (+)29h (Comparative)

(1S, 4R, 6R)-4-benzhydryl-3,7-dioxa-bicyclo[4.1.0]heptane **28d** (0.019 g, 0.071 mmol) was reacted with benzylamine (0.15 g, 1.43 mmol) (Procedure E) to yield (3S, 4S, 6R)-6-benzhydryl-4-benzylamino-tetrahydropyran-3-ol, **(+)-29h,** 0.023 (85%, [α]=(+)70.1, c=1, MeOH).

¹HNMR (CDCl₃, 400MHz) 1.38 (m, 1H, H-5) 1.81 (m, 1H, H-5) 2.96 (m, 1H, H-4) 3.48 (m, 1H, H-3) 3.62-3.78 (m, 3H, H-2, PhCH2) 3.96 (d, J=9.6Hz, 1H, Ph₂CH) 4.05 (m, 1H, H-2) 4.54 (dt, J=2.4Hz, 9.6Hz, 1H, H-6) 7.0-7.4 (m, 15H, aromatic-CH). ¹³CNMR (CDCl₃, 100MHz) 31.10 51.82 55.52 56.66 67.86 68.03 74.20 126.63 126.86 127.38 128.35 128.51 128.57 128.65 128.74 128.96 142.18 142.37.

Free base **(+)-29h** was converted into the oxalate: mp 259-260°C. C, H, N Anal: [C₂₅H₂₇NO₂ · (COOH)₂ · 0.25H₂O].

### Synthesis of (2S, 4R, 5R)-2-benzhydryl-5-(4-fluoro-benzylamino)-tetrahydro-pyran-4ol (-)29b (Comparative)

(1S, 4S, 6R)-4-benzhydryl-3,7-dioxa-bicyclo[4.1.0]-heptane **28a** (0.025 g, 0.094 mmol) was reacted with para-fluoro-benzylamine (0.24 g, 1.88 mmol) in ethanol (Procedure E) to yield (2S, 4R, 5R)-2-benzhydryl-5-(4-fluoro-benzylamino)-tetrahydropyran-4-ol, **(-)-29b,** 0.032 g (86%, [α]_{D}=(-)77.2, c=1, MeOH).

¹HNMR (CDCl₃, 400MHz) 1.40(m, 1H, H-3) 1.71(m, 1H, H-3) 1.78(bs, 2H, NH, OH) 2.41(m, 1H, H-5) 3.66(d, J=13.2Hz, 1H, H-6) 3.72-3.96(m, 5H, H-4, H-6, Ph₂CH, PhCH2) 4.49(dt, J=2.4Hz, 10.4Hz, 1H, H-2) 6.8-7.4(m, 14H, aromatic-CH). ¹³CNMR (CDCl₃, 100MHz) 33.69 50.85 56.70 56.85 65.05 67.70 73.80 115.29 115.50 126.57 126.77 128.61 128.64 128.86 129.74 129.83 142.19 142.31.

Free base **(-)-29b** was converted into the oxalate: mp 222-223°C. C, H, N Anal: [C₂₅H₂₆NFO₂ · (COOH)₂].

### Synthesis of (2R, 4S, 5S)-2-benzhydryl-5-(4-fluoro-benzylamino)-tetrahydro-pyran-4-ol (+)29b (Comparative)

(1R, 4R, 6S)-4-benzhydryl-3,7-dioxa-bicyclo[4.1.0]-heptane, **28c,** (0.02 g, 0.075 mmol) was reacted with para-fluoro-benzylamine (0.19 g, 1.50 mmol) in ethanol (Procedure E) to yield (2R, 4S, 5S)-2-benzhydryl-5-(4-fluoro-benzylamino)-tetrahydropyran-4-ol, **(+)-29b**, 0.028 g (94%, [α]_{D}=(+)77.6, c=1, MeOH).

¹HNMR (CDCl₃, 400MHz) 1.43(m, 1H, H-3) 1.68-1.78(m, 3H, H-3, NH, OH) 2.43(m, 1H, H-5) 3.68(d, J=13.2Hz, 1H, H-6) 3.74-4.00(m, 5H, H-4, H-6, Ph₂CH, PhCH2) 4.50(dt, J=2.4Hz, 10.4Hz, 1H, H-2) 6.8-7.4(m, 14H, aromatic-CH). ¹³CNMR (CDCl₃, 100MHz) 33.7150.87 56.72 56.85 65.06 67.75 73.81 115.30115.51 126.57 126.78 128.61 128.65 128.87 129.75 129.83 142.20 142.31.

Free base (+)-29b was converted into the oxalate: mp 223-225°C. C, H, N Anal: [C₂₅H₂₆NFO₂ · (COOH)₂ · 0.2H₂O].

### Synthesis of (2S, 4R, 5R)-2-benzhydryl-5-[2-(4-fluoro-phenyl)-ethylamino]-tetrahydropyran-4-ol (-)29c (Comparative)

(1S, 4S, 6R)-4-benzhydryl-3,7-dioxa-bicyclo[4.1.0]-heptane **28a** (0.025 g, 0.094 mmol) was reacted with 2-(4-fluoro-phenyl)-ethylamine (0.26 g, 1.88 mmol) in ethanol (Procedure E) to yield (2S, 4R, 5R)-2-benzhydryl-5-[2-(4-fluorophenyl)-ethylamino]-tetrahydropyran-4-ol, **(-)-29c,** 0.04 g (98%, [α]_{D}=(-)62.9, c=1, MeOH).

¹HNMR (CDCl₃, 400MHz) 1.40(m, 1H, H-3) 1.63(m, 1H, H-3) 1.84(s, 2H, NH, OH) 2.43(m, 1H, H-5) 2.73, 2.92(m, 4H, (F)PhCH2CH2) 3.70(dd, J=2Hz, 11.6Hz, 1H, H-6) 3.86-3.98(m, 3H, H-4, H-6, Ph₂CH) 4.49(dt, J=2.4Hz, 10Hz, 1H, H-2) 6.8-7.4(m, 14H, aromatic-CH). ¹³CNMR (CDCl₃, 100MHz) 33.70 36.19 49.28 56.74 57.66 65.21 67.35 73.81 115.34 115.55 126.58 126.79 128.61 128.88 130.20 130.30 142.18 142.30.

Free base (-)-29c was converted into the oxalate: mp 205-207°C. C, H, N Anal: [C₂₆H₂₈NFO₂ · (COOH)₂ · 0.1H₂O].

### Synthesis of (2R, 4S, 5S)-2-benzhydryl-5-[2-(4-fluoro-phenyl)-ethylamino]-tetrahydropyran-4-ol (+)29c (Comparative)

(1R, 4R, 6S)-4-benzhydryl-3,7-dioxa-bicyclo[4.1.0]-heptane **28c** (0.02 g, 0.075 mmol) was reacted with 2-(4-fluorophenyl)-ethylamine (0.21 g, 1.50 mmol) in ethanol (Procedure E) to yield (2R, 4S, 5S)-2-benzhydryl-5-[2-(4-fluorophenyl)-ethylamino]-tetrahydropyran-4-ol, **(+)-29c,** 0.030 g (98%, Cα]_{D}=(+)63.4, c=1, MeOH).

¹HNMR (CDCl₃, 400MHz) 1.40(m, 1H, H-3) 1.63(m, 1H, H-3) 1.84(s, 2H, NH, OH) 2.43(m, 1H, H-5) 2.73, 2.92(m, 4H, (F)PhCH2CH2) 3.70(dd,1=2Hz, 11.6Hz, 1H, H-6) 3.86-3.98(m, 3H, H-4, H-6, Ph₂CH) 4.49(dt, J=2.4Hz, 10Hz, 1H, H-2) 6.8-7.4(m, 14H, aromatic-CH). ¹³CNMR (CDCl₃, 100MHz) 33.72 36.26 49.33 56.74 57.67 65.28 67.47 73.80 115.33 115.53 126.57 126.78 128.61 128.88 130.22 130.30 142.19 142.30.

Free base **(+)-29c** was converted into the oxalate: mp 203-205°C. C, H, N Anal: [C₂₆H₂₈NFO₂ · (COOH)₂ · 0.5H₂O].

### Synthesis of (3S, 4R, 6S)-6-benzhydryl-4-benzyhmino-tetrahydropyran-3-ol (- )29h (Comparative)

(1R, 4S, 6S)-4-benzhydryl-3,7-dioxa-bicyclo[4.1.0]heptane **28b** (0.03 g, 0.09 mmol) reacted with benzylamine (0.20 g, 1.88 mmol) (Procedure E) to yield (3S, 4R, 6S)-6-benzhydryl-4-benzylamino-tetrahydropyran-3-ol, **(-)-29h,** 0.03 g (Yield; 86%), [α]_{D}=(-)70.6, c=1, MeOH).

¹HNMR (CDCl₃, 400MHz): 1.30 (td, J=3.2Hz, 14Hz, 1H, H-5eq), 1.68-1.80 (m, 3H, H-5ax, NH, OH), 2.88 (m, 1H, H-4), 3.40 (m, 1H, H-3), 3.54-3.70 (m, 3H, H-2, PhCH₂, 3.88 (d, J=9.60Hz, 1H, Ph₂CH), 3.96 (dd, J=1.60Hz, 12.00Hz, 1H, H-2), 4.46 (dt, J=2.40Hz, 10.00Hz, 1H, H-6) 7.00-7.40 (m, 15H, aromatic-CH).

Free base **(-)-29h** was converted into the oxalate: mp 259-260 °C. C, H, N Anal: [C₂₅H₂₇NO₂ · (COOH)₂ · 0.25H₂O].

### Synthesis of (2S, 4R, 5R)-2-benzhydryl-5-(3,5-dimethoxy-benzylamino)-tetrahydropyran-4-ol (-)-29f (Comparative)

(1S, 4S, 6R)-4-benzhydryl-3,7-dioxa-bicyclo[4.1.0]-heptane **28a** (0.020 g, 0.075 mmol) was reacted with 3,5-dimethoxybenzylamine (0.25 g, 1.50 mmol) (Procedure E) to yield (2S, 4R, 5R)-2-benzhydryl-5-(3,5-dimethoxy-benzylamino)-tetrahydropyran-4-ol, **(-)-29f,** 0.03 g (Yield; 95%, [α]_{D}=(-)58.60, c=1, CHCl₃).

¹HNMR (CDCl₃, 400MHz): 1.40 (m, 1H, H-3), 1.72 (m, 1H, H-3), 2.42 (m, 1H, H-5), 3.62-4.00 (m, 12H, H-4, H-6, PhCH₂, -OCH₃, Ph₂CH), 4.49 (dt, J=2.00Hz, 10.00Hz, 1H, H-2), 6.34, 6.48, 7.10-7.40 (m, 13H, aromatic-CH).

Free base **(-)-29f** was converted into oxalate: mp 245-247 °C. C, H, N Anal: [C₂₇H₃₁NO₄ · (COOH)₂ · 0.2H₂O].

### Synthesis of (2S, 4R, 5R)-2-benzhydryl-5-(2,4-dimethoxy-benzylamino)-tetrahydropyran-4-ol (-)-29e (Comparative)

(1S, 4S, 6R)-4-benzhydryl-3,7-dioxa-bicyclo[4.1.0]heptane **28a** (0.020 g, 0.075 mmol) was reacted with 2,4-dimethoxybenzylamine (0.25 g, 1.50 mmol) (Procedure E) to yield (2S, 4R, 5R)-2-benzhydryl-5-(2,4-dimethoxybenzylamino)-tetrahydropyran-4-ol, **(-)-29e,** 0.025 g (Yield; 70%, [α]_{D}=(-)3.70, c=1, CHCl₃).

¹HNMR (CDCl₃, 400MHz): 1.42 (m, 1H, H-3), 1.77 (m, 1H, H-3), 2.10(bs, 2H, OH, -NH), 2.47 (m, 1H, H-5), 3.66-4.06 (m, 12H, H-4, H-6, PhCH₂, -OCH₃, Ph₂CH), 4.50 (dt, J=2.80Hz, 9.60Hz, 1H, H-2), 6.40, 7.10-7.40 (m, 13H, aromatic-CH).

Free base **(-)-29e** was converted into the oxalate: mp 208-210 °C. C, H, N Anal: [C₂₇H₃₁NO₄ · (COOH)₂].

### Procedure F. Synthesis of (2S, 4R, 5R)-5-Azido-2-benzhydryl-tetrahydro-pyran-4-ol (30a) (Comparative)

A solution of (1S, 4S, 6R)-4-benzhydryl-3,7-dioxa-bicyclo[4.1.0]-heptane **28a** (0.05 g, 0.19 mmol) in a 8:1 MeOH/H₂O (2 ml) mixture was treated with NaN₃ (0.061 g, 0.94 mmol) and NH₄Cl (0.022 g, 0.41 mmol) and the resulting reaction mixture was stirred at 80°C overnight. The reaction mixture was diluted with ether and the organic layer was separated. Evaporation of the washed (saturated aqueous NaHCO₃, water) ether extracts afforded a crude solid product. Purification of the product by flash chromatography (Hexane/Ethyl Acetate = 4: 1) yielded (2S, 4R, 5R)-5-Azido-2-benzhydryl-tetrahydropyran-4-ol **30a** 0.05 g (95 % , [α]_{D}=(-)109.3, c=1, MeOH).

¹HNMR (CDCl₃, 400MHz) 1.44(m, 1H, H-3) 1.79(m, 1H, H-3) 1.91(s, 1H, OH) 3.258(m, 1H, H-5) 3.82-4.04(m, 4H, H-4, H-6, Ph₂CH) 4.49(dt, J=2.4Hz, 10Hz, 1H, H-2) 7.0-7.4(m, 10H, aromatic-CH). ¹³CNMR (CDCl₃, 100MHz) 33.56 56.96 59.63 64.81 66.32 73.56 126.64 126.88 128.62 128.64 128.67 128.92 142.04.

### Synthesis of (2R, 4S, 5S)-5-Azido-2-benzhydryl-tetrahydro-pyran-4-ol (30b)

(1R, 4R, 7S)-4-benzhydryl-3,7-dioxa-bicyclo[4.1.0]-heptane **8c** (0.04 g, 0.15 mmol) was treated with NaN₃ (0.05 g, 0.75 mmol) and NH₄Cl (0.018 g, 0.33 mmol) (Procedure F) yielded (2R, 4S, 5S)-5-Azido-2-benzhydryl-tetrahydro-pyran-4-ol **30b**, 0.04 g (95%, [α]_{D}=(+)108, c=1, MeOH).

¹HNMR (CDCl₃, 400MHz) 1.45(m, 1H, H-3) 1.80(m, 1H, H-3) 1.91(s, 1H, OH) 3.27(m, 1H, H-5) 3.84-4.05(m, 4H, H-4, H-6, Ph₂CH) 4.50(dt, J=2.4Hz, 10Hz, 1H, H-2) 7.0-7.4(m, 10H, aromatic-CH). ¹³CNMR (CDCl₃, 100MHz) 33.59 56.96 59.64 64.81 66.35 73.56 126.64 126.87 128.62 128.64 128.67 128.92 142.06.

### Procedure G. Synthesis of (2S, 4R, 5R)-5-Amino-2-benzhydryl-tetrahydro-pyran-4-ol (31a)

(2S, 4R, 5R)-5-Azido-2-benzhydryl-tetrahydro-pyran-4-ol (0.05 g, 0.18 mmol) dissolved in methanol (20 ml) was hydrogenated in the presence of 10% Pd/C (0.006 g). The mixture was filtered through a short bed of cellite, and evaporation of the solvent gave (2S, 4R, 5R)-5-amino-2-benzhydryl-tetrahydropyran-4-ol 0.05 g (97%, [α]_{D}=(-)66, c=1, MeOH), which was pure enough for the next reaction.

¹HNMR (CDCl₃, 400MHz) 1.40(m, 1H, H-3) 1.70(m, 1H, H-3) 2.73(s, 1H, H-5) 3.20(m, 3H, NH, OH) 3.60(m, 1H, H-6) 3.8-4.0(m, 3H, H-4, H-6, Ph₂CH) 4.46(t, J=10Hz, 1H, H-2) 7.0-7.4(m, 10H, aromatic-CH). ¹³CNMR (CDCl₃, 100MHz) 32.87 51.26 56.68 67.25 67.85 74.15 126.60 126.82 128.61 128.65 128.89 142.15 142.18.

### Synthesis of (2R, 4S, 5S)-5-Amino-2-benzhydryl-teahydro-pyran-4-ol (31b)

(2R, 4S, 5S)-5-Azido-2-benzhydryl-tetrahydropyran-4-ol (0.05 g, 0.14 mmol) was hydrogenated (Procedure G) to yield (2R, 4S, 5S)-5-amino-2-benzhydryl-tetrahydropyran-4-ol 0.04 g (97%, [α]_{D}=(+)66.2, c=1, MeOH).

¹HNMR (CD30D, 400MHz) 1.43(m, 1H, H-3) 1.72(m, 1H, H-3) 2.65(m, 1H, H-5) 3.57(m, 1H, H-6) 3.82(m, 1H, H-4) 3.92-4.0(m, 2H, H-6, Ph₂CH) 4.52(dt, J=2Hz, 10.4Hz, 1H, H-2) 7.0-7.4(m, 10H, aromatic-CH). ¹³CNMR (CD3OD, 100MHz) 32.40 50.67 56.92 66.65 67.47 74.04 125.96 126.35 128.01 128.38 128.42 142.44 142.77.

### Procedure H. Synthesis of (2S, 4R, 5R)-2-benzhydryl-5-(4-hydroxy-benzylamino)-tetrahydropyran-4-ol (-)32a (Comparative)

To a solution of (2S, 4R, 5R)-5-amino-2-benzhydryl-tetrahydro-pyran-4-ol 31a (0.02 g, 0.09 mmol), 4-hydroxybenzaldehyde (0.01 g, 0.09 mmol) and glacial acetic acid (0.005 g, 0.09 mmol) in 1,2-dichloroethane (5 ml) was added portionwise NaCNBH₃ (0.007 g, 0.11 mmol) in methanol (1 ml). The reaction was continued for 4 hr. Water was added to quench the reaction and the mixture was stirred for 30 minutes at 0°C. The reaction mixture was stirred with saturated aqueous NaHCO₃ and the product was extracted with methylene chloride (3 x 10 ml). The combined organic phases were washed with brine, water and dried over anhydrous Na₂SO₄. Solvent was removed under reduced pressure, and the residue was purified by flash chromatography (Hexane/Ethyl Acetate/Triethylamine 3:2:0.2) to give (2S, 4R, 5R)-2-benzhydryl-5-(4-hydroxy-benzylamino)-tetrahydropyran-4-ol, (-)-**32a**, 0.03 g (80%, [α]_{D}=(-)72.6, c=1, MeOH).

¹HNMR (CDCl₃, 400MHz) 1.40(m, 1H, H-3) 1.66(m, 1H, H-3) 2.45(s, 1H, H-5) 3.23(bs, NH, OH) 3.58(d, J=12.4Hz, 1H, (OH)PhCH2) 3.7-3.8(m, 2H, H-6, (OH)PhCH2) 3.84-4.0(m, 3H, H-4, H-6, Ph₂CH) 4.49(dt, J=2Hz, 10Hz, 1H, H-2) 6.57, 7.03, 7.1-7.36(m, 14H, aromatic-CH). ¹³CNMR (CDCl₃, 100MHz) 33.56 50.86 56.49 56.60 64.55 67.19 73.95 115.82 126.61 126.79 128.59 128.64 128.68 128.87 129.91 130.87 142.09 142.22 155.61.

Free base (-)-**32a** was converted into the oxalate: C, H, N Anal: [C₂₅H₂₁NO₃ · (COOH)₂ · 0.4H₂O].

### Synthesis of (2S, 4R, 5R)-2-benzhydryl-5-[(1H-indoi-5-ylmethyl)-amino]-tetrahydropyran-4-ol (-)32b (Comparative)

(2S, 4R, 5R)-5-amino-2-benzhydryl-tetrahydropyran-4-ol **31a** (0.03 g, 0.11 mmol) was reacted with 1H-indol-5-carbaldehyde (0.02 g, 0.11 mmol), glacial acetic acid (0.01 g, 0.11 mmol), and NaCNBH₃(0.01 g, 0.21 mmol) (Procedure C) to give (2S, 4R, 5R)-2-benzhydryl-5-[(1H-indol-5-ylmethyl)-amino]-tetrahydropyran-4-ol, (-)32b, 0.04 g (92%, [α]_{D}=(-)69.90, c=1, Acetone).

¹HNMR (DMSO, 400MHz): 1.24 (m, 1H, H-3eq), 1.63 (dt, J=2.80Hz, 12.00Hz, 1H, H-3ax), 2.35 (m, 1H, H-5), 3.35 (bs, NH, OH), 3.61 (d, J=10.40Hz, 1H, H-6), 3.68-3.90 (m, 4H, H-4, H-6, indol-CH₂), 3.97(d, J=10.00Hz, 1H, Ph₂CH), 4.45 (dt, J=2.00Hz, 10.00Hz, 1H, H-2), 6.40, 7.00-7.60 (m, 15H, aromatic-CH). ¹³CNMR (DMSO, 100MHz): 33.81, 51.72, 56.67, 56.87, 65.03, 65.93, 73.78, 101.52, 111.73, 120.01, 122.45, 126.00, 126.46, 126.88, 128.21, 128.78, 128,95, 128.99, 129.13, 135.69, 143.35, 143.93.

Free base **(-)-32b** was converted into the oxalate: C, H, N Anal: [C₂₇H₂₈N₂O₂ · (COOH)₂ · 0.5H₂O].

### Synthesis of (2R, 4S, 5S)-2-benzhydryl-5-(4-hydroxy-benzylamino)-tetrahydro-pyran-4-ol (+)32a (Comparative)

(2R, 4S, 5S)-5-amino-2-benzhydryl-tetrahydropyran-4-ol **31b** (0.02 g, 0.07 mmol) was reacted with 4-hydroxybenzaldehyde (0.009 g, 0.071 mmol), glacial acetic acid (0.004 g, 0.071 mmol) and NaCNBH₃ (0.005 g, 0.085 mmol) (Procedure H) to give (2R, 4S, 5S)-2-benzhydryl-5-(4-hydroxy-benzylamino)-tetrahydropyran-4-ol, (+)-**32a**, 0.023 g (85%, [α]_{D}=(+)72.4, c=1, MeOH).

¹HNMR (CDCl₃, 400MHz) 1.42(m, 1H, H-3) 1.68(m, 1H, H-3) 2.46(m, 1H, H-5) 3.52(bs, NH, OH) 3.60(d, J=13.6Hz, 1H, (OH)PhCH2) 3.72-3.82(m, 2H, H-6, (OH)PhCH2) 3.86-4.0(m, 3H, H-4, H-6, Ph₂CH) 4.50(dt, J=2.4Hz, 10.4Hz, 1H, H-2) 6.58, 7.05, 7.1-7.36(m, 14H, aromatic-CH). ¹³CNMR (CDCl₃, 100MHz) 33.62 50.94 56.59 64.64 67.36 73.93 115.78 126.62 126.79 128.59 128.64128.69 128.88 129.87 142.08 142.23 155.51.

Free base (+)-**32a** was converted into the oxalate: C, H, N Anal: [C₂₅H₂₇NO₃ · (COOH)₂ · 0.4H₂O].

### Synthesis of (2R, 4S, 5S)-2-benzhydryl-5-[(1H-indol-5-ylmethyl)-amino]-tetrahydropyran-4-ol (+)32b (Comparative)

(2R, 4S, 5S)-5-amino-2-benzhydryl-tetrahydropyran-4-ol **31b** (0.05 g, 0.18 mmol) was reacted with 1H-indol-5-carbaldehyde (0.03 g, 0.18 mmol), glacial acetic acid (0.01 g, 0.18 mmol) and NaCNBH₃(0.02 g, 0.35 mmol) (Procedure C) to give (2R, 4S, 5S)-2-benzhydryl-5-[(1H-indol-5-ylmethyl)-amino]-tetrahydropyran-4-ol, (+) **32b**, 0.05 g (69%, [α]_{D}=(+)70.9, c=1, Acetone).

¹HNMR (Acetone, 400MHz): 1.27 (td, J=2.80Hz, 14.00Hz, 1H, H-3eq), 1.61 (dt, J=2.80Hz, 14.00Hz, 1H, H-3ax), 2.34 (m, 1H, H-5), 3.58 (d, J=12.00Hz, 1H, H-6), 3.68-3.90 (m, 5H, H-4, H-6, indol-CH₂, Ph₂CH), 4.41 (dt, J=2.40Hz, 10.00Hz, 1H, H-2), 6.28, 6.94-7.44 (m, 15H, aromatic-CH). ¹³CNMR (Acetone, 100MHz): 33.59, 51.76, 56.74, 57.07, 64.94, 66.47, 73.74, 101.59, 111.20, 119.95, 122.39, 125.07, 126.01, 126.39, 128.20, 128.57, 128.74, 128.89, 143.22, 143.50.

Free base (+)-**32b** was converted into the oxalate: C, H, N Anal: [C₂₇H₂₈N₂O₂ · (CoOH)₂].

### Procedure I. Synthesis of (3R, 6S)-6-benzhydryl-tetrahydropyran-3-ol (33a)

(1S, 4S, 6R)-4-benzhydryl-3,7-dioxa-bicyclo[4.1.0]heptane **28a** (0.3 g, 1.13 mmol) in dry pentane (10 ml) was added to a suspension of LiAlH₄ (0.21 g, 5.64 mmol) in dry pentane (20 ml). The resulting reaction mixture was stirred under N₂ for 20 hr at room temperature, and then quenched with 10% NaOH, diluted with ethyl acetate (30 ml), and the precipitate removed by filtration. The organic phase was washed with brine and dried over anhydrous Na₂SO₄. Removal of solvent followed by flash chromatography of the crude product produced pure (3R, 6S)-6-benzhydryl-tetrahydro-pyran-3-ol, **33a**, 0.23 g (75%, [α]_{D}=(-)-61.6, c=1, MeOH).

¹HNMR (CDCl₃, 400MHz) 1.40(m, 2H, H-5) 1.58(m, 1H, H-4) 2.07(m, 1H, H-4) 3.14(t, J=10.4Hz, 1H, H-2) 3.69(m, 1H, H-3) 3.82-4.04(m, 3H, H-2, H-6, Ph₂CH) 7.1-7.4(m, 10H, aromatic-CH). ¹³CNMR (CDCl₃, 100MHz) 29.47 33.18 57.40 66.55 73.12 78.95 126.51 126.74 128.54 128.60 128.79 142.41 142.77.

### Synthesis of (3S, 6R)-6-benzhvdrpl-tetrahydropyran-3-ol (33b)

(1R, 4R, 6S)-4-benzhydryl-3,7-dioxa-bicyclo[4.1.0]-heptane (0.05 g, 0.19 mmol) was treated with LiAlH4 (0.036 g, 0.94 mmol) (Procedure I) in dry pentane to yield trans-(3S, 6R)-6-benzhydryl-tetrahydro-pyran-3-ol **33b** 0.035 g (70%, [α]_{D}=(+)61.7, c=1, MeOH).

¹HNMR (CDCl₃, 400MHz) 1.40 (m, 2H, H-5), 1.58 (m, 1H, H-4), 2.07 (m, 1H, H-4), 3.14 (t, J= 10.4Hz, 1H, H-2), 3.69 (m, 1H, H-3), 3.82-4.04 (m, 3H, H-2, H-6, Ph₂CH), 7.1-7.4 (m, 10H, aromatic-CH). ¹³CNMR (CDCl₃, 100MHz) 29.47 33.18 57.40 66.55 73.12 78.95 126.51 126.74 128.54 128.60 128.79 142.41 142.77.

### An Alternative Procedure for the synthesis of (3S, 6R)-6-benzhydryl-tetrahydro-pyran-3-ol (33b)

### Synthesis of (3R, 6R)-6-benzhydryl-tetrahydropyran-3-ol (38)

Treatment of (1S, 4R, 6R)-4-benzhydryl-3,7-dioxa-bicyclo[4.1.0]heptane **28d** (0.06 g, 0.23 mmol) with a suspension of LiAlH₄ (0.06 g, 1.58 mmol) in pentane along with 12-crown-4 ether (0.31 g, 1.74 mmol) for 15 h at room temperature afforded (3R, 6R)-6-benzhydryl-tetrahydropyran-3-ol **38** 0.046 g (77%, [α]_{D}=(+)74.9, c=1, MeOH).

¹HNMR (CDCl₃, 400MHz) 1.28(m, 1H, H-5) 1.58-1.74(m, 2H, H-4, H-5) 1.88(m, 1H, H-5) 2.20(bs, 1H OH) 3.63(m, 1H, H-2) 3.75(bs, 1H, H-3) 3.88-4.10(m, 3H, H-2, H-6, Ph₂CH) 7.1-7.4(m, 10H, aromatic-CH). ¹³CNMR (CDCl₃, 100MHz) 24.95 30.15 57.78 64.77 73.02 79.64 126.56 126.77 128.58 128.69 128.71 128.81 142.30 142.42.

### Procedure J. Synthesis of methanesulfonic acid cis-(3R, 6R)-6-benzhydryl-tetra-hydropyran-3-yl ester (39)

Methanesulfonyl chloride (0.067 g, 0.58 mmol) was reacted with cis-(3R, 6R)-6-diphenylmethyl-tetrahydropyran-3-ol **38** (0.078 g, 0.29 mmol) in the presence of triethylamine (0.044 g, 0.44 mmol) in dry methylene chloride (10 ml) to give cis-(3R, 6R)-6-diphenylmethyl tetrahydropyran-3-yl methanesulfonate **39** 0.1 g (quantitative yield, [α]_{D}=(+)65.7, c=1, MeOH).

¹HNMR (CDCl₃, 400MHz) 1.46(m, 1H, H-5) 1.62-1.78(m, 2H, H-4, H-5) 2.24(m, 1H, H-5) 2.96(s, 3H, CH₃SO₂) 3.36(t, J=10.4 Hz, 1H, H-2) 3.88(d, J=8.8 Hz, 1H, Ph₂CH) 4.0(dt, J=2 Hz, 8.8 Hz, 1H, H-2) 4.14(m, 1H, H-2) 4.61(m, 1H, H-3) 7.1-7.4(m, 10H, aromatic-CH). ¹³CNMR (CDCl₃, 100MHz) 29.49 30.58 38.71 57.10 69.87 75.23 79.07 126.69 126.93 128.57 128.60 128.67 128.89 141.94 142.33.

### Synthesis of (3S, 6R)-6-benzhydryl-tetrahydropyran-3-ol (13b)

*cis*-(3R, 6R)-6-diphenylmethyl tetrahydropyran-3-yl methanesulfonate **39** (0.1 g, 0.29 mmol) and 18-crown-6 (0.76 g, 2.9 mmol) are dissolved in a 1:1 mixture of DMSO and DMF (15 ml). KO₂ (0.062 g, 0.87 mmol) was added and the solution was stirred under N₂. After 5 hr, the reaction was over. H₂O (1 ml) and a few drops of 1M solution of HCl were added and the solution was extracted with Et₂O (3 X 10 ml). The ether phase was washed with water and saturated brine, dried over anhydrous Na2SO4 and evaporated to dryness. The crude product was chromatographed on silica gel using hexane/ethyl acetate 1:1 to yield pure trans-(3S, 6R)-6-benzhydryl-tetrahydropyran-3-ol **33b** 0.062 g (80%, [α]_{D}=(+)62.8, c=1, MeOH).

¹HNMR (CDCl₃, 400MHz) 1.40(m, 2H, H-5) 1.58(m, 1H, H-4) 2.07(m, 1H, H-4) 3.14(t, J=10.4Hz, 1H, H-2) 3.69(m, 1H, H-3) 3.82-4.04(m, 3H, H-2, H-6, Ph₂CH) 7.1-7.4(m, 10H, aromatic-CH). ¹³CNMR (CDCl₃, 100MHz) 29.47 33.18 57.40 66.55 73.12 78.95 126.51 126.74 128.54 128.60 128.79 142.41 142.77.

### Synthesis of methanesulfonic acid trans-(3R, 6S)-6-benzhydryl-tetra-hydropyran-3-yl ester (34a)

Methanesulfonyl chloride (0.20 g, 1.7 mmol) was reacted with *trans*-(3R,6S)-6-diphenylmethyl-tetrahydropyran-3-ol **33a** (0.23 g, 0.85 mmol) (Procedure J) to give *trans*-(3R, 6S)-6-diphenylmethyl tetrahydropyran-3-yl methanesulfonate **34a** 0.23 g (80%, [α]_{D}=(-)54, c=1, MeOH).

¹H NMR(400MHz, CDCl₃) 1.47(m, 1H, H-5) 1.62-1.80(m, 2H, H-5, H-4) 2.25(m, 1H, H-4) 2.98(s, 3H, CH₃SO₂) 3.37(t, J=10.4Hz, 1H, H-2ax) 3.89(d, J=8.8Hz, 1H, Ph₂CH) 4.01(dt, J=2Hz, 9.6Hz, 1H, H-6) 4.15(m, 1H, H-2eq) 4.62(m, 1H, H-3) 7.16-7.38(m, 10H, aromatic-CH). ¹³C NMR(100MHz, CDCl₃) δ(ppm) 29.46, 30.57, 38.71, 57.07, 69.85, 75.19, 79.04, 126.67, 126.90, 128.54, 128.57, 128.63, 128.86, 141.87, 142.28.

### Synthesis of methanesulfonic acid trans-(3S, 6R)-6-benzhydryl-tetra-hydropyran-3-yl ester 34b

Trans-(3S, 6R)-6-benzhydryl-tetrahydropyran-3-ol (0.025 g, 0.093 mmol) was reacted with methanesulfonyl chloride (0.021 g, 0.19 mmol) (Procedure J) to yield trans-(3S, 6R)-6-benzhydryl-tetrahydropyran-3-yl ester **34b** 0.028 g (88%, [α]_{D}=(+)54.8, c=1, MeOH).

¹H NMR(400MHz, CDCl₃) 1.47(m, 1H, H-5) 1.62-1.80(m, 2H, H-5, H-4) 2.25(m, 1H, H-4) 2.98(s, 3H, CH₃SO₂) 3.37(t, J=10.4Hz, 1H, H-2ax) 3.89(d, J=8.8Hz, 1H, Ph₂CH) 4.01(dt, J=2Hz, 9.6Hz, 1H, H-6) 4.15(m, 1H, H-2eq) 4.62(m, 1H, H-3) 7.16-7.38(m, 10H, aromatic-CH). ¹³C NMR(100MHz, CDCl₃) δ(ppm) 29.46, 30.57, 38.71, 57.07, 69.85, 75.19, 79.04, 126.67, 126.90, 128.54, 128.57, 128.63, 128.86, 141.87, 142.28.

### Procedure K. Synthesis of Cis-(3S, 6S)-3-azido-6-benzhydryl-tetrahydropyran 35a

*Trans*-(3R,6S)-6-diphenylmethyl-tetrahydropyran-3-yl methanesulfonate **34a** (0.23 g, 0.68 mmol) in dry DMF (10 ml) was reacted with sodium azide (0.13 g, 2.03 mmol) to yield *cis*-(3S, 6S)-3-azido-6-diphenylmethyl-tetrahydropyran, **35a,** 0.17 g (86%, [α]_{D} = (-)78.2, c = 1, MeOH).

¹H NMR (400MHz, CDCl₃) 1.38 (m, 1H, H-5) 1.60-1.84 (m, 2H, H-5, H-4) 1.98 (m, 1H, H-4), 3.55 (m, 1H, H-3), 3.63 (dd, J=2Hz, 12.4Hz, 1H, H-2) 3.98-4.12(m, 3H, H-2, H-6, Ph₂CH) 7.16-7.40(m, 10H, aromatic-CH). ¹³C NMR(100MHz, CDCl₃) 25.47, 27.70, 55.60, 57.58, 69.79, 79.48, 126.58, 126.84, 128.59, 128.69, 128.76, 128.86 142.28 142.29.

### Procedure L. Synthesis of Cis-(3S, 6S)-(6-benzhydryl-tetrahydropyran-3-yl)-amine (36a)

Cis-(3S, 6S)-3-azido-6-diphenylmethyl-tetrahydropyran 35a (0.17 g, 0.58 mmol) in methanol (25 ml) was hydrogenated employing as catalyst 10% Pd-C (0.017 g, 10% wt) for 4 hr to give *cis*-(3S, 6S)-(6-diphenylmethyl-tetrahydropyran-3-yl)-amine **36a,** 0.12 g (78%, [α]_{D}=(-)74.3, c=1, MeOH).

¹H NMR(400MHz, CD₃OD) 1.27(m, 1H, H-5) 1.52(m, 1H, H-5) 1.62-1.80(m, 2H, H-4) 2.78(bs, 1H, H-3) 3.63(m, 2H, H-2) 3.95(d, J=8.8Hz, 1H, Ph₂CH) 4.10(dt, J=2Hz, 9.6Hz, 1H, H-6) 7.0-7.40(m, 10H, aromatic-CH). ¹³C NMR(100MHz, CDCl₃) 24.47, 29.29, 45.15, 57.32, 72.08, 79.28, 125.97, 126.34, 128.02, 128.39, 128.42 128.54 142.72 142.82.

### Synthesis of Cis-(3R. 6R)-(6-benzhydryl-tetrahydropyran-3-yl)-amine (34b)

### Synthesis of Cis-(3R, 6R)-3-azido-6-benzhydryl-tetrahydropyran

*Trans*-(3S,6R)-6-diphenylmethyl-tetrahydropyran-3-yl methanesulfonate (0.028 g, 0.082 mmol) was reacted with NaN₃ (0.016 g, 0.25 mmol) (Procedure L) to yield *cis*-(3R, 6R)-3-azido-6-benzhydryl-tetrahydropyran 0.024 g (quantitative yield, [α]_{D}=(+)77.6, c=1, MeOH).

¹H NMR (400MHz, CDCl₃) 1.38 (m, 1H, H-5) 1.60-1.84 (m, 2H, H-5, H-4) 1.98 (m, 1H, H-4), 3.55 (m, 1H, H-3), 3.63 (dd, J=2Hz, 12.4Hz, 1H, H-2) 3.98-4.12(m, 3H, H-2, H-6, Ph₂CH) 7.16-7.40(m, 10H, aromatic-CH). ¹³C NMR(100MHz, CDCl₃) 25.47, 27.70, 55.60, 57.58, 69.79, 79.48, 126.58, 126.84, 128.59, 128.69, 128.76, 128.86 142.28 142.29.

Cis-(3R,6R)-3-azido-6-diphenylmethyl-tetrahydropyran (0.024 g, 0.082 mmol) was hydrogenated (Procedure M) to yield *cis*-(3R, 6R)-(6-benzhydryl-tetrahydropyran-3-yl)-amine **34b** 0.02 g (92%, [α]_{D}=(+)74.0, c=1, MeOH).

¹H NMR(400MHz, CD₃OD) 1.27(m, 1H, H-5) 1.52(m, 1H, H-5) 1.62-1.80(m, 2H, H-4) 2.78(bs, bs, 1H, H-3) 3.63(m, 2H, H-2) 3.95(d, J=8.8Hz, 1H, Ph₂CH) 4.10(dt, J=2Hz, 9.6Hz, 1H, H-6) 7.0-7.40(m, 10H, aromatic-CH). ¹³C NMR(100MHz, CDCl₃) 24.47, 29.29, 45.15, 57.32, 72.08, 79.28, 125.97, 126.34, 128.02, 128.39, 128.42 128.54 142.72 142.82.

### Synthesis of cis-(3S, 6S)-(6-benzhydryl-tetrahydropyran-3-yl)-(4-hydroxy-benzyl)-amine (-)37a (Comparative)

*Cis*-(3S, 6S)-3-amino-6-diphenylmethyl pyran 36a (0.02 g, 0.075 mmol) was reacted with 4-hydroxybenzaldehyde (0.009 g, 0.075 mmol) in the presence of glacial acetic acid (0.005 g, 0.075 mmol) in 1,2-dichloroethane (10 ml), then was reduced by NaCNBH₃ (0.0057 g, 0.09 mmol) (Procedure H) to give *cis*-(3S, 6S)-(6-benzhydryl-tetrahydropyran-3-yl)-(4-fluorobenzyl)-amine (-)-37a, 0.02 g (72%, [α]_{D}=(-) 38.3, c=1, MeOH).

¹H NMR (400MHz, CDCl₃) 1.36(m, 1H, H-5) 1.51(m, 1H, H-5) 1.68(m, 1H, H-4) 2.0(m, 1H, H-4) 2.71(s, 1H, H-3) 3.56(dd, J=1.6Hz, 11.6Hz, 1H, H-2) 3.64(m, 2H, (HO)Ph-CH₂) 3.96(d, J=8.4Hz, 1H, Ph₂CH) 4.02-4.16(m, 2H, H-6, H-2) 6.52(m, 2H, aromatic-CH) 6.98-7.38(m, 12H, aromatic-CH). ¹³C NMR (100MHz, CDCl₃) 25.28 27.31 50.39 50.68 57.21 69.88 79.45 116.04 126.56 126.67 128.54 128.70 128.73 128.93 129.86 130.47 142.16 142.58 155.93.

Free base (-)-37a was converted into the oxalate: mp 136-138 °C. C, H, N Anal: [C₂₅H₂₇NO₂· (COOH)₂· 0.6H₂O].

### Syntheis of cis-(3R, 6R)-(6-benzhydryl-tetrahydropyran-3-yl)-(4-hydroxy-benzyl)-amine (+)37a (Comparative)

*cis*-(3R, 6R)-3-amino-6-diphenylmethyl pyran 34b (0.024 g, 0.09 mmol) was reacted with 4-hydroxybenzaldehyde (0.011 g, 0.09 mmol) in the presence of glacial acetic acid (0.0054 g, 0.09 mmol) in 1,2-dichloroethane (10 ml), then was reduced by NaCNBH₃(0.012 g, 0.18 mmol) (Procedure H) to give *cis*-(3R, 6R)-(6-benzhydryl-tetrahydropyran-3-yl)-(4-fluorobenzyl)-amine 0.024 g (+)-**37a** (71 %, [α]_{D}=(+) 40.1, c=1, MeOH).

¹H NMR (400MHz, CDCl₃) 1.34(m, 1H, H-5) 1.51(m, 1H, H-5) 1.65(m, 1H, H-4) 1.96(m, 1H, H-4) 2.67(m, 1H, H-3) 3.56(dd, J = 1.6Hz, 11.6Hz, 1H, H-2) 3.66(m, 2H, (HO)Ph-CH₂) 3.96(d, J=8.8Hz, 1H, Ph₂CH) 3.98-4.12(m, 2H, H-6, H-2) 6.65(m, 2H, aromatic-CH) 7.06-7.38(m, 12H, aromatic-CH). ¹³C NMR (100MHz, CDCl₃) 25.28 27.31 50.39 50.68 57.21 69.88 79.45 116.04 126.56 126.67 128.54 128.70 128.73 128.93 129.86 130.47 142.16 142.58 155.93.

Free base (+)-**37a** was converted into the oxalate: mp 136-138 °C. C, H, N Anal: [C₂₅H₂₇NO₂ · (COOH)₂ · 1.8H₂O].

**Comparative TABLE 4**

| Affinity of Drugs at DAT, SERT, and NET in Rat Brain. | | | | |
|---|---|---|---|---|
| Compound | DAT binding, IC50, nM, [³H]Win 35, 428^{a} | DAT uptake, IC₅₀, nM [³H]DA^{a} | SERT uptake, IC₅₀, nM [³H]5-HT^{a} | NET uptake, IC₅₀, nM [³H]NE^{a} |
| GBR 12909^{b} | 10.6±1.9 | 14.2±2.9 | 101.4±14.2 | 114±36 |
| (+)-29a | 182±11 | 148±22 | 745±30 | 445±39 |
| (+)-29g | 3750±620 | 2670±260 | 3810±460 | 1840±580 |
| (+)-29b | 1030±120 | 440±30 | 5560±640 | 1130±580 |
| (+)-29c | 443±52 | 218±20 | 2950±380 | 77.3±3.0 |
| (+)-29d | 596±84 | 341±43 | 6120±730 | 770±33 |
| (+)-29h | 1250±100 | 962±97 | 4420±410 | 3220±570 |
| (-)-29a | 226±40 | 155±16 | 28.9±4.1 | 17.7±5.9 |
| (-)-29g | 771±86 | 822±120 | 1070±100 | 765±34 |
| (-)-29b | 308±25 | 169±20 | 676±33 | 13.3±1.0 |
| (-)-29c | 1050±40 | 427±67 | 3570±140 | 439±14 |
| (-)-29d | 1860±710 | 600±79 | 862±36 | 5.59±1.05 |
| (-)-29h | 4640±030 | 2610±140 | 10,000±1400 | 336±33 |
| (-)-29e | 1,060±100 | 710±130 | 24.0±4.0 | 115±14 |
| (-)-29f | 298±29 | 135±3 | 25.4±2.0 | 108±11 |
| (-)-32a | 289±23 | 232±28 | 265±14 | 11.22±1.01 |
| (-)-32b | 705±176 | 162±11 | 18.7±1.6 | 2.42±0.47 |
| (+)-32a | 135±6 | 123±10 | 2833±480 | 102±20 |
| (+)-32b | 494±96 | 247±21 | 272±55 | 15.0±4.5 |
| (-)-37a | 280±8 | 114±8 | 42±3 | 5.5±0.1 |
| (-)-37b | | | | |
| (+)-37a | 141±2.2 | 90.7±4 | 209±25 | 45.6±6 |
| (+)-37b | | | | |

**Comparative TABLE 5**

| Affinity of Drugs at Dopamine, Serotonin, and Norepinephrine Transporters in Rat Striatum | | | | |
|---|---|---|---|---|
| Compound | DAT binding, IC50, nM, [³H]Win 35, 428⁸ | SERT binding, IC50, nM [³H]citalopram^{a} | NET binding, IC₅₀, nM [³H]nisoxetine^{a} | DAT uptake, IC₅₀, nM [³H]DA^{a} |
| Cocaine | 266±37 | 737±160 | 3,130±550 | |
| GBR 12909 | 10.6±1.9 | 132±0 | 496±22 | |
| 48 | 80.4±17.4 | >10,000 | 1328±592 | 104±49 |
| 52 | 162±9 | > 10,000 | 1435± | 165±17 |
| 57 | 398±33 | 4400 | 3432±1752 | 215±14 |
| 63 | 420±38 | 1491±134^{b} | 1486±443^{b} | 158±28 |
| 64 | 296±42 | 2441±188 | 255±52 | |

| | | | | |
|---|---|---|---|---|
| b. Uptake inhibition values | | | | |

The most widely accepted basis of the cause of depression focuses on monoamines. Imbalances in the level of dopamine (DA), serotonin (5-HT) and norepinephrine (NE) neurotransmitter systems are responsible for such neurodegeneration. Clinical studies as well as basic research in neurobiology has demonstrated that two monoaminergic systems are involved in the etiology and therapy of affective disorders, namely serotonin and Norepinephrine. The common basis of pharmacotherapy is based on the increase of intracellular concentration of serotonin and norepinephrine by blocking the reuptake mechanism of serotonin and norepinephrine transporters. It is evident from Table 4 that the compounds (-)-29a, (-)-29e, (-)-29f, (-)-32b and (-)-37a are potent blockers for both SERT and NET. Compounds with such properties are known as SNRI and are potent antidepressants. The known antidepressant drugs belonging to this SNRI category and used in the clinics are venlafaxine, milnacipran, chlorimipramine and duloxetine. SNRI are considered to have faster onset of action compared to SSRI and are more effective to treat depression. On the other hand, compounds (-)-29b, (-)-29d, (-)-32a, (+)-32a and (+)-37a are selective blockers for NET. Compounds with such biological property are known as NRI and are also considered potent antidepressants. A known potent NRI which was recently approved for antidepressant treatment, is Reboxetine.

Current existing SNRI, SSRI and RNI are also being used in other related neurodisorders including post-traumatic stress disorder, social phobia, obsessive compulsive disorders, anxiety and urinary stress incontinence. For this reason the compounds included in this application will also have use in neurodisorders like post-traumatic stress disorder, social phobia, obsessive compulsive disorders, anxiety and urinary stress incontinence.

While embodiments of the invention have been illustrated and described, it is not intended that these embodiments illustrate and describe all possible forms of the invention. Rather, the words used in the specification are words of description rather than limitation.

## Claims

1. A substituted pyran compound having the structural formula selected from the group of compounds represented by formulae VIa, VIb, VIc, and VId: 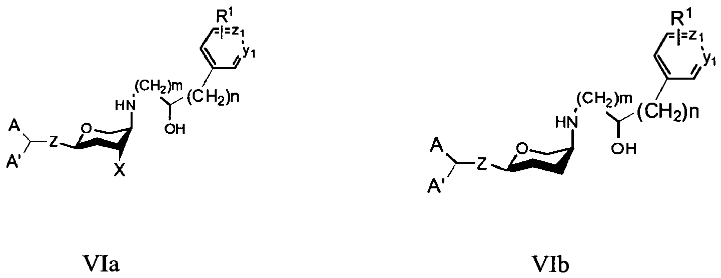 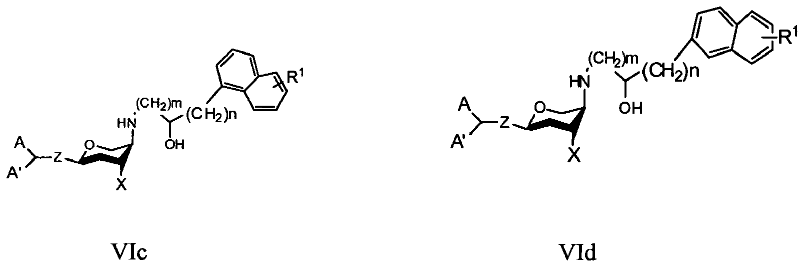 or a pharmaceutically acceptable derivative or salt thereof, wherein:
m and n are 0-4;
X is H, OH, NH₂, or NHR;
Z₁ and Y₁ are C- or N-atom;
Z is selected from the group consisting of a chemical bond and -Y-(CH₂)ₒ -;
Y is NH or O;
o is 0, 1, 2, 3, or 4;
R is H, C₁₋₈ alkyl, C₂₋₈ alkenyl or C₂₋₈ alkynyl;
at least one of A and A' are individually selected from the group of optionally substituted C₄-C₁₄ aryl and heteroaryl wherein heteroatoms of heteroaryl A and/or A' are selected from the group consisting of O, N and S,
wherein, at least one of A and A' are selected from the group consisting of: 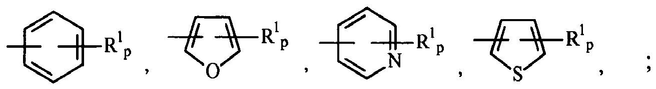 and 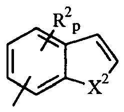
p is 0-6;
R¹ is C₁₋₄ alkyl, C₂₋₆ alkenyl, C₂₋₆ optionally halogenated alkynyl, C₂₋₆ hydroxyalkynyl, halo, -CN, -COOR⁴, -OH, -NO₂, -NH₂, -NHR⁴, -SO₂NH₂, -NHSO₂R⁸, -OCF₃, or -OR⁸;
R² has the meaning of R¹ and also a 5 or 6 membered heterocycle containing 1 or more heteroatoms selected from the group consisting ofN, O, and S;
X² is N, O, or S;
R⁴ is H, C₁₋₁₈ alkyl, C₅₋₁₀ cycloalkyl, or C₂₋₁₈ alkylene; and
R⁸ is C₁₋₈ alkyl, C₅₋₆ cycloalkyl,C₂₋₈ alkenyl, or a 5 or 6-member aromatic ring including heterocyclic rings.

2. A substituted pyran compound having a formula VII: 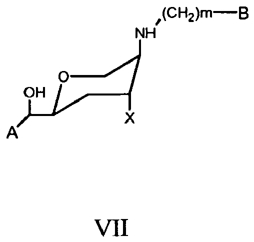 or a pharmaceutically acceptable derivative or salt thereof, wherein:
m is 0-4;
X is - H, -OH,- NH₂, or -NHR;
A is selected from the group consisting of: 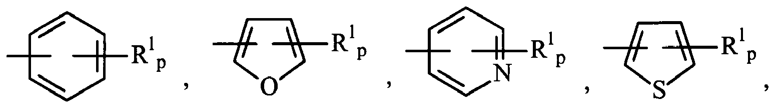 and 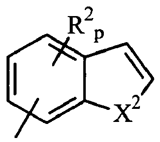
p is 0-6;
R¹ is C₁₋₄ alkyl, C₂₋₆ alkenyl, C₂₋₆ optionally halogenated alkynyl, C₂₋₆ hydroxyalkynyl, halo, -CN, -COOR⁴, -OH, -NO₂, -NH₂, -NHR⁴, -SO₂NH₂ -NHSO₂R⁸, -OCF₃, or -OR⁸;
R² has the meaning of R¹ and also a 5 or 6 membered heterocycle containing 1 or more heteroatoms selected from the group consisting of N, O, and S;
X² is N, O, or S;
R⁴ is H, C₁₋₁₈ alkyl, C₅₋₁₀ cycloakyl, or C₂₋₁₈ alkylene; and
R⁸ is C₁₋₈ alkyl, C₅₋₆ cycloalkyl,C₂₋₈ alkenyl, or a 5 or 6-member aromatic ring including heterocyclic rings; and
B is selected from the group consisting of

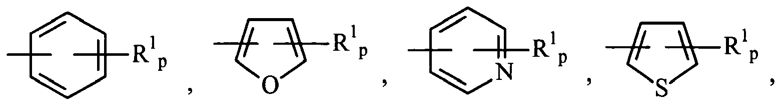

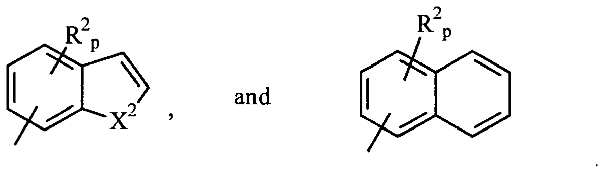

3. A compound of claim 1 or claim 2 for use in reducing monoamine reuptake in a mammalian species.

4. A compound of claim 1 or claim 2 for use in treating depression.

5. Compound of claim 1 or claim 2 for use in the manufacture of a medicament for reducing monoamine reuptake in a mammalian species.

6. Compound of claim 1 or claim 2 for use in the manufacture of a medicament for treating depression.

## Patentansprüche

1. Substituierte Pyranverbindung mit der Strukturformel, ausgewählt aus der Gruppe von Verbindungen, dargestellt durch Formeln VIa, VIb, VIc und VId: 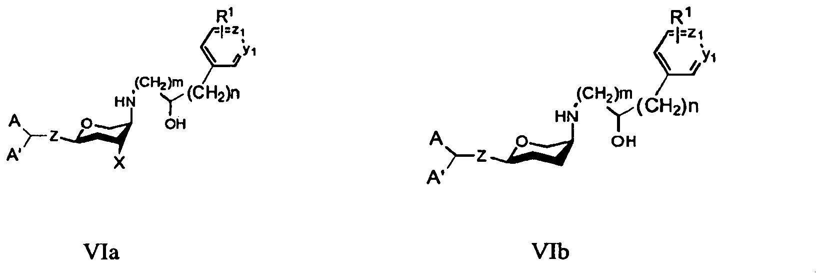 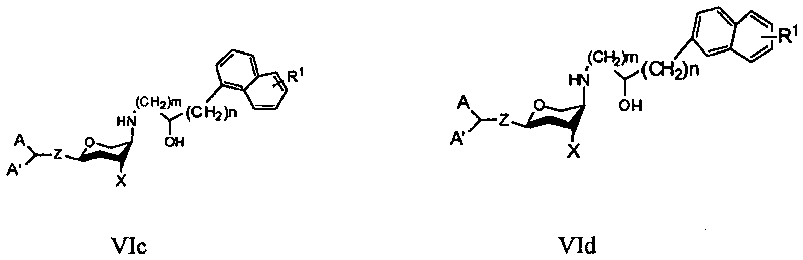 oder ein pharmazeutisch annehmbares Derivat oder Salz davon, worin:
m und n 0-4 sind;
X für H, OH, NH₂ oder NHR steht;
Z₁ und Y₁ für C- oder N-Atom stehen;
Z ausgewählt ist aus der Gruppe bestehend aus einer chemischen Bindung und -Y-(CH₂)ₒ-;
Y für NH oder O steht;
o 0, 1, 2, 3 oder 4 ist;
R für H, C₁₋₈-Alkyl, C₂₋₈-Alkenyl oder C₂₋₈-Alkinyl steht;
mindestens eines von A und A' individuell ausgewählt sind aus der Gruppe von gegebenenfalls substituiertem C₄-C₁₄-Aryl und Heteroaryl, worin Heteroatome von Heteroaryl A und/oder A' ausgewählt sind aus der Gruppe bestehend aus O, N und S,
worin mindestens eines von A und A' ausgewählt sind aus der Gruppe bestehend aus: 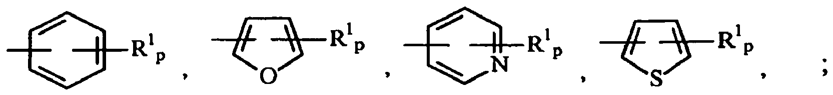 und 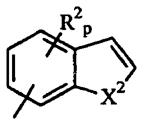
p 0-6 ist;
R¹ für C₁₋₄-Alkyl, C₂₋₆-Alkenyl, C₂₋₆- gegebenenfalls halogeniertes Alkinyl, C₂₋₆-Hydroxyalkinyl, Halogen, -CN, -COOR⁴, -OH, -NO₂, NH₂, -NHR⁴, -SO₂NH₂, -NHSO₂R⁸, -OCF₃ oder -OR⁸ steht;
R² die Bedeutung von R¹ hat und auch eines 5- oder 6-gliedrigen Heterocyclus, welcher 1 oder mehrere Heteroatome enthält, ausgewählt aus der Gruppe bestehend aus N, O und S;
X² für N, O oder S steht;
R⁴ für H, C₁₋₁₈-Alkyl, C₅₋₁₀-Cycloalkyl oder C₂₋₁₈-Alkylen steht; und
R⁸ für C₁₋₈-Alkyl, C₅₋₆-Cycloalkyl, C₂₋₈-Alkenyl oder einen 5-oder 6-gliedrigen aromatischen Ring einschließlich heterocyclische Ringe steht.

2. Substituierte Pyranverbindung mit einer Formel VII: 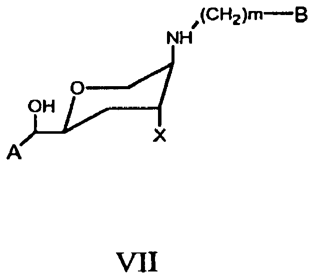 oder ein pharmazeutisch annehmbares Derivat oder Salz davon, worin:
m 0-4 ist;
X für -H, -OH, -NH₂ oder -NHR steht;
A ausgewählt ist aus der Gruppe bestehend aus: 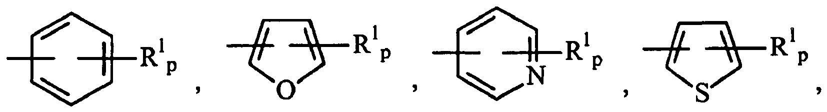 und 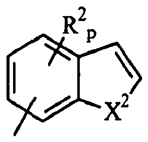
p 0-6 ist;
R¹ für C₁₋₄-Alkyl, C₂₋₆-Alkenyl, C₂₋₆- gegebenenfalls halogeniertes Alkinyl, C₂₋₆-Hydroxyalkinyl, Halogen, -CN, -COOR⁴, -OH, -NO₂, NH₂, -NHR⁴, -SO₂NH₂, -NHSO₂R⁸, -OCF₃ oder -OR⁸ steht;
R² die Bedeutung von R¹ hat und auch eines 5- oder 6-gliedrigen Heterocyclus, welcher 1 oder mehrere Heteroatome enthält, ausgewählt aus der Gruppe bestehend aus N, O und S;
X² für N, O oder S steht;
R⁴ für H, C₁₋₁₈-Alkyl, C₅₋₁₀-Cycloalkyl oder C₂₋₁₈-Alkylen steht; und
R⁸ für C₁₋₈-Alkyl, C₅₋₆-Cycloalkyl, C₂₋₈-Alkenyl oder einen 5-oder 6-gliedrigen aromatischen Ring einschließlich heterocyclische Ringe steht; und
B ausgewählt ist aus der Gruppe bestehend aus

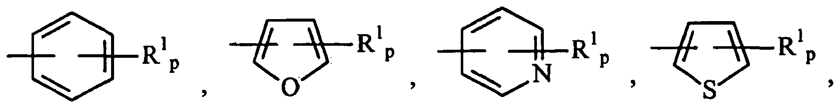

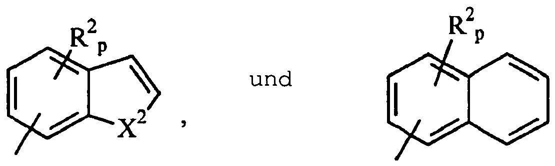

3. Verbindung nach Anspruch 1 oder Anspruch 2 zur Verwendung beim Verringern von Monoamin-Wiederaufnahme in einer Säugerart.

4. Verbindung nach Anspruch 1 oder Anspruch 2 zur Verwendung beim Behandeln von Depression.

5. Verbindung nach Anspruch 1 oder Anspruch 2 zur Verwendung in der Herstellung eines Medikaments zum Verringern von Monoamin-Wiederaufnahme in einer Säugerart.

6. Verbindung nach Anspruch 1 oder Anspruch 2 zur Verwendung in der Herstellung eines Medikaments zum Behandeln von Depression.

## Revendications

1. Composé de pyrane substitué ayant la formule développée choisie dans le groupe de composés représentés par les formules VIa, VIb, VIc et VId : 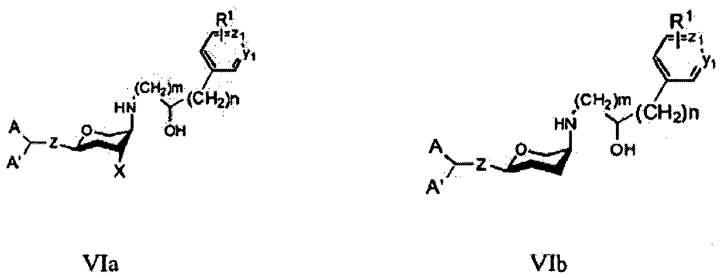 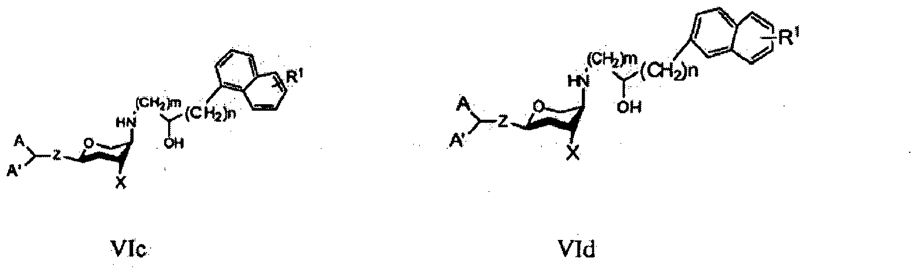 ou un dérivé pharmaceutiquement acceptable ou sel de celui-ci, dans lesquelles :
m et n sont 0 à 4 ;
X est H, OH, NH₂ ou NHR ;
Z₁ et Y₁ sont des atomes de C ou de N ;
Z est choisi dans le groupe comprenant une liaison chimique et -Y-(CH₂)ₒ- ;
Y est NH ou O ;
o est 0, 1, 2, 3 ou 4 ;
R est H, un groupe alkyle en C₁ à C₈, alcényle en C₂ à C₈ ou alcinyle en C₂ à C₈ ;
au moins l'un des A et A' est choisi individuellement dans les groupes aryle en C₄ à C₁₄ et hétéroaryle éventuellement substitués, les hétéroatomes des groupes hétéroaryle A et/ou A' étant choisis dans le groupe comprenant O, N et S,
dans lesquelles au moins l'un des A et A' est choisi dans le groupe comprenant : 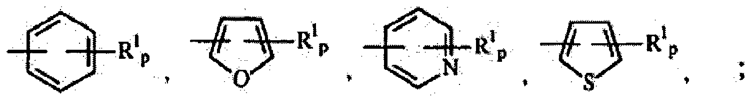 et 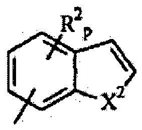
p est 0 à 6 ;
R¹ est un groupe alkyle en C₁ à C₄, alcényle en C₂ à C₆, alcynyle en C₂ à C₆ éventuellement halogéné, hydroxyalcynyle en C₂ à C₆, halogéno, -CN, -COOR⁴, -OH, -NO₂, -NH₂, -NHR⁴, -SO₂NH₂, -NHSO₂R⁸, -OCF₃ ou -OR⁸ ;
R² a la signification de R¹ et également, un hétérocycle de 5 ou 6 chaînons contenant 1 ou plusieurs hétéroatomes choisis dans le groupe comprenant N, O et S ;
X² est N, O ou S ;
R⁴ est H, un groupe alkyle en C₁ à C₁₈, cycloalkyle en C₅ à C₁₀ ou alkylène en C₂ à C₁₈ ; et
R⁸ est un groupe alkyle en C₁ à C₈, cycloalkyle en C₅ à C₆, alcényle en C₂ à C₈ ou un cycle aromatique de 5 ou 6 chaînons comprenant des cycles hétérocycliques.

2. Composé de pyrane substitué ayant une formule VII : 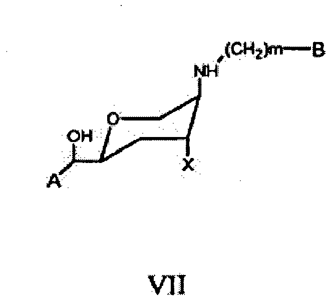 ou un dérivé pharmaceutiquement acceptable ou sel de celui-ci, dans laquelle :
m est 0 à 4 ;
X est -H, -OH, -NH₂ ou -NHR ;
A est choisi dans le groupe comprenant : 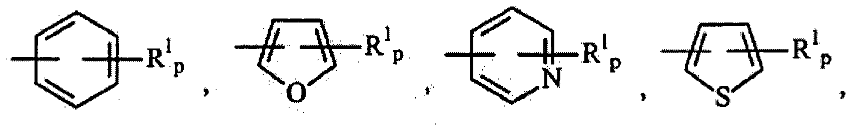 et 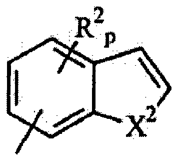
p est 0 à 6 ;
R¹ est un groupe alkyle en C₁ à C₄, alcényle en C₂ à C₆, alcynyle en C₂ à C₆ éventuellement halogéné, hydroxyalcynyle en C₂ à C₆, halogéno, -CN, -COOR⁴, -OH, -NO₂, -NH₂, -NHR⁴, -SO₂NH₂, -NHSO₂R⁸, -OCF₃ ou -OR⁸ ;
R² a la signification de R¹ et également, un hétérocycle de 5 ou 6 chaînons contenant 1 ou plusieurs hétéroatomes choisis dans le groupe comprenant N, O et S ;
X² est N, O ou S ;
R⁴ est H, un groupe alkyle en C₁ à C₁₈, cycloalkyle en C₅ à C₁₀ ou alkylène en C₂ à C₁₈ ; et
R⁸ est un groupe alkyle en C₁ à C₈, cycloalkyle en C₅ à C₆, alcényle en C₂ à C₈ ou un cycle aromatique de 5 ou 6 chaînons comprenant des cycles hétérocycliques ; et
B est choisi dans le groupe comprenant

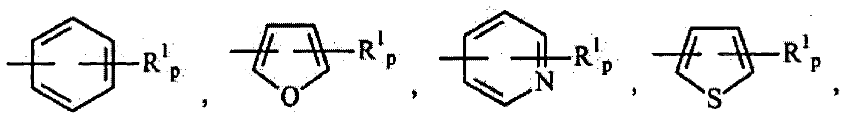

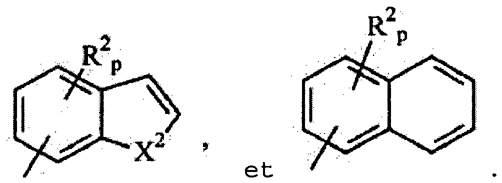

3. Composé selon la revendication 1 ou la revendication 2, pour son utilisation dans la réduction de la recapture de la monoamine chez une espèce de mammifère.

4. Composé selon la revendication 1 ou la revendication 2, pour son utilisation dans le traitement de la dépression.

5. Composé selon la revendication 1 ou la revendication 2, pour son utilisation dans la production d'un médicament pour la réduction de la recapture de la monoamine chez une espèce de mammifère.

6. Composé selon la revendication 1 ou la revendication 2, pour son utilisation dans la production d'un médicament pour le traitement de la dépression.
